# EUROPEAN PATENT APPLICATION

(11) **EP 4 019 538 A1**
(43) Date of publication of application: **29.06.2022**
(21) Application number: 20216649.2
(22) Date of filing: 22.12.2020
(51) Int. Cl.: C07K 14/725, C12N 9/22, A61K 35/17, A61P 35/00, C12N 5/0783

(54) **REPROGRAMMING IMMUNE CELLS BY TARGETED INTEGRATION OF ZETA-DEFICIENT CHIMERIC ANTIGEN RECEPTOR TRANSGENES**

(71) Applicant: Charité - Universitätsmedizin Berlin, 10117 Berlin (DE)
(72) Inventor: WAGNER, Dimitrios Laurin, 13347 Berlin (DE); KATH, Jonas Christian, 10437 Berlin (DE); SCHMÜCK-HENNERESSE, Michael, 10247 Berlin (DE); VOLK, Hans-Dieter, 10117 Berlin (DE); REINKE, Petra, 10117 Berlin (DE)
(74) Representative: Hertin und Partner Rechts- und Patentanwälte PartG mbB

(57) **Abstract**

The invention relates to a nucleic acid construct for targeting and integrating a CD3 zeta-deficient chimeric antigen receptor (CAR) fragment into an endogenous CD3 zeta/CD247 gene of a host genome. The invention further relates to a genetically modified human cell expressing an exogenous nucleic acid sequence encoding a CD3 zeta deficient CAR fragment, integrated in-frame into the endogenous CD3 zeta/CD247 gene for gene fusion, to form a functional CAR comprising an exogenous CAR fragment fused with an endogenous CD3 zeta domain.

SEQUENCE LISTING

<110>  Charité - Universitätsmedizin Berlin

<120>  REPROGRAMMING IMMUNE CELLS BY TARGETED INTEGRATION OF
       ZETA-DEFICIENT CHIMERIC ANTIGEN RECEPTOR TRANSGENES

<130>  2280-20EP

<160>  58

<170>  PatentIn version 3.5

<210>  1
<211>  508
<212>  PRT
<213>  Artificial Sequence

<220>
<223>  CAR fragment

<400>  1

Ala Gln Ser Phe Gly Leu Leu Asp Pro Lys Leu Cys Tyr Leu Leu Asp
1               5                   10                  15

Gly Ile Leu Phe Ile Tyr Arg Val Ile Leu Thr Ala Leu Phe Leu Arg
            20                  25                  30

Ala Thr Asn Phe Ser Leu Leu Lys Gln Ala Gly Asp Val Glu Glu Asn
        35                  40                  45

Pro Gly Pro Met Ala Leu Pro Val Thr Ala Leu Leu Leu Pro Leu Ala
    50                  55                  60

Leu Leu Leu His Ala Ala Arg Pro Asp Ile Gln Met Thr Gln Thr Thr
65                  70                  75                  80

Ser Ser Leu Ser Ala Ser Leu Gly Asp Arg Val Thr Ile Ser Cys Arg
                85                  90                  95

Ala Ser Gln Asp Ile Ser Lys Tyr Leu Asn Trp Tyr Gln Gln Lys Pro
            100                 105                 110

Asp Gly Thr Val Lys Leu Leu Ile Tyr His Thr Ser Arg Leu His Ser
        115                 120                 125

Gly Val Pro Ser Arg Phe Ser Gly Ser Gly Ser Gly Thr Asp Tyr Ser
    130                 135                 140

Leu Thr Ile Ser Asn Leu Glu Gln Glu Asp Ile Ala Thr Tyr Phe Cys
145                 150                 155                 160

Gln Gln Gly Asn Thr Leu Pro Tyr Thr Phe Gly Gly Gly Thr Lys Leu
                165                 170                 175

Glu Ile Thr Gly Gly Gly Gly Ser Gly Gly Gly Gly Ser Gly Gly Gly
            180                 185                 190

Gly Ser Glu Val Lys Leu Gln Glu Ser Gly Pro Gly Leu Val Ala Pro
        195                 200                 205

Ser Gln Ser Leu Ser Val Thr Cys Thr Val Ser Gly Val Ser Leu Pro
    210                 215                 220

Asp Tyr Gly Val Ser Trp Ile Arg Gln Pro Pro Arg Lys Gly Leu Glu
225                 230                 235                 240

Trp Leu Gly Val Ile Trp Gly Ser Glu Thr Thr Tyr Tyr Asn Ser Ala
                245                 250                 255

Leu Lys Ser Arg Leu Thr Ile Ile Lys Asp Asn Ser Lys Ser Gln Val
            260                 265                 270

Phe Leu Lys Met Asn Ser Leu Gln Thr Asp Asp Thr Ala Ile Tyr Tyr
        275                 280                 285

Cys Ala Lys His Tyr Tyr Tyr Gly Gly Ser Tyr Ala Met Asp Tyr Trp
    290                 295                 300

Gly Gln Gly Thr Ser Val Thr Val Ser Ser Glu Ser Lys Tyr Gly Pro
305                 310                 315                 320

Pro Cys Pro Pro Cys Pro Gly Gln Pro Arg Glu Pro Gln Val Tyr Thr
                325                 330                 335

Leu Pro Pro Ser Arg Asp Glu Leu Thr Lys Asn Gln Val Ser Leu Thr
            340                 345                 350

Cys Leu Val Lys Gly Phe Tyr Pro Ser Asp Ile Ala Val Glu Trp Glu
        355                 360                 365

Ser Asn Gly Gln Pro Glu Asn Asn Tyr Lys Thr Thr Pro Pro Val Leu
    370                 375                 380

Asp Ser Asp Gly Ser Phe Phe Leu Tyr Ser Lys Leu Thr Val Asp Lys
385                 390                 395                 400

Ser Arg Trp Gln Gln Gly Asn Val Phe Ser Cys Ser Val Met His Glu
                405                 410                 415

Ala Leu His Asn Ala Tyr Thr Gln Lys Ser Leu Ser Leu Ser Pro Gly
            420                 425                 430

Lys Lys Asp Pro Lys Phe Trp Val Leu Val Val Val Gly Gly Val Leu
        435                 440                 445

Ala Cys Tyr Ser Leu Leu Val Thr Val Ala Phe Ile Ile Phe Trp Val
    450                 455                 460

Arg Ser Lys Arg Ser Arg Leu Leu His Ser Asp Tyr Met Asn Met Thr
465                 470                 475                 480

Pro Arg Arg Pro Gly Pro Thr Arg Lys His Tyr Gln Pro Tyr Ala Pro
                485                 490                 495

Pro Arg Asp Phe Ala Ala Tyr Arg Ser Arg Val Lys
            500                 505

<210> 2
<211> 32
<212> PRT
<213> Artificial Sequence

<220>
<223> CAR component

<400> 2

Ala Gln Ser Phe Gly Leu Leu Asp Pro Lys Leu Cys Tyr Leu Leu Asp
1               5                   10                  15

Gly Ile Leu Phe Ile Tyr Arg Val Ile Leu Thr Ala Leu Phe Leu Arg
            20                  25                  30

<210> 3
<211> 19
<212> PRT
<213> Artificial Sequence

<220>
<223> CAR component

<400> 3

Ala Thr Asn Phe Ser Leu Leu Lys Gln Ala Gly Asp Val Glu Glu Asn
1               5                   10                  15

Pro Gly Pro

<210> 4
<211> 21

<212>  PRT
<213>  Artificial Sequence

<220>
<223>  CAR component

<400>  4

Met Ala Leu Pro Val Thr Ala Leu Leu Leu Pro Leu Ala Leu Leu Leu
1               5                   10                  15

His Ala Ala Arg Pro
            20

<210>  5
<211>  107
<212>  PRT
<213>  Artificial Sequence

<220>
<223>  CAR component

<400>  5

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
        35                  40                  45

Tyr His Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr
            100                 105

<210>  6
<211>  15
<212>  PRT
<213>  Artificial Sequence

<220>
<223>  CAR component

<400>  6

Gly Gly Gly Gly Ser Gly Gly Gly Gly Ser Gly Gly Gly Gly Ser
1               5                   10                  15

<210>  7
<211>  120
<212>  PRT
<213>  Artificial Sequence

<220>
<223>  CAR component

<400>  7

Glu Val Lys Leu Gln Glu Ser Gly Pro Gly Leu Val Ala Pro Ser Gln
1               5                   10                  15

Ser Leu Ser Val Thr Cys Thr Val Ser Gly Val Ser Leu Pro Asp Tyr
            20                  25                  30

Gly Val Ser Trp Ile Arg Gln Pro Pro Arg Lys Gly Leu Glu Trp Leu
        35                  40                  45

Gly Val Ile Trp Gly Ser Glu Thr Thr Tyr Tyr Asn Ser Ala Leu Lys
    50                  55                  60

Ser Arg Leu Thr Ile Ile Lys Asp Asn Ser Lys Ser Gln Val Phe Leu
65                  70                  75                  80

Lys Met Asn Ser Leu Gln Thr Asp Asp Thr Ala Ile Tyr Tyr Cys Ala
                85                  90                  95

Lys His Tyr Tyr Tyr Gly Gly Ser Tyr Ala Met Asp Tyr Trp Gly Gln
            100                 105                 110

Gly Thr Ser Val Thr Val Ser Ser
        115                 120

<210>  8
<211>  12
<212>  PRT
<213>  Artificial Sequence

<220>
<223>  CAR component

<400>  8

Glu Ser Lys Tyr Gly Pro Pro Cys Pro Pro Cys Pro
1               5                   10

<210>  9
<211>  107

<212>  PRT
<213>  Artificial Sequence

<220>
<223>  CAR component

<400>  9

Gly Gln Pro Arg Glu Pro Gln Val Tyr Thr Leu Pro Pro Ser Arg Asp
1               5                   10                  15

Glu Leu Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn Ala Tyr
                85                  90                  95

Thr Gln Lys Ser Leu Ser Leu Ser Pro Gly Lys
            100                 105


<210>  10
<211>  4
<212>  PRT
<213>  Artificial Sequence

<220>
<223>  CAR component

<400>  10

Lys Asp Pro Lys
1


<210>  11
<211>  27
<212>  PRT
<213>  Homo sapiens

<400>  11

Phe Trp Val Leu Val Val Val Gly Gly Val Leu Ala Cys Tyr Ser Leu
1               5                   10                  15

Leu Val Thr Val Ala Phe Ile Ile Phe Trp Val
            20                  25

<210>  12
<211>  41
<212>  PRT
<213>  Homo sapiens

<400>  12

Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210>  13

<400>  13
000

<210>  14
<211>  617
<212>  PRT
<213>  Artificial Sequence

<220>
<223>  CAR fragment

<400>  14

Ala Gln Ser Phe Gly Leu Leu Asp Pro Lys Leu Cys Tyr Leu Leu Asp
1               5                   10                  15

Gly Ile Leu Phe Ile Tyr Arg Val Ile Leu Thr Ala Leu Phe Leu Arg
            20                  25                  30

Ala Thr Asn Phe Ser Leu Leu Lys Gln Ala Gly Asp Val Glu Glu Asn
        35                  40                  45

Pro Gly Pro Met Ala Leu Pro Val Thr Ala Leu Leu Leu Pro Leu Ala
    50                  55                  60

Leu Leu Leu His Ala Ala Arg Pro Asp Ile Gln Met Thr Gln Thr Thr
65                  70                  75                  80

Ser Ser Leu Ser Ala Ser Leu Gly Asp Arg Val Thr Ile Ser Cys Arg
                85                  90                  95

Ala Ser Gln Asp Ile Ser Lys Tyr Leu Asn Trp Tyr Gln Gln Lys Pro
            100                 105                 110

Asp Gly Thr Val Lys Leu Leu Ile Tyr His Thr Ser Arg Leu His Ser
        115                 120                 125

Gly Val Pro Ser Arg Phe Ser Gly Ser Gly Ser Gly Thr Asp Tyr Ser
    130                 135                 140

Leu Thr Ile Ser Asn Leu Glu Gln Glu Asp Ile Ala Thr Tyr Phe Cys
145                 150                 155                 160

Gln Gln Gly Asn Thr Leu Pro Tyr Thr Phe Gly Gly Gly Thr Lys Leu
                165                 170                 175

Glu Ile Thr Gly Gly Gly Gly Ser Gly Gly Gly Gly Ser Gly Gly Gly
            180                 185                 190

Gly Ser Glu Val Lys Leu Gln Glu Ser Gly Pro Gly Leu Val Ala Pro
        195                 200                 205

Ser Gln Ser Leu Ser Val Thr Cys Thr Val Ser Gly Val Ser Leu Pro
    210                 215                 220

Asp Tyr Gly Val Ser Trp Ile Arg Gln Pro Pro Arg Lys Gly Leu Glu
225                 230                 235                 240

Trp Leu Gly Val Ile Trp Gly Ser Glu Thr Thr Tyr Tyr Asn Ser Ala
                245                 250                 255

Leu Lys Ser Arg Leu Thr Ile Ile Lys Asp Asn Ser Lys Ser Gln Val
            260                 265                 270

Phe Leu Lys Met Asn Ser Leu Gln Thr Asp Asp Thr Ala Ile Tyr Tyr
        275                 280                 285

Cys Ala Lys His Tyr Tyr Tyr Gly Gly Ser Tyr Ala Met Asp Tyr Trp
    290                 295                 300

Gly Gln Gly Thr Ser Val Thr Val Ser Ser Glu Ser Lys Tyr Gly Pro
305                 310                 315                 320

Pro Cys Pro Pro Cys Pro Gly Gln Pro Arg Glu Pro Gln Val Tyr Thr
                325                 330                 335

Leu Pro Pro Ser Arg Asp Glu Leu Thr Lys Asn Gln Val Ser Leu Thr
            340                 345                 350

Cys Leu Val Lys Gly Phe Tyr Pro Ser Asp Ile Ala Val Glu Trp Glu
        355                 360                 365

Ser Asn Gly Gln Pro Glu Asn Asn Tyr Lys Thr Thr Pro Pro Val Leu
    370                 375                 380

Asp Ser Asp Gly Ser Phe Phe Leu Tyr Ser Lys Leu Thr Val Asp Lys
385                 390                 395                 400

Ser Arg Trp Gln Gln Gly Asn Val Phe Ser Cys Ser Val Met His Glu
                405                 410                 415

Ala Leu His Asn Ala Tyr Thr Gln Lys Ser Leu Ser Leu Ser Pro Gly
            420                 425                 430

Lys Lys Asp Pro Lys Phe Trp Val Leu Val Val Val Gly Gly Val Leu
        435                 440                 445

Ala Cys Tyr Ser Leu Leu Val Thr Val Ala Phe Ile Ile Phe Trp Val
    450                 455                 460

Arg Ser Lys Arg Ser Arg Leu Leu His Ser Asp Tyr Met Asn Met Thr
465                 470                 475                 480

Pro Arg Arg Pro Gly Pro Thr Arg Lys His Tyr Gln Pro Tyr Ala Pro
                485                 490                 495

Pro Arg Asp Phe Ala Ala Tyr Arg Ser Arg Val Lys Phe Ser Arg Ser
            500                 505                 510

Ala Asp Ala Pro Ala Tyr Gln Gln Gly Gln Asn Gln Leu Tyr Asn Glu
        515                 520                 525

Leu Asn Leu Gly Arg Arg Glu Glu Tyr Asp Val Leu Asp Lys Arg Arg
    530                 535                 540

Gly Arg Asp Pro Glu Met Gly Gly Lys Pro Arg Arg Lys Asn Pro Gln
545                 550                 555                 560

Glu Gly Leu Tyr Asn Glu Leu Gln Lys Asp Lys Met Ala Glu Ala Tyr
                565                 570                 575

Ser Glu Ile Gly Met Lys Gly Glu Arg Arg Arg Gly Lys Gly His Asp
            580                 585                 590

Gly Leu Tyr Gln Gly Leu Ser Thr Ala Thr Lys Asp Thr Tyr Asp Ala
        595                 600                 605

Leu His Met Gln Ala Leu Pro Pro Arg

610                 615

<210>  15
<211>  505
<212>  PRT
<213>  Artificial Sequence

<220>
<223>  CAR fragment

<400>  15

Phe Ser Arg Ser Ala Asp Ala Pro Ala Tyr Ala Thr Asn Phe Ser Leu
1               5                   10                  15

Leu Lys Gln Ala Gly Asp Val Glu Glu Asn Pro Gly Pro Met Ala Leu
            20                  25                  30

Pro Val Thr Ala Leu Leu Leu Pro Leu Ala Leu Leu Leu His Ala Ala
        35                  40                  45

Arg Pro Asp Ile Gln Met Thr Gln Thr Thr Ser Ser Leu Ser Ala Ser
    50                  55                  60

Leu Gly Asp Arg Val Thr Ile Ser Cys Arg Ala Ser Gln Asp Ile Ser
65                  70                  75                  80

Lys Tyr Leu Asn Trp Tyr Gln Gln Lys Pro Asp Gly Thr Val Lys Leu
                85                  90                  95

Leu Ile Tyr His Thr Ser Arg Leu His Ser Gly Val Pro Ser Arg Phe
            100                 105                 110

Ser Gly Ser Gly Ser Gly Thr Asp Tyr Ser Leu Thr Ile Ser Asn Leu
        115                 120                 125

Glu Gln Glu Asp Ile Ala Thr Tyr Phe Cys Gln Gln Gly Asn Thr Leu
    130                 135                 140

Pro Tyr Thr Phe Gly Gly Gly Thr Lys Leu Glu Ile Thr Gly Gly Gly
145                 150                 155                 160

Gly Ser Gly Gly Gly Gly Ser Gly Gly Gly Gly Ser Glu Val Lys Leu
                165                 170                 175

Gln Glu Ser Gly Pro Gly Leu Val Ala Pro Ser Gln Ser Leu Ser Val
            180                 185                 190

Thr Cys Thr Val Ser Gly Val Ser Leu Pro Asp Tyr Gly Val Ser Trp
        195                 200                 205

Ile Arg Gln Pro Pro Arg Lys Gly Leu Glu Trp Leu Gly Val Ile Trp
    210                 215                 220

Gly Ser Glu Thr Thr Tyr Tyr Asn Ser Ala Leu Lys Ser Arg Leu Thr
225                 230                 235                 240

Ile Ile Lys Asp Asn Ser Lys Ser Gln Val Phe Leu Lys Met Asn Ser
                245                 250                 255

Leu Gln Thr Asp Asp Thr Ala Ile Tyr Tyr Cys Ala Lys His Tyr Tyr
            260                 265                 270

Tyr Gly Gly Ser Tyr Ala Met Asp Tyr Trp Gly Gln Gly Thr Ser Val
        275                 280                 285

Thr Val Ser Ser Glu Ser Lys Tyr Gly Pro Pro Cys Pro Pro Cys Pro
    290                 295                 300

Gly Gln Pro Arg Glu Pro Gln Val Tyr Thr Leu Pro Pro Ser Arg Asp
305                 310                 315                 320

Glu Leu Thr Lys Asn Gln Val Ser Leu Thr Cys Leu Val Lys Gly Phe
                325                 330                 335

Tyr Pro Ser Asp Ile Ala Val Glu Trp Glu Ser Asn Gly Gln Pro Glu
            340                 345                 350

Asn Asn Tyr Lys Thr Thr Pro Pro Val Leu Asp Ser Asp Gly Ser Phe
        355                 360                 365

Phe Leu Tyr Ser Lys Leu Thr Val Asp Lys Ser Arg Trp Gln Gln Gly
    370                 375                 380

Asn Val Phe Ser Cys Ser Val Met His Glu Ala Leu His Asn Ala Tyr
385                 390                 395                 400

Thr Gln Lys Ser Leu Ser Leu Ser Pro Gly Lys Lys Asp Pro Lys Phe
                405                 410                 415

Trp Val Leu Val Val Val Gly Gly Val Leu Ala Cys Tyr Ser Leu Leu
            420                 425                 430

Val Thr Val Ala Phe Ile Ile Phe Trp Val Arg Ser Lys Arg Ser Arg
        435                 440                 445

Leu Leu His Ser Asp Tyr Met Asn Met Thr Pro Arg Arg Pro Gly Pro

450                 455                 460

Thr Arg Lys His Tyr Gln Pro Tyr Ala Pro Pro Arg Asp Phe Ala Ala
465                 470                 475                 480

Tyr Arg Ser Arg Val Lys Phe Ser Arg Ser Ala Asp Ala Pro Ala Tyr
                485                 490                 495

Gln Gln Gly Gln Asn Gln Leu Tyr Asn
            500                 505

<210>  16
<211>  10
<212>  PRT
<213>  Artificial Sequence

<220>
<223>  CAR component

<400>  16

Phe Ser Arg Ser Ala Asp Ala Pro Ala Tyr
1               5                   10

<210>  17
<211>  473
<212>  PRT
<213>  Artificial Sequence

<220>
<223>  CAR component

<400>  17

Ala Thr Asn Phe Ser Leu Leu Lys Gln Ala Gly Asp Val Glu Glu Asn
1               5                   10                  15

Pro Gly Pro Met Ala Leu Pro Val Thr Ala Leu Leu Leu Pro Leu Ala
            20                  25                  30

Leu Leu Leu His Ala Ala Arg Pro Asp Ile Gln Met Thr Gln Thr Thr
        35                  40                  45

Ser Ser Leu Ser Ala Ser Leu Gly Asp Arg Val Thr Ile Ser Cys Arg
    50                  55                  60

Ala Ser Gln Asp Ile Ser Lys Tyr Leu Asn Trp Tyr Gln Gln Lys Pro
65                  70                  75                  80

Asp Gly Thr Val Lys Leu Leu Ile Tyr His Thr Ser Arg Leu His Ser
                85                  90                  95

Gly Val Pro Ser Arg Phe Ser Gly Ser Gly Ser Gly Thr Asp Tyr Ser
        100                 105                 110

Leu Thr Ile Ser Asn Leu Glu Gln Glu Asp Ile Ala Thr Tyr Phe Cys
    115                 120                 125

Gln Gln Gly Asn Thr Leu Pro Tyr Thr Phe Gly Gly Gly Thr Lys Leu
    130                 135                 140

Glu Ile Thr Gly Gly Gly Gly Ser Gly Gly Gly Gly Ser Gly Gly Gly
145                 150                 155                 160

Gly Ser Glu Val Lys Leu Gln Glu Ser Gly Pro Gly Leu Val Ala Pro
                165                 170                 175

Ser Gln Ser Leu Ser Val Thr Cys Thr Val Ser Gly Val Ser Leu Pro
        180                 185                 190

Asp Tyr Gly Val Ser Trp Ile Arg Gln Pro Pro Arg Lys Gly Leu Glu
    195                 200                 205

Trp Leu Gly Val Ile Trp Gly Ser Glu Thr Thr Tyr Tyr Asn Ser Ala
    210                 215                 220

Leu Lys Ser Arg Leu Thr Ile Ile Lys Asp Asn Ser Lys Ser Gln Val
225                 230                 235                 240

Phe Leu Lys Met Asn Ser Leu Gln Thr Asp Asp Thr Ala Ile Tyr Tyr
                245                 250                 255

Cys Ala Lys His Tyr Tyr Tyr Gly Gly Ser Tyr Ala Met Asp Tyr Trp
        260                 265                 270

Gly Gln Gly Thr Ser Val Thr Val Ser Ser Glu Ser Lys Tyr Gly Pro
    275                 280                 285

Pro Cys Pro Pro Cys Pro Gly Gln Pro Arg Glu Pro Gln Val Tyr Thr
    290                 295                 300

Leu Pro Pro Ser Arg Asp Glu Leu Thr Lys Asn Gln Val Ser Leu Thr
305                 310                 315                 320

Cys Leu Val Lys Gly Phe Tyr Pro Ser Asp Ile Ala Val Glu Trp Glu
                325                 330                 335

Ser Asn Gly Gln Pro Glu Asn Asn Tyr Lys Thr Thr Pro Pro Val Leu
        340                 345                 350

Asp Ser Asp Gly Ser Phe Phe Leu Tyr Ser Lys Leu Thr Val Asp Lys
        355                 360                 365

Ser Arg Trp Gln Gln Gly Asn Val Phe Ser Cys Ser Val Met His Glu
    370                 375                 380

Ala Leu His Asn Ala Tyr Thr Gln Lys Ser Leu Ser Leu Ser Pro Gly
385                 390                 395                 400

Lys Lys Asp Pro Lys Phe Trp Val Leu Val Val Val Gly Gly Val Leu
                405                 410                 415

Ala Cys Tyr Ser Leu Leu Val Thr Val Ala Phe Ile Ile Phe Trp Val
            420                 425                 430

Arg Ser Lys Arg Ser Arg Leu Leu His Ser Asp Tyr Met Asn Met Thr
        435                 440                 445

Pro Arg Arg Pro Gly Pro Thr Arg Lys His Tyr Gln Pro Tyr Ala Pro
    450                 455                 460

Pro Arg Asp Phe Ala Ala Tyr Arg Ser
465                 470

<210>  18
<211>  8
<212>  PRT
<213>  Artificial Sequence

<220>
<223>  CAR component

<400>  18

Arg Val Lys Phe Ser Arg Ser Ala
1               5

<210>  19
<211>  14
<212>  PRT
<213>  Artificial Sequence

<220>
<223>  CAR component

<400>  19

Asp Ala Pro Ala Tyr Gln Gln Gly Gln Asn Gln Leu Tyr Asn
1               5                   10

<210>  20
<211>  595

<212>  PRT
<213>  Artificial Sequence

<220>
<223>  CAR fragment

<400>  20

Phe Ser Arg Ser Ala Asp Ala Pro Ala Tyr Ala Thr Asn Phe Ser Leu
1               5                   10                  15

Leu Lys Gln Ala Gly Asp Val Glu Glu Asn Pro Gly Pro Met Ala Leu
            20                  25                  30

Pro Val Thr Ala Leu Leu Leu Pro Leu Ala Leu Leu Leu His Ala Ala
        35                  40                  45

Arg Pro Asp Ile Gln Met Thr Gln Thr Thr Ser Ser Leu Ser Ala Ser
    50                  55                  60

Leu Gly Asp Arg Val Thr Ile Ser Cys Arg Ala Ser Gln Asp Ile Ser
65                  70                  75                  80

Lys Tyr Leu Asn Trp Tyr Gln Gln Lys Pro Asp Gly Thr Val Lys Leu
                85                  90                  95

Leu Ile Tyr His Thr Ser Arg Leu His Ser Gly Val Pro Ser Arg Phe
            100                 105                 110

Ser Gly Ser Gly Ser Gly Thr Asp Tyr Ser Leu Thr Ile Ser Asn Leu
        115                 120                 125

Glu Gln Glu Asp Ile Ala Thr Tyr Phe Cys Gln Gln Gly Asn Thr Leu
    130                 135                 140

Pro Tyr Thr Phe Gly Gly Gly Thr Lys Leu Glu Ile Thr Gly Gly Gly
145                 150                 155                 160

Gly Ser Gly Gly Gly Gly Ser Gly Gly Gly Gly Ser Glu Val Lys Leu
                165                 170                 175

Gln Glu Ser Gly Pro Gly Leu Val Ala Pro Ser Gln Ser Leu Ser Val
            180                 185                 190

Thr Cys Thr Val Ser Gly Val Ser Leu Pro Asp Tyr Gly Val Ser Trp
        195                 200                 205

Ile Arg Gln Pro Pro Arg Lys Gly Leu Glu Trp Leu Gly Val Ile Trp
    210                 215                 220

Gly Ser Glu Thr Thr Tyr Tyr Asn Ser Ala Leu Lys Ser Arg Leu Thr
225                 230                 235                 240

Ile Ile Lys Asp Asn Ser Lys Ser Gln Val Phe Leu Lys Met Asn Ser
                245                 250                 255

Leu Gln Thr Asp Asp Thr Ala Ile Tyr Tyr Cys Ala Lys His Tyr Tyr
            260                 265                 270

Tyr Gly Gly Ser Tyr Ala Met Asp Tyr Trp Gly Gln Gly Thr Ser Val
        275                 280                 285

Thr Val Ser Ser Glu Ser Lys Tyr Gly Pro Pro Cys Pro Pro Cys Pro
    290                 295                 300

Gly Gln Pro Arg Glu Pro Gln Val Tyr Thr Leu Pro Pro Ser Arg Asp
305                 310                 315                 320

Glu Leu Thr Lys Asn Gln Val Ser Leu Thr Cys Leu Val Lys Gly Phe
                325                 330                 335

Tyr Pro Ser Asp Ile Ala Val Glu Trp Glu Ser Asn Gly Gln Pro Glu
            340                 345                 350

Asn Asn Tyr Lys Thr Thr Pro Pro Val Leu Asp Ser Asp Gly Ser Phe
        355                 360                 365

Phe Leu Tyr Ser Lys Leu Thr Val Asp Lys Ser Arg Trp Gln Gln Gly
    370                 375                 380

Asn Val Phe Ser Cys Ser Val Met His Glu Ala Leu His Asn Ala Tyr
385                 390                 395                 400

Thr Gln Lys Ser Leu Ser Leu Ser Pro Gly Lys Lys Asp Pro Lys Phe
                405                 410                 415

Trp Val Leu Val Val Val Gly Gly Val Leu Ala Cys Tyr Ser Leu Leu
            420                 425                 430

Val Thr Val Ala Phe Ile Ile Phe Trp Val Arg Ser Lys Arg Ser Arg
        435                 440                 445

Leu Leu His Ser Asp Tyr Met Asn Met Thr Pro Arg Arg Pro Gly Pro
    450                 455                 460

Thr Arg Lys His Tyr Gln Pro Tyr Ala Pro Pro Arg Asp Phe Ala Ala
465                 470                 475                 480

Tyr Arg Ser Arg Val Lys Phe Ser Arg Ser Ala Asp Ala Pro Ala Tyr
                485                 490                 495

Gln Gln Gly Gln Asn Gln Leu Tyr Asn Glu Leu Asn Leu Gly Arg Arg
            500                 505                 510

Glu Glu Tyr Asp Val Leu Asp Lys Arg Arg Gly Arg Asp Pro Glu Met
        515                 520                 525

Gly Gly Lys Pro Arg Arg Lys Asn Pro Gln Glu Gly Leu Tyr Asn Glu
    530                 535                 540

Leu Gln Lys Asp Lys Met Ala Glu Ala Tyr Ser Glu Ile Gly Met Lys
545                 550                 555                 560

Gly Glu Arg Arg Arg Gly Lys Gly His Asp Gly Leu Tyr Gln Gly Leu
                565                 570                 575

Ser Thr Ala Thr Lys Asp Thr Tyr Asp Ala Leu His Met Gln Ala Leu
            580                 585                 590

Pro Pro Arg
        595

<210> 21
<211> 112
<212> PRT
<213> Homo sapiens

<400> 21

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  22
<211>  239
<212>  PRT
<213>  Artificial Sequence

<220>
<223>  enhanced GFP

<400>  22

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  23
<211>  25
<212>  PRT
<213>  Artificial Sequence

<220>
<223>  CAR component

<400>  23

Ile Gln Asn Pro Asp Pro Ala Val Tyr Gln Leu Arg Asp Ser Lys Ser
1               5                   10                  15

Ser Asp Lys Ser Val Cys Leu Phe Thr
            20                  25

<210>  24
<211>  2221
<212>  DNA
<213>  Artificial Sequence

<220>
<223>  CAR fragment

<400>  24
ggcaggagct ctgtgcacag agaacagcct catctgctcg ccttgtttcc acctcccctc      60

ccattgcccc aggttctttg gccccacagc ggccacatct gccgttggtg ccaataggtt     120

ttccaggagc tggttgaggt gggagggagg gagagggttg tgatcaggct gaggcatggg     180

gattggatat agtctccgtg tcatgattta tttggtcagt cagtcctagt gccaccctgg     240

ggtaatgggg atgtgttctc gtcaccttgg gcctggctga ccagctttat ctcttggcac     300

agaggcacag agctttggcc tgctggatcc caaactctgc tacctgctgg atggaatcct     360

cttcatctat cgtgtcattc tcactgcctt gttcctgaga gccaccaact tctctctttt     420

gaagcaggcc ggagatgtgg aagaaaatcc tgggcccatg gctcttcctg tgactgccct     480

tctgctgccc ctggctctcc tgctccatgc tgcccggcca gacatccaga tgacacagac     540

aaccagcagc ctcagcgcca gcctggggga cagagtgacc attagctgcc gggcctctca     600

ggacatcagc aaatacctga actggtacca gcagaaacca gatggcactg tcaagctgct     660

gatttaccac acctccaggc tccacagcgg cgtgcccagt cgcttcagcg gcagtgggag     720

cgggacagat tattccctca caatctccaa cctggagcag gaagatattg ccacatactt 780

ctgccagcaa ggcaacaccc tgccatacac atttggaggc ggcaccaaat tggagatcac 840

cggcggtggt ggatctggag gagggggcag cggaggtggc ggctctgagg tgaaactgca 900

ggagagtggc cctggcctgg tggctcccag ccagagcctt tctgtcacct gcaccgtgtc 960

tggggtgtcc ctgcctgact atggagtctc ctggatccgg cagcctccaa gaaaaggact 1020

ggaatggctg ggcgtcatct ggggaagtga gaccacctac tataattcag ccctcaagtc 1080

ccggctcacc atcattaagg acaactccaa atcccaggtg ttcctgaaga tgaattctct 1140

ccagactgat gacacagcca tctactactg tgccaagcat tattattacg gcgggtccta 1200

tgccatggac tactggggcc aggggaccag tgtcactgtt tcttctgaaa gcaagtatgg 1260

ccccccatgc cctccctgcc ccggacagcc gcgggagccc caggtctaca ccctcccacc 1320

ctccagagat gagctgacca agaaccaggt ttcactgaca tgcctggtga agggcttcta 1380

cccctctgac attgctgtgg agtgggagag caatgggcag ccagagaaca actacaagac 1440

cacacctcct gtcctggaca gcgacggctc cttcttcctc tacagtaagc tcactgtgga 1500

caagagccgc tggcagcagg gcaatgtctt ctcctgcagc gtgatgcacg aggccctgca 1560

caatgcctat acccagaagt cactctctct gagccctgga aagaaggatc ccaagttttg 1620

ggtcctcgtg gtggtcgggg gtgtgctggc ctgctacagc ttgctggtca cagtggcctt 1680

catcatcttc tgggtgcgct ccaagaggag ccggctgctt cacagtgatt acatgaacat 1740

gacccccagg aggccaggac ccaccaggaa gcactaccag ccctacgctc ccccgcggga 1800

ctttgctgct taccgcagca gagtgaaggt gggtaccact gggctttggg aggagggcac 1860

ggggtccccc acttgatgga tgttcagagg ggccttggtc ttggaaggtc tcaagctcgg 1920

gtggtgcctg gggcttggta tccaggagca aagcaaggac cagccaagtg tgtgccttga 1980

gtgggctgag gaggaggtgg cagtgtctgg ctgagatgga cagggtagga gggagagcct 2040

ggtgctaggc acctccatga caagccgtac aaatgtgtgc acatcagagt gtcccaggga 2100

aggcgatgct actggtgaca aaggggctta cactcaggca gaggtccttc tttccaagtg 2160

tgaatgaagg ccatgttagc ctttctcttg aaaaggccct ttcctcatct gtaactgggg 2220

a 2221

<210> 25
<211> 400
<212> DNA
<213> Artificial Sequence

<220>
<223> CAR construct

<400> 25

ggcaggagct ctgtgcacag agaacagcct catctgctcg ccttgtttcc acctcccctc 60

ccattgcccc aggttctttg gccccacagc ggccacatct gccgttggtg ccaataggtt 120

ttccaggagc tggttgaggt gggagggagg gagagggttg tgatcaggct gaggcatggg 180

gattggatat agtctccgtg tcatgattta tttggtcagt cagtcctagt gccaccctgg 240

ggtaatgggg atgtgttctc gtcaccttgg gcctggctga ccagctttat ctcttggcac 300

agaggcacag agctttggcc tgctggatcc caaactctgc tacctgctgg atggaatcct 360

cttcatctat cgtgtcattc tcactgcctt gttcctgaga 400

<210> 26
<211> 57
<212> DNA
<213> Artificial Sequence

<220>
<223> CAR component

<400> 26
gccaccaact tctctctttt gaagcaggcc ggagatgtgg aagaaaatcc tgggccc 57

<210> 27
<211> 63
<212> DNA
<213> Artificial Sequence

<220>
<223> CAR component

<400> 27
atggctcttc ctgtgactgc ccttctgctg cccctggctc tcctgctcca tgctgcccgg 60

cca 63

<210> 28
<211> 321
<212> DNA
<213> Artificial Sequence

<220>
<223> CAR component

<400> 28
gacatccaga tgacacagac aaccagcagc ctcagcgcca gcctggggga cagagtgacc 60

attagctgcc gggcctctca ggacatcagc aaatacctga actggtacca gcagaaacca 120

gatggcactg tcaagctgct gatttaccac acctccaggc tccacagcgg cgtgcccagt 180

cgcttcagcg gcagtgggag cgggacagat tattccctca caatctccaa cctggagcag 240

gaagatattg ccacatactt ctgccagcaa ggcaacaccc tgccatacac atttggaggc 300

ggcaccaaat tggagatcac c 321

<210>  29
<211>  45
<212>  DNA
<213>  Artificial Sequence

<220>
<223>  CAR component

<400>  29
ggcggtggtg gatctggagg agggggcagc ggaggtggcg gctct                45


<210>  30
<211>  360
<212>  DNA
<213>  Artificial Sequence

<220>
<223>  CAR component

<400>  30
gaggtgaaac tgcaggagag tggccctggc ctggtggctc ccagccagag cctttctgtc      60

acctgcaccg tgtctggggt gtccctgcct gactatggag tctcctggat ccggcagcct     120

ccaagaaaag gactggaatg gctgggcgtc atctggggaa gtgagaccac ctactataat     180

tcagccctca agtcccggct caccatcatt aaggacaact ccaaatccca ggtgttcctg     240

aagatgaatt ctctccagac tgatgacaca gccatctact actgtgccaa gcattattat     300

tacggcgggt cctatgccat ggactactgg ggccagggga ccagtgtcac tgtttcttct     360


<210>  31
<211>  36
<212>  DNA
<213>  Artificial Sequence

<220>
<223>  CAR component

<400>  31
gaaagcaagt atggcccccc atgccctccc tgcccc                          36


<210>  32
<211>  321
<212>  DNA
<213>  Artificial Sequence

<220>
<223>  CAR component

<400>  32
ggacagccgc gggagcccca ggtctacacc ctcccaccct ccagagatga gctgaccaag      60

aaccaggttt cactgacatg cctggtgaag ggcttctacc cctctgacat tgctgtggag     120

tgggagagca atgggcagcc agagaacaac tacaagacca cacctcctgt cctggacagc     180

gacggctcct tcttcctcta cagtaagctc actgtggaca agagccgctg gcagcagggc     240

aatgtcttct cctgcagcgt gatgcacgag gccctgcaca atgcctatac ccagaagtca 300

ctctctctga gccctggaaa g 321

<210> 33
<211> 12
<212> DNA
<213> Artificial Sequence

<220>
<223> CAR component

<400> 33
aaggatccca ag 12

<210> 34
<211> 81
<212> DNA
<213> Homo sapiens

<400> 34
ttttgggtcc tcgtggtggt cggggggtgg ctggcctgct acagcttgct ggtcacagtg 60

gccttcatca tcttctgggt g 81

<210> 35
<211> 123
<212> DNA
<213> Homo sapiens

<400> 35
cgctccaaga ggagccggct gcttcacagt gattacatga acatgacccc caggaggcca 60

gggcccacca ggaagcacta ccagccctac gctcccccgc gggactttgc tgcttaccgc 120

agc 123

<210> 36
<211> 402
<212> DNA
<213> Artificial Sequence

<220>
<223> CAR construct

<400> 36
agagtgaagg tgggtaccac tgggctttgg gaggagggca cggggtcccc cacttgatgg 60

atgttcagag gggccttggt cttggaaggt ctcaagctcg ggtggtgcct ggggcttggt 120

atccaggagc aaagcaagga ccagccaagt gtgtgccttg agtgggctga ggaggaggtg 180

gcagtgtctg gctgagatgg acagggtagg agggagagcc tggtgctagg cacctccatg 240

acaagccgta caaatgtgtg cacatcagag tgtcccaggg aaggcgatgc tactggtgac 300

aaaggggctt acactcaggc agaggtcctt ctttccaagt gtgaatgaag gccatgttag 360

cctttctctt gaaaaggccc tttcctcatc tgtaactggg ga 402

<210>  37
<211>  2812
<212>  DNA
<213>  Artificial Sequence

<220>
<223>  CAR fragment

<400>  37
ggcaggagct ctgtgcacag agaacagcct catctgctcg ccttgtttcc acctcccctc      60

ccattgcccc aggttctttg gccccacagc ggccacatct gccgttggtg ccaataggtt     120

ttccaggagc tggttgaggt gggagggagg gagagggttg tgatcaggct gaggcatggg     180

gattggatat agtctccgtg tcatgattta tttggtcagt cagtcctagt gccaccctgg     240

ggtaatgggg atgtgttctc gtcaccttgg gcctggctga ccagctttat ctcttggcac     300

agaggcacag agctttggcc tgctggatcc caaactctgc tacctgctgg atggaatcct     360

cttcatctat cgtgtcattc tcactgcctt gttcctgaga gccaccaact tctctctttt     420

gaagcaggcc ggagatgtgg aagaaaatcc tgggcccatg gctcttcctg tgactgccct     480

tctgctgccc ctggctctcc tgctccatgc tgcccggcca gacatccaga tgacacagac     540

aaccagcagc ctcagcgcca gcctggggga cagagtgacc attagctgcc gggcctctca     600

ggacatcagc aaatacctga actggtacca gcagaaacca gatggcactg tcaagctgct     660

gatttaccac acctccaggc tccacagcgg cgtgcccagt cgcttcagcg gcagtgggag     720

cgggacagat tattccctca caatctccaa cctggagcag gaagatattg ccacatactt     780

ctgccagcaa ggcaacaccc tgccatacac atttggaggc ggcaccaaat tggagatcac     840

cggcggtggt ggatctggag gagggggcag cggaggtggc ggctctgagg tgaaactgca     900

ggagagtggc cctggcctgg tggctcccag ccagagcctt tctgtcacct gcaccgtgtc     960

tggggtgtcc ctgcctgact atggagtctc ctggatccgg cagcctccaa gaaaaggact    1020

ggaatggctg ggcgtcatct ggggaagtga gaccacctac tataattcag ccctcaagtc    1080

ccggctcacc atcattaagg acaactccaa atcccaggtg ttcctgaaga tgaattctct    1140

ccagactgat gacacagcca tctactactg tgccaagcat tattattacg gcgggtccta    1200

tgccatggac tactggggcc aggggaccag tgtcactgtt tcttctgaaa gcaagtatgg    1260

ccccccatgc cctccctgcc ccggacagcc gcgggagccc caggtctaca ccctcccacc    1320

ctccagagat gagctgacca agaaccaggt ttcactgaca tgcctggtga agggcttcta    1380

cccctctgac attgctgtgg agtgggagag caatgggcag ccagagaaca actacaagac    1440

cacacctcct gtcctggaca gcgacggctc cttcttcctc tacagtaagc tcactgtgga    1500

caagagccgc tggcagcagg gcaatgtctt ctcctgcagc gtgatgcacg aggccctgca    1560

caatgcctat acccagaagt cactctctct gagccctgga aagaaggatc ccaagttttg 1620

ggtcctcgtg gtggtcgggg gtgtgctggc ctgctacagc ttgctggtca cagtggcctt 1680

catcatcttc tgggtgcgct ccaagaggag ccggctgctt cacagtgatt acatgaacat 1740

gacccccagg aggccaggac ccaccaggaa gcactaccag ccctacgctc ccccgcggga 1800

ctttgctgct taccgcagca gggtcaaatt ttctagatct gcagatgcgc cggcctatca 1860

acaaggccag aaccagctct ataacgagct caatctagga cgaagagagg agtacgatgt 1920

tttggacaag agacgtggcc gggaccctga gatgggggga aagccgagaa ggaagaaccc 1980

tcaggaaggc ctgtacaatg aactgcagaa agataagatg gcggaggcct acagtgagat 2040

tgggatgaaa ggcgagcgcc ggaggggcaa ggggcacgat ggcctttacc agggtctcag 2100

tacagccacc aaggacacct acgacgccct tcacatgcag gccctgcccc ctcgctaata 2160

agaattctaa ctagagctcg ctgatcagcc tcgactgtgc cttctagttg ccagccatct 2220

gttgtttgcc cctcccccgt gccttccttg accctggaag gtgccactcc cactgtcctt 2280

tcctaataaa atgaggaaat tgcatcgcat tgtctgagta ggtgtcattc tattctgggg 2340

ggtggggtgg ggcaggacag caagggggag gattgggaag agaatagcag gcatgctggg 2400

gagcggccgc agagtgaagg tgggtaccac tgggctttgg gaggagggca cggggtcccc 2460

cacttgatgg atgttcagag gggccttggt cttggaaggt ctcaagctcg ggtggtgcct 2520

ggggcttggt atccaggagc aaagcaagga ccagccaagt gtgtgccttg agtgggctga 2580

ggaggaggtg gcagtgtctg gctgagatgg acagggtagg agggagagcc tggtgctagg 2640

cacctccatg acaagccgta caaatgtgtg cacatcagag tgtcccaggg aaggcgatgc 2700

tactggtgac aaaggggctt acactcaggc agaggtcctt ctttccaagt gtgaatgaag 2760

gccatgttag cctttctctt gaaaaggccc tttcctcatc tgtaactggg ga 2812

<210> 38
<211> 2258
<212> DNA
<213> Artificial Sequence

<220>
<223> CAR fragment

<400> 38
attgtcctca gagtccccgg aggaagggat cctagagaat actcatgtcc ccgggggcgc 60

agaccaagaa ccttatacct ctgcgcacgc gcgagggcgc tagcccggga agaataaact 120

ccagcgggtc ctgggccagc gtgatgaatg cacgtgtcac aggcgggagg aaaaaggaca 180

actgggctca gaaaaagggg ctgccacgcc ccggcccctg caagggctca gccccaggag 240

gcagggcagc attgttagtt gccaaggagc ggagtagggc tgtccctcgg gtcgccgcgc 300

gccctggcac cccggctccc ctgcgcgccc tcgcggagca gtaacgcgct ttgctttctg 360

tgttgcagtt cagcaggagc gcagacgccc ccgcgtacgc caccaacttc tctcttttga 420

agcaggccgg agatgtggaa gaaaatcctg ggcccatggc tcttcctgtg actgcccttc 480

tgctgcccct ggctctcctg ctccatgctg cccggccaga catccagatg acacagacaa 540

ccagcagcct cagcgccagc ctgggggaca gagtgaccat tagctgccgg gcctctcagg 600

acatcagcaa atacctgaac tggtaccagc agaaaccaga tggcactgtc aagctgctga 660

tttaccacac ctccaggctc cacagcggcg tgcccagtcg cttcagcggc agtgggagcg 720

ggacagatta ttccctcaca atctccaacc tggagcagga agatattgcc acatacttct 780

gccagcaagg caacaccctg ccatacacat ttggaggcgg caccaaattg gagatcaccg 840

gcggtggtgg atctggagga gggggcagcg gaggtggcgg ctctgaggtg aaactgcagg 900

agagtggccc tggcctggtg gctcccagcc agagcctttc tgtcacctgc accgtgtctg 960

gggtgtccct gcctgactat ggagtctcct ggatccggca gcctccaaga aaaggactgg 1020

aatggctggg cgtcatctgg ggaagtgaga ccacctacta taattcagcc ctcaagtccc 1080

ggctcaccat cattaaggac aactccaaat cccaggtgtt cctgaagatg aattctctcc 1140

agactgatga cacagccatc tactactgtg ccaagcatta ttattacggc gggtcctatg 1200

ccatggacta ctggggccag gggaccagtg tcactgtttc ttctgaaagc aagtatggcc 1260

ccccatgccc tccctgcccc ggacagccgc gggagcccca ggtctacacc ctcccaccct 1320

ccagagatga gctgaccaag aaccaggttt cactgacatg cctggtgaag ggcttctacc 1380

cctctgacat tgctgtggag tgggagagca atgggcagcc agagaacaac tacaagacca 1440

cacctcctgt cctggacagc gacggctcct tcttcctcta cagtaagctc actgtggaca 1500

agagccgctg gcagcagggc aatgtcttct cctgcagcgt gatgcacgag gccctgcaca 1560

atgcctatac ccagaagtca ctctctctga gccctggaaa gaaggatccc aagttttggg 1620

tcctcgtggt ggtcgggggt gtgctggcct gctacagctt gctggtcaca gtggccttca 1680

tcatcttctg ggtgcgctcc aagaggagcc ggctgcttca cagtgattac atgaacatga 1740

cccccaggag gccaggaccc accaggaagc actaccagcc ctacgctccc ccgcgggact 1800

ttgctgctta ccgcagcagg gtcaaatttt ctagatctgc agatgcgccg gcctatcaac 1860

aaggccagaa ccagctctat aacgtaagtc agcctcgccg tagaccctcc ggaaaggaag 1920

ggcagcctcc tcctcggacc tagggaacgc gcccgccaga gtcttgggtt taggtttggt 1980

ccctagctat cacctctgcg acccggctgc aacttgttta acctcagttt tctaacccac 2040

aaaacaggtg tagtaattgt acctgtctca aagggcgcca taaagattac atgctttaat 2100

gtcttgatgc atggaacgag cttagtctag tgcctgccac tagtgagccc ccaagccatc 2160

agcaatttgc aaaggctaga ggggggtgga gaaggaatgt attagaatct tcccagagga 2220

ggggaaagcg atttcacact ccctgtgcaa tgggactg                              2258

<210>  39
<211>  398
<212>  DNA
<213>  Artificial Sequence

<220>
<223>  CAR construct

<400>  39
attgtcctca gagtccccgg aggaagggat cctagagaat actcatgtcc ccgggggcgc      60

agaccaagaa ccttatacct ctgcgcacgc gcgagggcgc tagcccggga agaataaact     120

ccagcgggtc ctgggccagc gtgatgaatg cacgtgtcac aggcgggagg aaaaaggaca     180

actgggctca gaaaaagggg ctgccacgcc ccggcccctg caagggctca gccccaggag     240

gcagggcagc attgttagtt gccaaggagc ggagtagggc tgtccctcgg gtcgccgcgc     300

gccctggcac cccggctccc ctgcgcgccc tcgcggagca gtaacgcgct ttgctttctg     360

tgttgcagtt cagcaggagc gcagacgccc ccgcgtac                             398

<210>  40
<211>  1419
<212>  DNA
<213>  Artificial Sequence

<220>
<223>  CAR body

<400>  40
gccaccaact tctctctttt gaagcaggcc ggagatgtgg aagaaaatcc tgggcccatg      60

gctcttcctg tgactgccct tctgctgccc ctggctctcc tgctccatgc tgcccggcca     120

gacatccaga tgacacagac aaccagcagc ctcagcgcca gcctggggga cagagtgacc     180

attagctgcc gggcctctca ggacatcagc aaatacctga actggtacca gcagaaacca     240

gatggcactg tcaagctgct gatttaccac acctccaggc tccacagcgg cgtgcccagt     300

cgcttcagcg gcagtgggag cgggacagat tattccctca caatctccaa cctggagcag     360

gaagatattg ccacatactt ctgccagcaa ggcaacaccc tgccatacac atttggaggc     420

ggcaccaaat tggagatcac cggcggtggt ggatctggag gagggggcag cggaggtggc     480

ggctctgagg tgaaactgca ggagagtggc cctggcctgg tggctcccag ccagagcctt     540

tctgtcacct gcaccgtgtc tggggtgtcc ctgcctgact atggagtctc ctggatccgg     600

cagcctccaa gaaaaggact ggaatggctg ggcgtcatct ggggaagtga gaccacctac     660

tataattcag ccctcaagtc ccggctcacc atcattaagg acaactccaa atcccaggtg     720

ttcctgaaga tgaattctct ccagactgat gacacagcca tctactactg tgccaagcat     780

tattattacg gcgggtccta tgccatggac tactggggcc aggggaccag tgtcactgtt     840

tcttctgaaa gcaagtatgg ccccccatgc cctccctgcc ccggacagcc gcgggagccc 900

caggtctaca ccctcccacc ctccagagat gagctgacca agaaccaggt ttcactgaca 960

tgcctggtga agggcttcta cccctctgac attgctgtgg agtgggagag caatgggcag 1020

ccagagaaca actacaagac cacacctcct gtcctggaca gcgacggctc cttcttcctc 1080

tacagtaagc tcactgtgga caagagccgc tggcagcagg gcaatgtctt ctcctgcagc 1140

gtgatgcacg aggccctgca caatgcctat acccagaagt cactctctct gagccctgga 1200

aagaaggatc ccaagttttg ggtcctcgtg gtggtcgggg gtgtgctggc ctgctacagc 1260

ttgctggtca cagtggcctt catcatcttc tgggtgcgct ccaagaggag ccggctgctt 1320

cacagtgatt acatgaacat gacccccagg aggccaggac ccaccaggaa gcactaccag 1380

ccctacgctc ccccgcggga ctttgctgct taccgcagc 1419

<210> 41
<211> 24
<212> DNA
<213> Artificial Sequence

<220>
<223> CAR component

<400> 41
agggtcaaat tttctagatc tgca 24

<210> 42
<211> 417
<212> DNA
<213> Artificial Sequence

<220>
<223> CAR construct

<400> 42
gatgcgccgg cctatcaaca aggccagaac cagctctata acgtaagtca gcctcgccgt 60

agaccctccg gaaaggaagg gcagcctcct cctcggacct agggaacgcg cccgccagag 120

tcttgggttt aggtttggtc cctagctatc acctctgcga cccggctgca acttgtttaa 180

cctcagtttt ctaacccaca aaacaggtgt agtaattgta cctgtctcaa agggcgccat 240

aaagattaca tgctttaatg tcttgatgca tggaacgagc ttagtctagt gcctgccact 300

agtgagcccc caagccatca gcaatttgca aaggctagag gggggtggag aaggaatgta 360

ttagaatctt cccagaggag gggaaagcga tttcacactc cctgtgcaat gggactg 417

<210> 43
<211> 399
<212> DNA
<213> Artificial Sequence

<220>
<223>  CAR construct

<400>  43
caaggccaga accagctcta taacgtaagt cagcctcgcc gtagaccctc cggaaaggaa      60

gggcagcctc ctcctcggac ctagggaacg cgcccgccag agtcttgggt ttaggtttgg     120

tccctagcta tcacctctgc gacccggctg caacttgttt aacctcagtt ttctaaccca     180

caaaacaggt gtagtaattg tacctgtctc aaagggcgcc ataaagatta catgctttaa     240

tgtcttgatg catggaacga gcttagtcta gtgcctgcca ctagtgagcc cccaagccat     300

cagcaatttg caaaggctag aggggggtgg agaaggaatg tattagaatc ttcccagagg     360

aggggaaagc gatttcacac tccctgtgca atgggactg                            399


<210>  44
<211>  339
<212>  DNA
<213>  Homo sapiens

<400>  44
agggtcaaat tttctagatc tgcagatgcg ccggcctatc aacaaggcca gaaccagctc      60

tataacgagc tcaatctagg acgaagagag gagtacgatg ttttggacaa gagacgtggc     120

cgggaccctg agatgggggg aaagccgaga aggaagaacc ctcaggaagg cctgtacaat     180

gaactgcaga aagataagat ggcggaggcc tacagtgaga ttgggatgaa aggcgagcgc     240

cggaggggca aggggcacga tggcctttac cagggtctca gtacagccac caaggacacc     300

tacgacgccc ttcacatgca ggccctgccc cctcgctaa                            339


<210>  45
<211>  252
<212>  DNA
<213>  Artificial Sequence

<220>
<223>  CAR construct

<400>  45
taagaattct aactagagct cgctgatcag cctcgactgt gccttctagt tgccagccat      60

ctgttgtttg cccctccccc gtgccttcct tgaccctgga aggtgccact cccactgtcc     120

tttcctaata aaatgaggaa attgcatcgc attgtctgag taggtgtcat tctattctgg     180

ggggtggggt ggggcaggac agcaaggggg aggattggga agagaatagc aggcatgctg     240

gggagcggcc gc                                                         252


<210>  46
<211>  720
<212>  DNA
<213>  Artificial Sequence

<220>
<223>  enhanced GFP

<400>  46
atggtatcca agggtgaaga attgttcact ggcgtagttc ctatcttggt ggagctcgac      60

ggagatgtca atggacacaa gttttcagtc agtggcgaag gtgagggtga tgcaacttac     120

gggaagctta cactgaaatt catttgtact actggcaagt tgccggtgcc atggcccacg     180

ctggtaacga cccttaccta cggtgtccaa tgtttttctc gataccccga tcatatgaaa     240

cagcatgact tttttaaatc tgctatgccc gagggatacg tacaggaaag gactatattt     300

tttaaggacg atggtaacta taagacccgc gccgaagtca agtttgaggg agatactctt     360

gttaatcgaa tcgagctcaa ggggatagat tttaaggagg atggcaacat tctcggccac     420

aagctggagt acaattacaa tagtcacaat gtttacataa tggctgataa gcaaaaaaac     480

ggcatcaaag ttaattttaa aattaggcat aatatagaag acggttcagt tcaactcgcc     540

gaccactacc agcagaatac ccctattggc gatgggccgg ttctcctgcc tgacaaccat     600

tacctctcaa cacagtctgc tttgagtaaa gaccctaacg agaagcgaga tcatatggtc     660

ttgctcgagt tcgtaacggc cgccggtata accctgggca tggatgagct ctacaaataa     720


<210>  47
<211>  400
<212>  DNA
<213>  Artificial Sequence

<220>
<223>  CAR construct

<400>  47
cctattaaat aaaagaataa gcagtattat taagtagccc tgcatttcag gtttccttga      60

gtggcaggcc aggcctggcc gtgaacgttc actgaaatca tggcctcttg gccaagattg     120

atagcttgtg cctgtccctg agtcccagtc catcacgagc agctggtttc taagatgcta     180

tttcccgtat aaagcatgag accgtgactt gccagcccca cagagccccg cccttgtcca     240

tcactggcat ctggactcca gcctgggttg gggcaaagag ggaaatgaga tcatgtccta     300

accctgatcc tcttgtccca cagatatcca gaaccctgac cctgccgtgt accagctgag     360

agactctaaa tccagtgaca agtctgtctg cctattcacc                           400


<210>  48
<211>  399
<212>  DNA
<213>  Artificial Sequence

<220>
<223>  CAR construct

<400>  48
attttgattc tcaaacaaat gtgtcacaaa gtaaggattc tgatgtgtat atcacagaca      60

aaactgtgct agacatgagg tctatggact tcaagagcaa cagtgctgtg gcctggagca 120

acaaatctga ctttgcatgt gcaaacgcct tcaacaacag cattattcca gaagacacct 180

tcttccccag cccaggtaag ggcagctttg gtgccttcgc aggctgtttc cttgcttcag 240

gaatggccag gttctgccca gagctctggt caatgatgtc taaaactcct ctgattggtg 300

gtctcggcct tatccattgc caccaaaacc ctctttttac taagaaacag tgagccttgt 360

tctggcagtc cagagaatga cacgggaaaa aagcagatg 399

<210> 49
<211> 399
<212> DNA
<213> Artificial Sequence

<220>
<223> CAR construct

<400> 49
   accgtttttc tggacaaccc caaagtaccc cgtctccctg gctttagcca cctctccatc 60
   ctcttgcttt ctttgcctgg acaccccgtt ctcctgtgga ttcgggtcac ctctcactcc 120
   tttcatttgg gcagctcccc tacccccctt acctctctag tctgtgctag ctcttccagc 180
   cccctgtcat ggcatcttcc aggggtccga gagctcagct agtcttcttc ctccaacccg 240
   ggcccctatg tccacttcag gacagcatgt ttgctgcctc cagggatcct gtgtccccga 300
   gctgggacca ccttatattc ccagggccgg ttaatgtggc tctggttctg ggtactttta 360
   tctgtcccct ccaccccaca gtggggccac tagggacag 399

<210> 50
<211> 2221
<212> DNA
<213> Artificial Sequence

<220>
<223> Promoter sequence

<400> 50
ggcaggagct ctgtgcacag agaacagcct catctgctcg ccttgtttcc acctcccctc 60

ccattgcccc aggttctttg gccccacagc ggccacatct gccgttggtg ccaataggtt 120

ttccaggagc tggttgaggt gggagggagg gagagggttg tgatcaggct gaggcatggg 180

gattggatat agtctccgtg tcatgattta tttggtcagt cagtcctagt gccaccctgg 240

ggtaatgggg atgtgttctc gtcaccttgg gcctggctga ccagctttat ctcttggcac 300

agaggcacag agctttggcc tgctggatcc caaactctgc tacctgctgg atggaatcct 360

cttcatctat cgtgtcattc tcactgcctt gttcctgaga gccaccaact tctctctttt 420

gaagcaggcc ggagatgtgg aagaaaatcc tgggcccatg gctcttcctg tgactgccct 480

tctgctgccc ctggctctcc tgctccatgc tgcccggcca gacatccaga tgacacagac 540

aaccagcagc ctcagcgcca gcctggggga cagagtgacc attagctgcc gggcctctca 600

ggacatcagc aaatacctga actggtacca gcagaaacca gatggcactg tcaagctgct 660

gatttaccac acctccaggc tccacagcgg cgtgcccagt cgcttcagcg gcagtgggag 720

cgggacagat tattccctca caatctccaa cctggagcag gaagatattg ccacatactt 780

ctgccagcaa ggcaacaccc tgccatacac atttggaggc ggcaccaaat tggagatcac 840

cggcggtggt ggatctggag gagggggcag cggaggtggc ggctctgagg tgaaactgca 900

ggagagtggc cctggcctgg tggctcccag ccagagcctt tctgtcacct gcaccgtgtc 960

tggggtgtcc ctgcctgact atggagtctc ctggatccgg cagcctccaa gaaaaggact 1020

ggaatggctg ggcgtcatct ggggaagtga gaccacctac tataattcag ccctcaagtc 1080

ccggctcacc atcattaagg acaactccaa atcccaggtg ttcctgaaga tgaattctct 1140

ccagactgat gacacagcca tctactactg tgccaagcat tattattacg gcgggtccta 1200

tgccatggac tactggggcc aggggaccag tgtcactgtt tcttctgaaa gcaagtatgg 1260

ccccccatgc cctccctgcc ccggacagcc gcgggagccc caggtctaca ccctcccacc 1320

ctccagagat gagctgacca agaaccaggt ttcactgaca tgcctggtga agggcttcta 1380

cccctctgac attgctgtgg agtgggagag caatgggcag ccagagaaca actacaagac 1440

cacacctcct gtcctggaca gcgacggctc cttcttcctc tacagtaagc tcactgtgga 1500

caagagccgc tggcagcagg gcaatgtctt ctcctgcagc gtgatgcacg aggccctgca 1560

caatgcctat acccagaagt cactctctct gagccctgga aagaaggatc ccaagttttg 1620

ggtcctcgtg gtggtcgggg gtgtgctggc ctgctacagc ttgctggtca cagtggcctt 1680

catcatcttc tgggtgcgct ccaagaggag ccggctgctt cacagtgatt acatgaacat 1740

gacccccagg aggccaggac ccaccaggaa gcactaccag ccctacgctc ccccgcggga 1800

ctttgctgct taccgcagca gagtgaaggt gggtaccact gggctttggg aggagggcac 1860

ggggtccccc acttgatgga tgttcagagg ggccttggtc ttggaaggtc tcaagctcgg 1920

gtggtgcctg gggcttggta tccaggagca aagcaaggac cagccaagtg tgtgccttga 1980

gtgggctgag gaggaggtgg cagtgtctgg ctgagatgga cagggtagga gggagagcct 2040

ggtgctaggc acctccatga caagccgtac aaatgtgtgc acatcagagt gtcccaggga 2100

aggcgatgct actggtgaca aaggggctta cactcaggca gaggtccttc tttccaagtg 2160

tgaatgaagg ccatgttagc ctttctcttg aaaaggccct ttcctcatct gtaactgggg 2220

a 2221

<210> 51
<211> 400

<212>  DNA
<213>  Artificial Sequence

<220>
<223>  CAR construct

<400>  51
gattggtgac agaaaagccc catccttagg cctcctcctt cctagtctcc tgatattggg      60

tctaaccccc acctcctgtt aggcagattc cttatctggt gacacacccc catttcctgg     120

agccatctct ctccttgcca gaacctctaa ggtttgctta cgatggagcc agagaggatc     180

ctgggaggga gagcttggca gggggtggga gggaaggggg ggatgcgtga cctgcccggt     240

tctcagtggc caccctgcgc taccctctcc cagaacctga gctgctctga cgcggccgtc     300

tggtgcgttt cactgatcct ggtgctgcag cttccttaca cttcccaaga ggagaagcag     360

tttggaaaaa caaaatcaga ataagttggt cctgagttct                          400


<210>  52
<211>  20
<212>  RNA
<213>  Artificial Sequence

<220>
<223>  guide RNA

<400>  52
gauggaaucc ucuucaucua                                                  20


<210>  53
<211>  20
<212>  RNA
<213>  Artificial Sequence

<220>
<223>  guide RNA

<400>  53
gacgcccccg cguaccagca                                                  20


<210>  54
<211>  20
<212>  RNA
<213>  Artificial Sequence

<220>
<223>  guide RNA

<400>  54
agagucucuc agcugguaca                                                  20


<210>  55
<211>  20
<212>  RNA
<213>  Artificial Sequence

<220>
<223>  guide RNA

<400>  55
gggaaucaaa aucggugaau                                                   20


<210>  56
<211>  20
<212>  RNA
<213>  Artificial Sequence

<220>
<223>  guide RNA

<400>  56
ggggccacua gggacaggau                                                   20


<210>  57
<211>  20
<212>  RNA
<213>  Artificial Sequence

<220>
<223>  guide RNA

<400>  57
ugguacccac cuucacucuc                                                   20


<210>  58
<211>  20
<212>  RNA
<213>  Artificial Sequence

<220>
<223>  guide RNA

<400>  58
ugccuuguuc cugagaguga                                                   20

## Description

The invention relates to the field of genetic modification of therapeutic cell products. The invention relates to a nucleic acid construct for targeting and integrating a CD3 zeta-deficient chimeric antigen receptor (CAR) fragment (transgene) into an endogenous CD3 zeta/CD247 gene for generating a gene fusion and therefore a functional CAR comprising an exogenous CAR fragment fused with an endogenous CD3 zeta domain.

The invention further relates to said construct, comprising three nucleic acid sequence regions, wherein a first nucleic acid sequence region encodes a chimeric antigen receptor (CAR) fragment without a sequence encoding a functional intracellular domain of a CD3 zeta protein, a second nucleic acid sequence region comprises a targeting sequence configured for integrating the construct at an endogenous CD3 zeta gene of a human cell, and a third nucleic acid sequence region encoding and or comprising a protein separation site upstream of the first sequence region.

In further aspects, the invention relates to a CAR polypeptide as an in-frame fusion protein comprising the exogenous CAR fragment and an endogenous CD3 zeta intracellular domain with one or more immunoreceptor tyrosine-based activation motifs (ITAMs). The invention further relates to a genetically modified human cell expressing the CAR fragment, without a sequence encoding a functional intracellular domain of a CD3 zeta protein, integrated in-frame into the endogenous CD3 zeta/CD247 gene, to produce a fusion CAR comprising an exogenous CAR fragment with an endogenous CD3 zeta chain.

The invention further relates to a pharmaceutical composition comprising a genetically modified cell and a pharmaceutically acceptable carrier for therapeutic inventions in human diseases. The invention further relates to corresponding medical uses and therapeutic methods in the treatment and/or prevention of a medical condition, such as conditions associated with the presence of pathogenic cells expressing oncogenes, or for use in the treatment of an autoimmune disease or graft versus host disease.

### BACKGROUND OF THE INVENTION

The most promising immunotherapy strategy against cancer to date is the adoptive cell transfer (ACT) of genetically modified T lymphocytes. ACT has the potential to overcome many of the pitfalls associated with vaccine-based approaches to cancer treatment, such as the de novo activation and expansion of tumor-specific T cells in vivo, even in immunocompromised patients. Using cells genetically modified to recognize tumor-specific or tumor-associated antigens, ACT enables eradication of tumor and tumor stem cells and offers a robust form of immunotherapy to treat established tumors. In contrast to conventional chemo-, radiation- and surgical therapies, this approach potentially prevents tumor recurrence.

A major obstacle in the field of cancer immunotherapy is the breaking of tolerance to "own" antigens. Depending on the type of target, tumor-specific immune responses are directed against either "own" or "foreign" antigens. Foreign antigens can be produced in virally induced cancers or by genetic aberrations unique to a particular tumor, such as point mutations or chromosomal rearrangements that produce epitopes recognized by T cells and B cells. However, immune responses against tumor-associated self-antigens are subject to suppression by central or peripheral tolerance mechanisms. A major problem is the elimination of high-affinity T-cell receptors (TCRs) by the thymus, resulting in TCRs with relatively low affinity for self-antigens compared to foreign antigens being predominant.

There are a number of approaches to overcome the problem of tolerance in the application of cancer immunotherapy. One strategy is the genetic redirection and reprogramming of T cells with chimeric antigen receptors (CARs). Genes encoding antigen receptors can be introduced into the patient's T cells, which are then rapidly expanded to derive memory and effector lymphocytes capable of robust in vivo proliferation and strong anti-tumor activity. These synthetic receptors combine the effector functions of T lymphocytes and the ability of antibodies to recognize predefined surface antigens with high specificity in a non-MHC restricted manner.

The clinically most prominent example of this technology to date is the CAR-T cell therapy for CD19-positive tumors. Here, the patient's T-cells are expanded outside the body in a clean room laboratory and equipped with a CD19-recognising CAR construct by means of retro/lentiviral gene transfer. After infusion into the patient, the genetically modified CAR-T cells recognize the CD19 antigen on B cells and on cancer cells that arise from the B cell line. The clinical success in the treatment of corresponding lymphomas and leukemia has led to the clinical approval of two commercial CAR-T cell products in the USA and Europe (Tisagenlecleucel (Kymriah), Novartis and Axicabtagen-Ciloleucel (Yescarta), Kite/Gilead), others are in the pipeline.

Known CARs usually consist of an extracellular antigen-binding domain, which is connected to signal molecules inside the cell via a linker and a transmembrane domain. CAR constructs are variable in their composition. A "first generation" CAR has a signaling chain for primary T cell activation, usually the CD3 zeta signaling chain; a "second generation" CAR uses the CD3 zeta chain together with a costimulatory unit, preferably CD28 or 4-1BB, to provide a sustained T cell to achieve activation. The "third generation" CAR includes multiple co-stimulatory signaling modules, such as CD28 and 4-1BB (or others), in addition to CD3 zeta.

After interaction with the target antigen, receptor clustering at the membrane and conformational changes occur, which promote growth and survival via co-stimulatory domains and mimic a T-cell receptor signal via the CD3-zeta chain. The latter activates the cytotoxic function of T cells and also stimulates the production and release of cytokines. All functional components of CAR aside from the antigen-binding domain are naturally part of the genome of human cells.

A major disadvantage of the prior art technology are complications in the clinical implementation of CAR-T cell therapy (Elsallab et al. 2020), such as:
a) the complexity of producing an autologous cell product, especially in intensively pre-treated cancer patients,
b) the enormously high price (Kymriah, Novartis = 320.000€) of therapy with an autologous cell product,
c) the critical to fatal side effects for the patient when applying a universal, hence allogenic, CAR-T cell product,
d) the exhaustion of the transgenic T cell product due to a constitutively expressing promoter, and
e) the inefficient gene transfer and integration due to the size of the construct inserted, in particular when gene transfer is performed via non-viral and site-specific gene insertion.

Allogeneic T-cell products have a high risk of leading to the often fatal acute graft-versus-host (GvHD) reaction, in which the donor's CAR-modified T-cells triggered by their endogenous TCR recognize and attack the patient's tissue as "foreign". The use of highly efficient and specific nucleases, such as TALENs and CRISPR-Cas9, has made it possible to specifically switch off genes for the TCR of the donor's CAR-modified T cells (Gundry et al. 2016, Osborn et al. 2016). An initial study with an allogenic TCR-deleted CAR-T cell product showed promising results. However, despite TCR-deletion, one of the patients developed mild GvHD and T-cells from the donor were detected in the skin (Quasim et al. 2017). In the study, the CAR transgene was randomly integrated into the genome of the T-cells by means of a retrovirus and the TCR deletion was subsequently achieved in a separate step. The disadvantage of this technology is that, despite subsequent sorting of the cells, still about 0.1% of all CAR-T cells in the final cell product continue to express their original TCR. This may explain the development of GvHD.

Another established strategy allows the combination of CAR insertion and TCR deletion. In this case the CAR insertion automatically leads to TCR deletion. For this purpose the CAR transgene is inserted into the human TRAC gene locus, via a TRAC knock-in strategy, which encodes a part of the TCR-alpha chain (Eyquem et al. 2017). This leads to a TRAC knock-out and a loss of surface expression of the TCR-CD3 protein complex. In this study, homology-mediated gene transfer specific for exon 1 of the TRAC gene was initiated using CRISPR-Cas9 and an adenovirus-associated virus (serotype 6) to provide the DNA repair template. The CAR transgene including a poly-A terminator sequence is flanked by TRAC gene homologous sequences. These homology regions mimic the sister chromosome to the respective DNA section, which carries the double-strand break induced by the inserted nuclease. Thus, the DNA repair machinery of the T cells inserts the CAR transgene into the TRAC gene locus. This provides a TRAC gene locus controlled expression of CAR in T cells in the absence of a TCR complex. The TRAC modified CAR-T cells showed an improved tumor eradication potential (Eyquem et al. 2017, MacLeod et al. 2017). A weakness of the strategy is that a subset of T cells is able to use alternative TCRs independent of the TRAC gene, such as γδ-T cells. No TCR knock-out takes place in these cells, which makes allogenic use more difficult and the risk of GvHD still remains. This prior art technology is also not suitable for immune cells with potent anti-tumor function, such as natural killer cells, which do not express the TRAC gene and therefore cannot be reprogrammed using a knock-in at the TRAC locus.

To the knowledge of the inventors, all universal CAR-T cell products described to date aim for a complete TCR-KO and employ a complete CAR transgene including all its intracellular domains, in particular including the CD3-zeta domain, to redirect their cytotoxic activity. Further disadvantages in the prior art are the use of viral vectors and the size of the gene constructs to be transferred, which on the one hand are complex and cost-intensive in production under GMP conditions (Qasim et al. 2017 and Eyquem et al. 2017). On the other hand, non-viral methods for site-specific gene insertion are associated with low gene transfer efficiency due to the size of the transgenes (Roth et al. 2018, Nguyen et al. 2020).

Therefore, improving CAR immunotherapy requires overcoming complex technical and biological problems. The present invention addresses three challenging areas in CAR nucleic acid construct design, such as improving integration and expression, reducing the risk of side effects, and the development of a TCR complex-deficient CAR-expressing therapeutic cell. The invention addresses these challenges by (1) enabling CAR transgene expression under the intrinsic control of a TCR complex component promoter and the endogenous 3'-UTR (without the need for a transgenic transcription termination sequence) by taking advantage of intracellular gene regulatory elements (2) providing TCR complex-deficient therapeutic cells, and (3) enabling efficient manufacturing and provision of "off the shelf' therapeutic immune cells.

### SUMMARY OF THE INVENTION

In light of the prior art, the technical problem underlying the present invention is to provide a novel strategy for immunotherapy using genetically engineered therapeutic immune cells. In particular, a problem underlying the invention is the provision of means for an intrinsic, immune cell-specific and target-dependent transgene expression in therapeutic immune cells while avoiding exhaustion, activity loss, and differentiation of said therapeutic immune cells.

A further problem underlying the invention is the provision of TCR complex-deficient therapeutic immune cells, such as allogeneic T cells, for immunotherapy of cancer while avoiding adverse effects caused by allogeneic immunotherapy.

Another problem underlying the invention is the provision of means for an efficient transfer and genomic integration of a transgene while avoiding the complex and cost-intensive production of viral vectors under GMP conditions. A further problem to be solved by the underlying invention is the provision of a fast-track and optimized immunotherapy, at lower cost.

These problems are solved by the features of the independent claims. Preferred embodiments of the present invention are provided in the dependent claims.

The invention therefore relates to a nucleic acid construct, comprising:
(a) a first nucleic acid sequence region, encoding a chimeric antigen receptor (CAR) fragment without a sequence encoding a functional intracellular domain of a CD3 zeta protein,
(b) a second nucleic acid sequence region, comprising a targeting sequence configured for integrating the construct at an endogenous CD3 zeta gene of a human cell, and
(c) a third nucleic acid sequence region encoding and or comprising a protein separation site upstream of the first sequence region.

In one embodiment, the nucleic acid construct is configured for integrating the construct (in particular the first and the third nucleic acid sequence regions) at a single site, preferably into a CD3 zeta/CD247 gene, in the genome of a human cell, preferably a human immune cell.

In one embodiment, the first nucleic acid sequence directly follows, i.e. is adjacent and downstream with respect to the reading frame, the third nucleic acid sequence encoding or comprising a protein separation site.

In one embodiment, the first nucleic acid region encoding a CAR fragment is integrated into the genome of an immune cell, wherein integration of said first region disrupts or reduces endogenous expression of a protein selected from the group consisting of CD3 gamma chain, CD3 delta chain, CD3 epsilon chain, and CD3 zeta chain.

In one embodiment, the first nucleic acid sequence encoding a CAR fragment, is suitable for integration into a CD3 zeta/CD247 gene of the immune cell, preferably downstream of the promotor of said gene, such that expression of said first nucleic acid sequence is under control of an endogenous promoter and preferably a poly-A signal by taking advantage of intracellular gene regulatory elements of said gene within the immune cell. In some embodiments, the integration site is an exon of the endogenous gene expressed by the endogenous promoter in the immune cell.

In one embodiment, the first region encodes a CAR-fragment that is nonfunctional or has substantially reduced function. For example, the CAR fragment lacks a functional intracellular CD3 zeta domain of a functional CAR. Typically, a functional CAR comprises a TCR complex component with at least one ITAM domain, wherein a protein of the CD3 chain of the TCR complex includes at least one ITAM domain. A functional CAR of the prior art typically comprises an intracellular CD3 zeta chain, which is absent in the construct of the invention. The construct of the invention is designed and/or configured for targeting the endogenous CD3 zeta/CD247 gene and subsequent expression of a functional CAR from the combination of the exogenous CAR fragment in frame with the endogenous CD3 zeta coding region, under regulatory control of (expression by) the endogenous CD3 zeta promoter, and other intronic or 3'-UTR located regulatory elements and followed by the endogenous poly-adenylation signal.

In one embodiment, the present invention provides a nucleic acid construct that leads to prevention of formation of the TCR-CD3 complex at the surface of an engineered human CAR-T cell product.

In one embodiment, the invention relates to a nucleic acid construct comprising one or more targeting sequences configured for integrating said first sequence region (or the construct itself) at an endogenous CD3 zeta/CD247 gene of a human cell. In a preferred embodiment, an incomplete and non-functional CAR transgene is inserted into the endogenous CD3 zeta/CD247 gene of a human cell by homology-mediated DNA repair, whereby the CAR fragment transgene to be inserted contains neither CD3 zeta nor expression regulatory components, e.g. promoter or poly-A Signal, and is therefore shorter than the CARs to be transferred in the state of the art (refer to Example 1).

A skilled person would not have expected that integration of an incomplete, non-functional CAR fragment transgene into the CD3 zeta/CD247 gene would lead to reliable formation of a functional CAR transcript comprising the exogenous CAR transgene complemented with the endogenous CD3 zeta chain, expressed at levels sufficient for therapeutic application. In addition, the insertion of the CD3 zeta-deficient CAR transgene into an exon of the CD3 zeta/CD247 gene, which encodes the intracellular protein, also surprisingly produced a TCR-negative CAR-T cell (as shown in Example 2). An advantageous and unforeseeable aspect of the invention is the provision of a functional, correctly spliced CAR comprising the exogenous CAR transgene complemented with the endogenous CD3 zeta chain, which is presented on the surface of the human immune cell, e.g. a CAR-T cell or CAR-NK cell, that enables therapeutic function.

In one embodiment, the first nucleic acid sequence region encoding a CAR fragment is configured to be integrated into the genome of a cell such that the CAR is expressed by the immune cell at the surface of said cell, and wherein integration of said first nucleic acid into the genome disrupts or reduces expression of a functional TCR complex, if any, at the surface of the cell.

In one embodiment, the first nucleic acid sequence region encoding a CAR fragment is configured to be integrated into a chromosomal sequence on at least one allele of an endogenous gene, preferably two alleles of said gene, preferably an endogenous CD3 zeta/CD247 gene, more preferably two alleles of the CD3 zeta/CD247 gene.

The CAR-T cell product of the present invention, generated using the construct described herein, is in some embodiments also a TCR-negative CAR-T cell product which is particularly well suited for use as an "off-the-shelf" CAR-T cell product in cancer therapy with allogeneic cell therapy products or for cellular immunosuppression approaches in regenerative approaches. The TCR absence on CAR-expressing T cells preferably reduces or negates the risk of graft versus host disease.

In some embodiments, the invention therefore relates to a nucleic acid construct and genetically modified immune cells expressing the first and the third nuclei acid sequence regions of said construct, preferably a CAR-T cell product or CAR-NK cell product conferring human T cells or NK cells a high cytotoxic activity against defined antigens, such as those of solid or liquid tumors, while sparing non-pathogenic cells, for example inside the tissue surrounded by a tumor, such as breast, pancreatic, prostate, lung, colon or liver cells as well as hematological cells.

In preferred embodiments, essentially all other endogenous T cells, B cells, NK cells are likewise spared as the CAR-T cell or CAR-NK cell products of the invention show no or negligible activity against these cells.

The present invention enables the stimulation of cells involved in an anti-tumor immune response and thereby the local activation, support and/or strengthening of an anti-tumor immune response. The present invention enables an effective and therapeutically relevant dose of one or more chimeric antigen receptors endogenously regulated through integration of a nucleic acid construct encoding a CD3 zeta-deficient CAR fragment into an endogenous gene, preferably CD3 zeta coding gene, in transplanted CAR-T cells or CAR-NK cells, while avoiding/suppressing TCR expression. TCR expression in allogeneic CAR-T cells are at times associated with severe side effects, e.g. GvHD. The present invention provides a strategy and method that avoids adverse TCR expression, while disrupting CD3 zeta expression essential for a functional TCR complex formation. In a further aspect, the invention provides a strategy and a method for employing a CD3 zeta-deficient CAR transgene, for example, dependent on immune cell specificity, site, expression state (e.g. expression of a transgene only in the vicinity of a tumor) or timing (e.g. before or after intervention in a tumor cell), wherein CAR expression is preferably under the control of an endogenous promoter and an endogenous Poly-A signal, preferably a CD3 zeta/CD247 gene promoter. CAR expression strategies in the prior art typically provide CAR-T cells, wherein CAR expression is regulated under an exogenous constitutive active RNA-polymerase II promoter combined with other exogenous gene regulatory elements.

In a preferred embodiment, the CD3 zeta/CD247 locus is the target of construction integration, whereby a CAR fragment is transferred to the CD3 zeta/CD247 locus, preferably downstream of the CD3 zeta/CD247 promoter. In a further embodiment, the expression of the CAR transgene takes place under control of the endogenous CD3 zeta/CD247 promoter and poly-A signal, whereby the CAR transgene preferentially forms a fusion gene with the endogenous CD3 zeta/CD247. The preferential integration and fusion of the CAR transgene into the endogenous CD3 zeta/CD247 gene leads in particular to loss of the endogenous CD3 zeta/CD247 gene expression and formation of the natural CD3 zeta protein. In one embodiment, loss of CD3 zeta/CD247 expression also leads to the suppression or absence of expression of a functional TCR complex, resulting in TCR-negative CAR T cells.

In one embodiment, a fusion gene is formed by a CAR transgene preferably integrated in frame into the expression pattern of CD3 zeta/CD247 gene, whereby an open reading frame for a truncated CD3 zeta/CD247 protein is maintained downstream of the CAR transgene insertion site, preferably for a truncated CD3 zeta/CD247 protein with at least one intracellular ITAM domain. In one embodiment, a CAR-CD3 zeta fusion protein is expressed under control of the endogenous CD3 zeta promoter with simultaneous loss of endogenous CD3 zeta/CD247 expression (refer to Examples 2 and 3).

It was a surprising result that the natural immune response could indeed be mirrored or enhanced, whereby a functional CAR at the cell surface was induced by the integration of a CD3 zeta-deficient CAR fragment into the endogenous CD3 zeta/CD247 gene in CAR-T cell (refer to Examples 3 or 5) or CAR-NK cell (Example 4) products by the means of the invention.

In one embodiment, in NK cells, the CD3 zeta deficient CAR transgene as provided by the invention can be expressed under control of the CD3 zeta/CD247 promoter but not under the TRAC promoter (refer to Example 4). Another advantageous aspect of the invention is therefore targeting the gene encoding the CD3 zeta chain for integrating the CAR fragment transgene of the invention, since the CD3 zeta/CD247 gene is also expressed in immune cells, in addition to T-cells, which are also applicable for effective and safe immunotherapy, including NK-cells (refer to Examples 4 and 12). The TCR complex component TRAC, as used in the prior art as integration site (WO2017/1809893), does not provide this advantage.

The combination of the CD3 zeta deficient CAR transgene integrated into the CD3 zeta/CD247 gene locus, with NK cells or other immune cells, such as Treg, is a preferred embodiment of the present invention.

It was surprising and is particularly advantageous, that by integrating the CAR transgene into the CD3 zeta/CD247 gene, in particular the region of the gene encoding for the cytoplasmic domain, the endogenous CD3 zeta expression is disrupted and no endogenous TCR is presented on the CAR T cell. It was not expected that the construct of the invention would directly influence expression of the intracellular human CD3 zeta chain. The present invention therefore also provides means to disrupt the endogenous TCR expression and to enable a safe immunotherapy with allogeneic CAR-T cells, thus making immunotherapy not only safer but also easier, faster and significantly cheaper. Furthermore, it was surprising that the targeted immune cells correctly splice the CAR transgene integrated in the CD3 zeta/CD247 gene without exonic sequences of the CD3 zeta chain, in order to form a functional CAR transcript complemented with an endogenous CD3 zeta domain.

In one embodiment, the insertion of a truncated CD3 zeta-deficient CAR transgene into the endogenous CD3-zeta/CD247 gene allows for a sufficient expression of the CAR (refer to Example 7). It was surprising that the level of expression of CAR in the present invention, which is lower than that of prior art CARs, leads to a sufficient and therapeutically relevant expression of the CAR in CAR-T cells or CAR-NK cells (refer to Example 8). It was not expected that this lower expression rate of the CAR, as a CAR-CD3 zeta fusion protein in the CAR-T cell and CAR-NK cell, which corresponds to the endogenous expression rate of CD3 zeta/CD247 in said cells, is particularly advantageous for efficacy in tumor eradication and safety of immunotherapy (refer to Example 3). Of particular advantage is that the mentioned CAR-T cells or CAR-NK cells show a reduced cell depletion and differentiation associated with the improved anti-tumor activity and expansion of the CAR-T cell or CAR-NK cell products.

The endogenous regulation of expression was surprisingly beneficial for the performance and endurance of CAR-T cells and CAR-NK cells of the invention described herein.

The present invention therefore provides means for the production of CD3 zeta promoter-dependent expression of a CD3 zeta deficient CAR in CAR-T cells or CAR-NK cells with a potent and antigen-specific ability to eliminate tumor cells in vitro (refer to Examples 8 and 9). Integration of a CD3-zeta-deficient CAR into other genomic loci than the CD3 zeta/CD247 gene lacks a cytotoxic effect. As a result, fully functional, TCR-negative CAR-T cells can be produced by a precisely positioned insertion of the CD3 zeta-deficient CAR transgene.

In some embodiments, the provision of a combination of transgenes encoding a tumor-specific CAR, an immune stimulating protein, e.g. cytokine or checkpoint inhibitor molecule, a CAR-regulating protein and/or a therapeutic protein in CAR-T cells or CAR-NK cells, also achieves a locally more effective and safer anti-tumor response.

In some embodiments, additional sequences can be placed within the construct, preferably in the first nucleic acid sequence, wherein these sequences encode one or more therapeutic proteins that are separated from the CAR fragment-encoding region by a sequence encoding a self-cleavage peptide, proteolytic cleaving site, and/or an IRES site.

The combination with additional therapeutic proteins, preferably expressed under the same promoter as the CAR fragment, leads to a unique local expression and secretion of such proteins, which induces a local anti-tumor response encompassing multiple arms of the immune response without inducing toxicity as frequently observed in the application of immunostimulatory molecules, e.g. cytokines, in tumor patients.

A further advantage of the invention is the cell type-dependent regulation of gene expression for the inserted CAR transgene. The CD3 zeta/CD247 gene is expressed in cells of the immune system, preferably in lymphocytes, such as T cells and NK cells. Other cell types, e.g. muscle cells, do not express CD3 zeta/CD247 or at a very low extent. Thus, the strategy and methods of the invention also allow that CAR transgenes, which are misguidedly transferred into non-target cells and inserted into their genome, remain non-expressed in these non-target cells or show an expression rate without any therapeutic or toxic relevance.

In one embodiment, the first nucleic acid sequence region encoding the CAR fragment comprises:
- a nucleic acid sequence encoding an extracellular antigen-binding domain, said antigen-binding domain preferably comprising an antibody or antibody fragment,
- a nucleic acid sequence encoding a transmembrane domain, and
- a nucleic acid sequence encoding one or more intracellular co-stimulatory domains.

In one embodiment, the CAR antigen-binding domain binds to a cancer antigen. In some embodiments, the antigen binding domain binds to a cancer associated antigen selected from the group consisting of: CD19, APRIL/TNFSF13, Mic A/B, T cell associated antigens: CD3, CD5, CD7, Folate receptor alpha (FRa), ERBB2 (Her2/neu), EphA2, IL-13Ra2, epidermal growth factor receptor (EGFR), Mesothelin, TSHR, CD123, CD22, CD30, CD171, CS-1, CLL-1, CD33, EGFRvlll, GD2, GD3, BCMA, Tn Ag, prostate specific membrane antigen (PSMA), ROR1, FLT3, FAP, TAG72, CD38, CD44v6, CEA, EPCAM, B7H3, KIT, interleukin-11 receptor a (IL-11Ra), PSCA, PRSS21, VEGFR2, LewisY, CD24, platelet-derived growth factor receptor-beta (PDGFR-beta), SSEA-4, CD20, MUC1, NCAM, Prostase, PAP, ELF2M, Ephrin B2, IGF-I receptor, CAIX, LMP2, gp100, bcr-abl, tyrosinase, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin, telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, IGLL1, and derivates thereof comprising post-translational modifications of the polypeptide, for example, glycosylations, ubiquitination, acetylations, phosphorylations and the like, as well as other modifications known in the art, both naturally occurring and non-naturally occurring.

In one embodiment, the CAR antigen-binding domain binds to a self- or alloantigen, wherein the self- or alloantigen is selected from a group of HLA haplotypes including HLA-A2, HLA-B7, HLA-DR as well as HLA associated antigens, such as Mic A, or Mic B.

In one embodiment, the CAR antigen-binding domain binds to a self-antigen associated with inflammation or an exogenously introduced antigen, wherein said exogenously introduced self-antigen can be selected from a group including MOG, Aquaporin 4, Gluten-tissue transglutaminase complex, adenovirus, or AAV.

In some embodiments, an immune cell, preferably NK cell or T cell, in which the first nucleic acid encoding the CAR fragment is expressed, is sensitized to a target antigen. In one embodiment, the antigen binding domain of the CAR fragment recognizes (preferably specifically binds to) CD19. Corresponding immune cells expressing said CAR fragment, preferably a CAR-T cell or CAR-NK cell product, exhibit a high cytotoxic activity against CD19 positive pathogenic cells.

In another aspect, the invention also relates to a cell product, such as an advanced therapy medicinal product, designed and manufactured for medical use, comprising next generation anti-CD19 CAR-transfected or transduced immune cells that can be used in the treatment of cancer with CD19 positive tumor cells (refer to Example 8).

Further embodiments of the first nucleic acid sequence region encoding the CAR fragment are listed below. In some embodiments, the nucleic acid region encoding the antigen binding domain of the CAR described herein, encodes an antigen binding domain coupled to a transmembrane domain by a hinge region.

In some embodiments, the transmembrane domain comprises a protein selected from the group consisting of the alpha, beta or zeta chain of the T cell receptor, CD28, CD3 epsilon, CD45, CD4, CD5, CD8, CD9, CD16, CD22, CD33, CD37, CD64, CD80, CD86, CD134, CD137, and CD154.

In some embodiments, the intracellular signaling domain comprises a costimulatory signaling domain comprising a functional signaling domain obtained from a protein selected from the group consisting of a MHC class I molecule, a TNF receptor protein, an Immunoglobulin-like protein, a cytokine receptor, an integrin, a signaling lymphocytic activation molecule (SLAM protein), an activating NK cell receptor, gp130, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a or derivates thereof with at least 80% amino acid sequence identity.

In one embodiment, the nucleic acid construct encodes a CD3 zeta deficient CAR fragment, comprising:
- a CAR signal sequence, preferably according to SEQ ID NO 27 or a sequence with at least 80% sequence identity to SEQ ID NO 27;
- an antigen-binding domain of a CAR that specifically binds to an antigen, more preferably CD19 according to SEQ ID NO 28 and SEQ ID NO 30 linked with a linker according to SEQ ID NO 29, or a sequence with at least 80% sequence identity to SEQ ID NO 28, 29, and 39;
- an immunoglobulin heavy chain constant region of a CAR, preferably according to SEQ ID NO 31-33, or a sequence with at least 80% sequence identity to SEQ ID NO 31-33; and/or
- a CD28 signalling domain, preferably according to SEQ ID NO 34 and 35 or a sequence with at least 80% sequence identity to SEQ ID NO 34 and 35; wherein the CD28 signaling domain comprises a transmembrane domain, preferably according to SEQ ID NO 34, or a sequence with at least 80% sequence identity to SEQ ID NO 34.

In some embodiments, the nucleic acid construct encodes a CD3 zeta deficient CAR fragment further comprising
- a cytoplasmic CD3 zeta signaling domain comprising a stop signal for protein translation, preferably according to SEQ ID NO 44, or a sequence with at least 80% sequence identity to SEQ ID NO 44, and
- a poly-A signal, preferably according to SEQ ID NO 45, or a sequence with at least 80% sequence identity to SEQ ID NO 45, or
- a truncated CD3 zeta signaling domain lacking a stop signal for protein translation and lacking a poly-A signal, preferably according to SEQ ID NO 41, or a sequence with at least 80% sequence identity to SEQ ID NO 41.

In one embodiment, a poly-A signal is known to a skilled person and can be an endogenous poly-A signal within a gene structure, a synthetic transcription terminator sequence or a poly-A sequence adapted from mammalian genes.

In some embodiments, the sequence variants with 70% or more sequence identity to the specific CAR sequences of SEQ ID 28-33 maintain CD19 binding with essentially the same or similar functional properties as VH and VL domains with the specific CAR sequences of SEQ ID NO 28-33, i.e. the CD19 binding is essentially the same or similar with respect to affinity, specificity and epitope binding mode.

In some embodiments, the nucleic acid sequence encoding a CD3 zeta deficient CAR fragment is codon-optimized in order to improve expression of said CAR fragment. Such modifications enable sufficient surface expression on T cells or NK cells and still maintain proper antigen binding. High affinity and high avidity enable CAR-T cells or CAR-NK cells or CAR-Treg cells to i) recognize, ii) be activated against, and iii) kill tumor target cells with high, intermediate or low tumor-antigen surface expression.

In one embodiment, the antigen-binding domain of the CAR fragment specifically binds CD19. The anti-CD19 CAR-T cell and the anti-CD19 CAR-NK cell product described herein are characterized by beneficial properties. The anti-CD19 CAR as described herein has a high affinity and confers high specificity and avidity to T cells and/or NK cells. These properties enable CAR-T cells or CAR-NK cells to i) recognize, ii) be activated against, and iii) kill tumor target cells with high or low CD19 surface expression. For example, the number of CD19 antigens expressed on the surface of tumor cells can be quantified using an anti-CD19 antibody coupled to a fluorescent-dye in conjunction with Quantibrite beads (e.g. from BD). The preferred method applied to quantify CD19 antigens expressed on the surfaces of tumor cells is "fluorescence activated cell sorting/cell analysis" (FACS).

Anti-CD19 CAR-T cells without TCR expression, in particular in allogenic CAR cell products, will enable targeting of the tumor cells in the tumor microenvironment as shown in the examples described herein (refer to Example 11).

The tumor antigen-specific CAR-T cells described herein are applicable in preferred embodiments for the treatment of patients with solid or liquid tumors that are not eligible for other therapies. More specifically, the embodiments of the invention relate to the treatment of the following patient populations:
i. patients with multidrug resistances,
ii. patients not eligible for allogeneic/hematopoietic stem cell transplantation,
iii. patients with co-morbidities that preclude further chemotherapies,
iv. patients suffering from solid and/or liquid cancers,
v. aged patients who do not tolerate chemotherapies,
vi. the CAR is applicable for salvage therapies even after progressive disease and multiple lines of other standard of care therapies have failed, and/or
vii. it is applicable even at low antigen density on target tumor cells, where antibodies can fail, and/or
viii. it is applicable as a monotherapy which is not the case for antibodies, and/or
ix. it is applicable in combination with one or more anti-cancer treatments, anti-cancer medicaments or transplantation.

As demonstrated in the examples below, in an in vitro co-culture system, anti-CD19 CAR-T cells or anti-CD19 CAR-NK cells become activated upon exposure to CD19-expressing human tumor cell lines (refer to Example 8 and 9). These T cells or NK cells then develop an effector phenotype with high level of a cytotoxic activity. Additionally, a cytotoxicity assay against selected target cell lines, CD19-negative cells, shows that selective cellular cytotoxicity is obtained only against cell lines positive for CD19 (refer to Example 8).

In one embodiment, the targeting sequence is configured for integrating said first nucleic acid sequence region in-frame with the endogenous CD3 zeta encoding sequence, preferably into an exon of an endogenous CD3 zeta gene.

In one embodiment, an insertion site of the CD3 zeta deficient CAR transgene is preferably downstream of the CD3 zeta/CD247 promoter, wherein the insertion can take place into an exon or into an intron of the CD3 zeta/CD247 gene, preferably an exon. In some embodiments, the exon of the CD3 zeta/CD247 gene for insertion of said CAR transgene can be exon 1, exon 2, exon 3, exon 4, exon 5, exon 6, exon 7 or exon 8, preferably exon 1, 2, 3, 4, 5, or 6, more preferably exon 2 or exon 3, more preferably exon 2, wherein the resulting CAR polypeptide includes at least one intracellular ITAM domain.

In one embodiment, the CAR transgene encoded by said first nucleic acid sequence region is not operably linked to an exogenous promoter.

In one embodiment, the third nucleic acid sequence region positioned upstream of the first sequence region, encodes a first self-cleavage peptide, protease cleavage site or an internal ribosomal entry site (IRES).

Sequences downstream of a promoter may be transcribed but are not necessarily translated. An untranslated region downstream of a promoter and extending to the first start codon for translation is the 5' untranslated region (5' UTR). In one embodiment of the invention, the insertion site for the CAR transgene is therefore downstream of the 5' UTR.

In some cases, a sequence segment upstream of the CAR fragment is transcribed together with the exogenous CAR transgene. In some embodiments, means are therefore provided to separate any sequence upstream of CAR polypeptide during or after translation.

In some embodiments, a nucleic acid sequence region encoding a protein separation site, for example a self-cleavage peptide, a protease cleavage site or an internal ribosomal entry site (IRES), preferably a self-cleavage peptide, is positioned upstream of the nucleic acid sequence region encoding the CAR fragment. The protein separation site is employed to separate any sequences upstream of the CAR, that are translated together with the CAR, that may be dysfunctional or unwanted in the CAR polypeptide.

In one embodiment, the CAR fragment encoding region is therefore separated from endogenous gene and/or mRNA sequence segments by a sequence encoding a self-cleavage peptide, proteolytic cleavage site, and/or an IRES site.

In one embodiment, a polypeptide cleavage site is selected from the group consisting of P2A, T2A, E2A and F2A.

In one embodiment, the proteolytic cleavage site is an amino acid recognition site for an intracellular proteolytic cleavage enzyme.

In one embodiment, the IRES site, for example enabling mRNA translation initiation at the ribosome in a cap-independent manner, is a viral IRES site, e.g. HCV-like IRES, picornavirus IRES or encephalomyocarditis virus IRES.

In some embodiments, the self-cleavage peptide is preferably a 2A peptide, more preferably selected from the group consisting of a foot-and-mouth disease virus (FMDV) 2A peptide, an equine rhinitis A virus (ERAV) 2A peptide, a Thosea asigna virus (TaV) 2A peptide, a porcine tescho virus- 1 (PTV-I) 2A peptide, a Theilovirus 2A peptide, and an encephalomyocarditis virus 2A peptide.

In one embodiment, the nucleic acid construct is configured for expression of a chimeric antigen receptor (CAR), comprising the CAR fragment encoded by the first sequence region and an intracellular domain encoded by the endogenous CD3 zeta gene, under control of an endogenous CD3 zeta promoter.

Such a construct enables integration of the transgene, encoding a CD3 zeta deficient CAR fragment, into a location within the genome, such that the integrated nucleic acid sequence, i.e. the transgene, is under the control of an endogenous promoter and an endogenous poly-A signal by taking advantage of intracellular gene regulatory elements. In some embodiments, the endogenous promoter is a promoter of a TCR complex component.

The endogenous promoter is therefore intrinsically regulated and/or induced, e.g. by cellular signals, from the immune cell. In some embodiments, the endogenous promoter is active in a subset of immune cells, preferably NK cells or T cells or Treg cells. In some embodiments, the endogenous promoter is preferably a promoter of genes encoding proteins of the TCR complex, e.g. a CD3 zeta, CD3 epsilon, CD3 delta, CD3 gamma, preferably a promoter of a gene encoding a protein comprising at least one intracellular ITAM domain.

In one embodiment, expression of the first nucleic acid sequence region after integration is terminated by an endogenous CD3 zeta gene expression termination signal and/or use of the endogenous poly-A signal.

It is a particular advantage of the present invention that it employs not only endogenous promoter expression, but also endogenous termination signals. The endogenous gene regulation of CD3 zeta/CD247 is a rate-limiting step in TCR complex formation and is influenced by tumor microenvironment. In addition, due to the absence of exogenous termination signals, the construct of the invention is smaller and results in a particularly effective transfer and integration of the nucleic acid sequences.

A surprising effect of the CD3 zeta-deficient CAR construct, lacking both an exogenous promoter and expression termination signals including a poly-A signals, is that it exhibits an unexpectedly low toxicity to target cells compared to prior art CAR constructs, which would not have been predicted by a skilled person. In preferred embodiments this is particularly evident for non-viral transfer methods, such as those disclosed herein. This is advantageous for target cell survival, transgene integration rate, and function of the targeted cell (refer to Examples 3 and 6).

In one embodiment, an endogenous CD3 zeta/CD247 gene expression termination signal comprises a transcription terminator sequence, a poly-A signal, and a translation stop codon, and is known to a skilled person, who is capable of identifying a suitable fusion site and/or generating nucleic acid constructs configured for fusion, depending on the intended target site for fusion within an endogenous CD3 zeta/CD247 gene.

In some embodiments, the target site of the CD3 zeta gene, into which the first sequence region is integrated, is an exon and/or an intron, preferably exon 2, and is positioned upstream of an endogenous CD3 zeta sequence encoding an intracellular domain or fragment thereof, said intracellular domain or fragment thereof comprising one or more immunoreceptor tyrosine-based activation motifs (ITAMs).

In some embodiments, an ITAM-comprising CD3 protein domain for a CAR can be CD3 zeta, CD3 delta, CD3 gamma, CD3 epsilon, preferably CD3 zeta. The intracellular parts of CD3 zeta, CD3 delta, CD3 gamma, CD3 epsilon each carry at least one conserved motif known as an activation motif based on immune receptor tyrosine, termed "ITAM", which is essential for the TCR signaling, in particular to generate an activation signal in T lymphocytes. The endogenous intracellular domain of CD3 zeta contains three ITAM motifs, although not all are necessarily essential. In a preferred embodiment, the CAR fusion protein comprises a CAR fragment fused to functional CD3 zeta fragment with at least one ITAM domain.

The CD3 zeta protein comprises an extracellular and a transmembrane domain, followed by an intracellular domain comprising three ITAMs. For its function as a signaling domain within a CAR, at least one ITAM is essential. In certain embodiments, when selecting the integration site for the construct, a site within the CD3 zeta/CD247 gene is selected which is upstream of at least one ITAM, more preferably upstream of all three ITAMs. A skilled person is capable of identifying a sequence encoding an ITAM domain and a suitable site for an in-frame integration.

The coding sequence for the ITAM-containing intracellular domain of CD3 zeta begins in exon 2. In one aspect of the invention, exon 2 is therefore particularly advantageous as an insertion site, thereby producing a CAR without an endogenous extracellular or transmembrane region of CD3 zeta. In further embodiments, the insertion site for the CAR transgene can also be located in exon 3, exon 4, exon 5 or exon 6.

In some embodiments, the first nucleic acid sequence region encoding a CAR fragment additionally comprises a sequence encoding a CAR-regulating, immune-stimulating and/or therapeutic protein, in-frame and separated from the CAR fragment-encoding region by a sequence encoding a second self-cleavage peptide, a promoter and/or an IRES site.

The construct can therefore lead to expression of additional beneficial proteins, e.g. cytokine and/or checkpoint inhibitor molecules with the aim to attract immune effector and helper cells, to induce immune activation, promote maturation of memory immune cells and/or suppress the development and persistence of suppressive and/or regulatory immune cells.

In some embodiments, the first nucleic acid sequence region encoding a CAR fragment additionally comprises at least one sequence encoding an immune-stimulating protein, e.g. chemokines, cytokines and/or checkpoint inhibitors. Cytokine, chemokines, and checkpoint inhibitors that stimulate the immune response are known to a skilled person and can be assessed and/or determined by established routine assays. In some embodiments, the immune-stimulating protein that is a cytokine is selected from the group consisting of IL2, IL12, IL15, IL18, IL-7, or constitutively active cytokine receptors, exogenously activated co-stimulatory or cytokine signaling promoting receptors. This strategy avoids potential adverse effects of systemic application of said immune stimulatory proteins.

Tumor-induced immunosuppression is largely mediated by the secretion of anti-inflammatory cytokines by immune cells and control proteins, e.g. immune checkpoint proteins such as PD-1, present in a tumor that have a regulatory phenotype (e.g. regulatory T cells and suppressor cells derived from monocytes). The invention therefore additionally provides advantageous means for modifying the tumor microenvironment, making it pro-inflammatory, promoting the activation of immune cells present in the tumor and the recruitment and activation of external immune cells, thereby facilitating broad activation of the immune system against the tumor and/or increasing the effectiveness of anti-tumor immunotherapeutic treatments.

In one embodiment, the construct encodes a checkpoint inhibitory protein, e.g. a PD-L1 and/or PD-1 inhibitor. Cancer cells utilize mechanisms that avoid regular immune system control. Checkpoint proteins have been shown to function by communicating to the immune system that a potentially cancerous cell is not to be destroyed.

In one embodiment, a CAR-regulating protein is encoded. In some cases, it may be desirable to provide a safety mechanism that allows selective deletion of the administered T cells, as the manipulated T cells can potentially spread and persist for years after administration. The invention provides additional means to optionally switch off the genetically modified cell product expressing the CAR, as described herein, i.e. for safe clinical use. Therefore, in some embodiments the construct encodes a suicide gene, capable of reducing the risk of toxicity and/or uncontrolled proliferation of T cells after administration to a subject. Suicide genes enable the selective deletion of transformed cells in vivo.

In some embodiments, additional sequences within the first nucleic acid sequence region are inserted in-frame and separated from the CAR fragment-encoding region by a sequence encoding a self-cleavage peptide, proteolytic cleavage site, and/or an IRES site.

In one embodiment, the first sequence region encoding the CAR fragment is linked to additional nucleic acid sequence region encoding, for example, a CAR-regulating protein, an immunostimulatory protein or a checkpoint inhibitor molecule, and is configured to encode a polycistronic mRNA.

In some embodiments, the targeting sequence of the second sequence region is configured for integration into a host genome by a gene editing technique, preferably CRISPR-Cas, zinc finger nucleases (ZFNs), integrases, site specific recombinases, meganucleases, homing endonucleases, or TALENs, more preferably a CRISPR-Cas9 derived from *Streptococcus pyogenes.*

In one embodiment, a gene editing technique is used to create a double strand break, preferably at a target site in the CD3 zeta allele, and the nucleic acid construct is incorporated into the target site by homology directed repair at the site of the double-strand break. A non-viral vector is preferably used for transferring the nucleic acid construct encoding the CAR fragment into the cell. In one embodiment, a delivery method is non-viral, preferably electroporation (refer Example 6). The gene transfer of the CRISPR-Cas9 machinery with the construct can be carried out by electroporation or virus-mediated, e.g. lentivirus, or transposon-controlled.

In some embodiments, the nucleic acid construct, preferably a linear DNA, or a vector, such as a plasmid vector, viral vector, or a transposon vector, enables an unusually high transfer rate into human T cells or NK cells.

In a further aspect of the invention, the invention relates to an isolated nucleic acid molecule, preferably in the form of a vector, such as a synthetic linear DNA or a circular plasmid vector, viral vector or a transposon vector, preferably a plasmid vector, selected from the group consisting of:
i. a nucleic acid molecule comprising a nucleotide sequence,
   which encodes a CD3 zeta deficient CAR polypeptide according to any embodiment described herein,
   which encodes an extracellular antigen-binding domain, a transmembrane domain, and an intracellular co-stimulatory domain, or
   wherein the extracellular antigen-binding domain is encoded by at least one sequence of SEQ ID NO 28, 29, 30 ,31, 32, 33,
   and/or
ii. a nucleic acid molecule comprising a nucleotide sequence,
   which encodes a targeting sequence comprising identical sequences to a recognition site of the endogenous DNA sequence into which integration is intended, also termed homology arms, according to any embodiment of the CAR fragment described herein, wherein the homology arm is according to at least one sequence of SEQ ID NO 25, 36, 39, 42, 43,
   and/or
iii. a nucleic acid molecule comprising a nucleotide sequence,
   which encodes or comprises a protein separation site according to any embodiment described herein, wherein the protein separation site is encoded by at least one sequence of SEQ ID NO 26, and/or
iv. a nucleic acid molecule which is complementary to a nucleotide sequence in accordance with i, ii and/or iii,
v. a nucleic acid molecule comprising a nucleotide sequence having sufficient sequence identity to be functionally analogous/equivalent to a nucleotide sequence according to i-iv, comprising preferably a sequence identity to a nucleotide sequence according to i-iv of at least 70%;
vi. a nucleic acid molecule which, as a consequence of the genetic code, is degenerate to a nucleotide sequence according to i-v; and/or
vii. a nucleic acid molecule according to a nucleotide sequence of i-vi which is modified by deletions, additions, substitutions, translocations, inversions and/or insertions and is functionally analogous/equivalent to a nucleotide sequence according to i-vi.

In one embodiment, a gene editing technique may be used to generate a genetically modified immune cell that comprises a chromosomal sequence edited on at least one allele of an endogenous gene, preferably two alleles of said gene, preferably an endogenous CD3 zeta/CD247 gene, more preferably two alleles of the CD3 zeta/CD247 gene.

In another aspect, the invention further relates to a method for producing a genetically modified cell, comprising delivering or transferring a nucleic acid construct as described herein.

Additionally, the signaling components of the CAR encoding nucleic acid construct may be exchanged, for example in a three-step cloning procedure, that allows for a modular composition, and tailor-made construction by a skilled person, of clinically applicable anti-tumor-antigen CARs, for example anti-CD19 CARs (refer to Examples 10 and 11).

In some embodiments, the targeting sequence comprises an identical sequence to a recognition site of the endogenous DNA sequence into which integration is intended, preferably selected from the group consisting of a homology arm, a guide RNA target site, a restriction enzyme recognition site, a ZFN recognition site, a ZFN cleaving site, a TALEN DNA-binding site, a recombinase recognition site, an integrase site and/or a homing nuclease recognition site.

In certain embodiments, the targeting sequence, termed "homology arm", comprises at least one sequence complementary to an endogenous genomic sequence of the target cell. In one embodiment, the targeting sequence of the second sequence region configured for integration into a host genome comprises a homology arm complementary to an endogenous integration site, described above, preferably complementary to a site within the CD3 zeta/CD247 gene.

In some embodiments, the nucleic acid construct comprises in 5' to 3' order: a first vector DNA sequence, a left homology arm, a nucleic acid sequence encoding a self-cleaving peptide, the nucleic acid sequence encoding the CD3 zeta-deficient CAR fragment, a right homology arm, and a second vector DNA sequence. In certain embodiments, the first and the second vector DNA sequence can also be a first and second viral sequence (refer to Example 1). In some embodiments, no vector sequence is required or used.

In some embodiments, the nucleic acid construct comprises or consists of, in 5' to 3' order: a left homology arm, a nucleic acid sequence encoding a self-cleaving peptide, the nucleic acid sequence encoding the CD3 zeta-deficient CAR fragment, and a right homology arm.

In certain embodiments, a gene editing system for introducing the CD3 zeta deficient CAR fragment into an immune cell comprises:
i. a nucleic acid sequence encoding a CD3 zeta deficient CAR fragment,
ii. a nucleic acid sequence encoding or comprising a protein separation site, and
iii. a nucleic acid sequence encoding a homologous recombination system suitable for targeted integration of the nucleic acid sequence at a target site within the genome of the cell,
whereby the homologous recombination system integrates the construct at a site within the CD3 zeta/CD247 gene of said cell.

In one embodiment, the immune cells are preferably co-electroporated with a guide RNA, e.g. a sgRNA, targeting the CD3 zeta/CD247 gene, wherein the second nucleic acid sequence region of the construct comprises:
i) a left homology arm upstream of the CAR fragment at the 5' end of the construct complementary to a first target sequence within the CD3 zeta/CD247 gene and
ii) a right homology arm downstream of the CAR fragment at the 3' end of the construct complementary to a second target region within the CD3 zeta/CD247 gene.

In some embodiments, a homology directed repair (HDR) template is a nucleic acid molecule, preferably a double strand DNA, also used in the homology directed repair gene editing based technique. In certain embodiments of the method for generating a genetically modified immune cell, the double strand DNA repair template is a homology directed repair (HDR) template.

In some embodiments, a homology directed repair (HDR) template is generated as described in Roth et al. (2018 Nature).

In one embodiment, the invention provides means to insert the transgene into a cell using the homology-directed repair (HDR) method. In one embodiment, the HDR dsDNA template together with CRISPR-Cas9 ribonucleoprotein (Cas9) complexed with the single guide RNA (sgRNA) is transferred into the cell by means of co-electroporation (refer to Examples 1 and 5) Genome editing through HDR is advantageous for the specific and efficient and viral vector free insertion of large transgenes into target cells. It is advantageous that the viability and cell function of the transfected cells is maintained and allows a high yield of genetically modified cells for clinical application (refer to Example 6).

A further aspect of the invention relates to a genetically modified human cell, comprising an exogenous nucleic acid sequence region encoding a chimeric antigen receptor (CAR) fragment, without a sequence encoding a functional intracellular domain of a CD3 zeta protein, integrated in-frame into an endogenous CD3 zeta gene.

In some embodiments, the genetically modified cell is a product of modifying a cell with the construct as described herein. Features disclosed with respect to the construct or the method described above are applicable to the genetically modified cell, and vice versa.

In some embodiments, the genetically modified cell expresses a chimeric antigen receptor (CAR) as an in-frame fusion protein comprising the CAR fragment and an intracellular domain of the endogenous CD3 zeta gene, said intracellular domain comprising one or more immunoreceptor tyrosine-based activation motifs (ITAMs).

As described above, CD3 zeta protein comprises an extracellular and a transmembrane domain followed by an intracellular domain comprising three ITAMs. For a functional CAR in genetically modified immune cell, at least one ITAM is required, so that for choosing the integration site for the fusion of the CAR transgene into the CD3 zeta/CD247 gene, a site within the CD3 zeta/CD247 gene is preferably selected which is upstream of at least one ITAM, more preferably three ITAMs. A skilled person is capable of identifying a sequence encoding an ITAM domain and a suitable site for an in-frame integration for CAR/CD3 zeta fusion.

In one embodiment of a genetically modified immune cell, at least the first nucleic acid sequence region encoding the CAR fragment and the endogenous CD3 zeta sequence are fused to enable a polycistronic mRNA comprising coding regions for the polypeptide sequences of the CAR fragment and the endogenous CD3 zeta, wherein an amino acid sequence comprising a protein separation site, e.g. a peptide cleavage site, is positioned upstream of the CAR polypeptide.

In one embodiment of a genetically modified immune cell, the construct can additionally comprise sequences, e.g. encoding CAR regulating, immune-stimulating and/or therapeutic proteins, as described above, fused to said CAR fragment and configured to encode a polycistronic mRNA , wherein an amino acid sequence comprising a protein separation site, e.g. a peptide cleavage site, is disposed between the CAR polypeptide and each additional polypeptide.

In one embodiment of the genetically modified immune cell, a first nucleic acid encoding a CD3 zeta deficient CAR fragment directly follows a third nucleic acid region, providing a protein separation site. The spliceosome of the cell is capable of correctly splicing a transcript consisting of endogenous CD3 zeta sequences fused to said CD3 zeta deficient CAR fragment and said protein separation site, so that an open reading frame for the exogenous CAR fragment followed by at least one endogenous CD3 zeta ITAMs is maintained for translation. Upstream of the CAR fragment, an exogenous protein separation site follows endogenous CD3 zeta sequences. The protein separation site, preferably a P2A peptide cleavage site, is suitable to separate the CAR fragment polypeptide fused with the CD3 zeta ITAM polypeptides from the unwanted endogenous CD3 zeta segments during or after translation.

In one embodiment of the genetically modified immune cell, said the fusion CAR polypeptide is functional and presented on the surface of said immune cell.

In one embodiment of the genetically modified immune cell, the chimeric antigen receptor (CAR)-CD3 zeta fusion gene is expressed under the control of an endogenous CD3 zeta promoter and an endogenous CD3 zeta expression termination signal.

In some embodiments of the genetically modified cell, the CAR transgene is fused to an endogenous CD3 zeta/CD247 gene, wherein the fusion site is downstream of the endogenous CD3 zeta/CD247 gene promoter, so that said fusion CAR is under control of the promoter and expression termination signal of the endogenous CD3 zeta/CD247 gene.

In some embodiments, the CAR transgene fused into the CD3 zeta/CD247 gene is or may be termed a CAR-CD3 zeta fusion gene and its corresponding transcript CAR-CD3 zeta fusion mRNA, wherein a polypeptide translated from a CAR-CD3 zeta fusion mRNA is or may be termed a CAR-CD3 zeta fusion protein, or fusion CAR.

The expert would not expect that the endogenous expression regulation of a CAR/CD3 zeta fusion provides such a beneficial effect on anti-tumor activity, endurance, expansion of the CAR cell product, especially as, in some embodiments, the expression of the CAR/CD3 zeta fusion protein is reduced compared to prior art CARs.

The control of gene expression under an endogenous promoter of a TCR-complex component offers the advantage of regulated gene expression as required by the cell to become active against a pathogenic cell, preferably a tumor cell, depending on tumor signals and signals that activate an immune cell.

In some embodiments, the endogenous expression and/or function of a TCR complex, preferably the expression and/or function of a TCR alpha and/or beta chain, or TCR alpha, beta, delta and/or gamma chain, is disturbed (perturbed, disrupted and/or inhibited) in the genetically modified cell.

In some embodiments, integration of the construct disrupts or reduces expression of a protein selected from the group consisting of CD3 gamma chain, CD3 delta chain, CD3 epsilon chain, and CD3 zeta chain (refer to Examples 2 and 3).

In some embodiments, an immune cell is provided in which the fusion CAR is expressed at the surface of the cell, and wherein the expression of a functional TCR complex and presentation at the surface of the cell is reduced and/or absent.

In some embodiments, the genetically modified cell is an immune cell, preferably selected from the group consisting of a T lymphocyte, a T regulatory (Treg) cell, natural killer (NK) cell, a natural killer T (NKT) cell, cytokine-induced killer cell (CIK), an immortalized immune cell including NKT, NK-92 and YT cells, or a cell derived from peripheral human blood, human cord blood, bone marrow stem cells or induced pluripotent stem cells (iPSC), wherein the T lymphocyte is preferably a CD4 or CD8 T-cell, more preferably a cytotoxic T lymphocyte or a T helper cell.

In adoptive cell transfer, T-cell based cytotoxic reactions are used to attack cancer cells. T cells that have a natural or genetically engineered response to a patient's cancer are generated in vitro and then transferred back to the cancer patient. Such T cells can be stimulated to proliferate in vitro by high concentrations of IL-2 and/or other cytokines (such as IL-7 and IL-15), anti-CD3 and anti-CD28 coated microbeads. The present invention therefore encompasses, in some embodiments, adoptive cell transfer in combination with the administration of the CAR expressing cells described herein.

The expression of stimulatory cytokines enhances the activation of T cells only locally, preferably within or near the tumor, and subsequently leads to a memory effector cell phenotype, thereby prolonging the therapeutic effect of the treatment. A surprisingly reduced cytokine production after antigen-engagement by the CD3 zeta truncated CAR of the invention integrated into the CD3 zeta/CD247 gene is additionally advantageous compared to the prior art CARs inserted into the TRAC locus. Furthermore, in some embodiments, of particular advantage for the CAR transgene is a reduced CAR expression and CAR surface density, leading to reduced toxicity, lower risk for over-activation or on-target off-tumor toxicity such as cytokine release syndrome or ICANS. (refer to Example 10).

In embodiments of the genetically modified immune cell, the immune cell is a T cell, an NK cell or a Treg cell. As shown in Example 12, integration of the CD3 zeta truncated CAR into the CD3 zeta/CD247 gene can be used to generate CAR positive CD4+ regulatory T cells.

In one embodiment, the CAR expressing cells express more than one immune stimulating and/ or immune suppression defeating gene and/or an inducible suicide gene allowing said cells to be destroyed. A suicide gene, as non-limiting embodiments, is one that codes for the thymidine kinase of an alpha herpesvirus (HHV1-3), the bacterial gene cytosine deaminase, which can convert 5-fluorocytosine into the highly toxic compound 5-fluorouracil, and inducible caspase-9 or caspase-8 or cytosine deaminase. Inducible caspase-9 can be activated by a specific chemical inducer of dimerization (CID). Suicide genes may also be polypeptides that are expressed on the cell surface and can make the cells sensitive to therapeutic monoclonal antibodies. The suicide gene expression may be inducible, for example by doxycyclin adapted to human cells.

A further aspect of the invention relates to a genetically modified cell for use as a medicament.

In some embodiments, the cell is for use in the treatment of a medical disorder associated with the presence of pathogenic cells expressing oncogenes, such as cancer cells, more preferably cancer stem cells of solid and hematologic malignancies.

In some embodiments, the cell is for use in the treatment of an autoimmune disease or a graft versus host disease.

As such, the CAR of this invention represents a remarkable and beneficial approach to the treatment of various diseases described herein, susceptible to targeted activity of a modified immune cell. The minimal (if any) adverse side effects due to the selectivity and specificity of the expression regulation also represent a beneficial and surprising aspect of the present invention. Especially in patients who have developed resistance to other solid or liquid tumor treatments, the present invention represents a promising approach to the eradication of malignant tumors. As shown in Example 11, a CAR integration into CD3 zeta/CD247 abolishes allo-reactivity of CAR T cells similar to TRAC inserted CAR T cells.

In some embodiments it is advantageous, that the CAR construct in the present invention can be used in therapy of solid tumors and/or liquid tumors, i.e. leukaemia or lymphoma. In some embodiments, the genetically modified cell is used as a medicament, wherein the disorder to be treated with said immune cell is selected from a group of cancers as described herein, preferably consisting of prostate cancer, breast cancer, melanoma, colon carcinoma, acute lymphocytic leukemia (ALL), and Non-Hodgkin lymphoma (NHL).

The various aspects of the invention are bound by the surprising and beneficial concept of local immune system stimulation in an anti-tumor immune response, either by the innate immune system or by combined immunotherapies. It was unexpected that a TCR deficient CAR-T cell would express and present a functional, transgenic CAR/CD3 zeta fusion protein, as shown in the examples 2 to 5, exhibiting such an effective anti-tumor effect. Furthermore, it was unexpected that a CAR-NK cell and a CAR-Treg cell would effectively express and present the transgenic CAR/CD3 zeta fusion protein, as shown in the examples.

The present invention enables a surprising and advantageous endogenous CAR expression rate beneficial for an anti-tumor effect via the CAR/CD3 zeta fusion protein in CAR-T cell or CAR-NK cell as described herein. The endogenously regulated CAR expression (Example 3) combined with improved cell expansion and endurance as described herein supports an anti-tumor immune response and leads to reduction in tumor size and/or growth, and shows a distinct reduction in and/or avoidance of the side effects produced by overexpression of CARs and TCR complex expression in an allogeneic condition (refer to Example 11). Side effects such as GvHD, ICANS (immune effector cell-associated neurotoxicity syndrome), cytokine release syndrome, immune response to recombinant proteins, tumor escape, nausea and vomiting, sores in the mouth or on the lips, diarrhea, drowsiness, allergic reactions, fever or chills, hives, itching, headache, coughing, shortness of breath, or swelling of the face, tongue, or throat, may be avoided by the approach described herein.

The present invention therefore provides means for reducing the side effects of CAR therapy and CAR cell product exhaustion, by enabling spatial, temporal, and persevering tumor-specific effects, achieved preferably by systemic administration of the cells, but exerted in a tissue specific manner due to CAR expression under the appropriate conditions.

As a further positive effect in the avoidance or reduction of side effects in CAR therapy, a significantly lower cytokine production is observed after antigen exposure by a CD3 zeta deficient CAR integrated in the CD247 gene, compared to a prior art CAR.

In one embodiment, the genetically modified cell is a CD4+ and/or CD8+ T cell, preferably a mixture of CD4+ and CD8+ T cells. These T cell populations, and preferably the composition comprising both CD4+ and CD8+ transformed cells, show particularly effective cytolytic activity against various solid or liquid tumors.

In one embodiment, the modified cell comprises CD4+ and CD8+ T cells, preferably in a ration of 1:10 to 10:1, more preferably in a ratio of 5:1 to 1:5, 2:1 to 1:2 or 1:1. Administration of anti-tumor-antigen-directed modified CAR-T cells expressing the CAR fragment described herein, for example anti-CD19 CAR fragment, at the ratios mentioned, preferably at a 1:1 CD4+/CD8+ ratio, lead to beneficial characteristics during treatment of the diseases mentioned herein, for example these ratios lead to improved therapeutic response and reduced toxicity (refer to Examples 8 and 11).

In one embodiment, the genetically modified cell carrying a nucleic acid construct encoding a CD3 zeta-deficient CAR fragment in another gene locus than CD3 zeta/CD247 provides no or not therapeutically relevant cytotoxicity levels.

In one embodiment, the genetically modified cell is allogeneic with respect to a patient, preferably an allogeneic T-cell, NK-cell or Treg.

In one embodiment, the genetically modified cell is autologous with respect to a patient, preferably an autologous NK-cell.

In some embodiments, cells can be obtained from a subject different from the intended patient, therefore allogeneic cells. As used herein, a cell is "allogeneic" with respect to a subject if it or one of its progenitor cells is derived from another subject of the same species. As used herein, a cell is "autologous" with respect to a subject if it or its progenitor cells originate from the same subject.

For the use of "off-the-shelf' allogeneic or autologous CAR-T cells or CAR-NK cells, preferably CAR-NK cells, the inventors developed a method of engineering a tumor-antigen expressing CAR-T cells or CAR-NK cells that exhibit fewer unwanted side effects, if any, than allogeneic CAR-expressing cells to date.

Allogeneic "off the shelf" CAR-T cell products, as described in the present invention, can be produced from cells of healthy donors in stock and are applicable to a large patient population, depending on the indication and/or tumor-specific antigen. The means provided by the invention enable to reduce the cost of individual doses of CAR-T cell products and failures in the production of CAR-T cells. In particular, the start of therapy is accelerated to the benefit of the patient (refer to Example 12).

The most serious risk associated with the use of allogeneic T-cell products is an often fatal acute graft-versus-host disease (GvHD), whereby the donor's (CAR-modified) T-cells recognize and attack the patient's tissue as "foreign" through their endogenous TCR. Due to the beneficial properties of the cells of the invention these risks are reduced compared to known approaches.

Transplantation of autologous or allogeneic cells expressing the fusion CAR of the invention is feasible. For example, when reintroduced back to patients after autologous cell transplantation, the T cells modified with the CAR fragment of the invention as described herein may recognize and kill tumor cells (refer to Example 9).

Employing CAR-NK cells allows to reduce risks for a graft versus host disease in an allogeneic cell context. The CAR-NK cell product can be preferably used as allogeneic "off the shelf" product. The present invention enables a surprisingly safe and advantageous use of allogeneic immune cells, in particular of allogeneic CAR-T cells, by their beneficial loss of TCR presentation on the cell surface, as described above, and the intracellular, tumor-dependent regulated expression of the CAR/CD3 zeta fusion protein in the CAR-T cell or CAR-NK cell, as described here.

The invention further relates to a chimeric antigen receptor (CAR) polypeptide expressed from a genetically modified cell as described herein, comprising a chimeric antigen receptor (CAR) as an in-frame fusion protein comprising an exogenous CAR fragment without a sequence encoding a functional intracellular domain of a CD3 zeta protein, and an intracellular domain of the endogenous CD3 zeta/CD247 gene, said intracellular domain comprising one or more immunoreceptor tyrosine-based activation motifs (ITAMs).

In one embodiment, the CAR fragment comprises:
- a CAR signal sequence, preferably according to SEQ ID NO 4, or a sequence with at least 80% sequence identity to SEQ ID NO 4, and
- a single chain variable fragment, for example a murine single chain variable fragment specific to the human CD19 antigen, preferably according to SEQ ID NO 5 and 7, or a sequence with at least 80% sequence identity to SEQ ID NO 5 and 7, and
- an immunoglobulin heavy chain constant region or a spacer of a CAR, preferably according to SEQ ID NO 8-10, or a sequence with at least 80% sequence identity to SEQ ID NO 8-10; and
- a CD28 signalling domain, preferably according to SEQ ID NO 11 and 12 or a sequence with at least 80% sequence identity to SEQ ID NO 11 and 12; wherein the CD28 signaling domain comprises a transmembrane domain, preferably according to SEQ ID NO 11, or a sequence with at least 80% sequence identity to SEQ ID NO 11.

In some embodiments, the construct may further comprise
- a truncated CD3 zeta signaling domain including a stop signal for protein translation, preferably according to SEQ ID NO 21, or a sequence with at least 80% sequence identity to SEQ ID NO 21, or
- a truncated CD3 zeta signaling domain lacking a stop signal for protein translation, preferably according to SEQ ID NO 18, or a sequence with at least 80% sequence identity to SEQ ID NO 18, or

In further embodiments, the invention relates to a CAR polypeptide that comprises one or more exogenous linker, an exogenous spacer, an exogenous transmembrane, and exogenous and endogenous signaling domains. In one embodiment, the CAR comprises an intracellular domain, which comprises an exogenous co-stimulatory domain and an endogenous signaling (activation) domain, wherein the endogenous signaling domain is preferably a CD3 zeta domain.

The exchange of exogenous signaling domains meets the demands for either a strong and rapid effector phase (CD28 co-stimulatory domain), or a long-lasting relapse control as secured by a T cell memory population (4-1BB signaling domain). As demonstrated herein, the various signaling domains may be exchanged in multiple configurations, providing a CAR with flexibility with respect to its design without loss of the advantageous binding properties.

Due to the variants (by adding alternative components) employed as the linker, spacer, transmembrane and intracellular domains, it becomes apparent that the various components may be exchanged at required by the skilled person, and the tumor-antigen binding properties may be maintained, thereby maintaining the desired biological effects.

It is also possible to exchange the intracellular ITAM comprising protein domain as required, for example the CD3 chains epsilon, gamma and delta are known to a skilled person.

In some embodiments, the use of a "TCR complex specific" promoter may show a synergistic effect in combination with the CAR-T cell or CAR-NK cell activation signal, cell specificity, and cell expansion with respect to reduction of unwanted systemic side effects. The CAR-T cell or CAR-NK of the present invention migrates towards inflammatory, in particular tumor, tissue. The use of a promoter for the expression of the cytokine that is preferentially expressed under conditions of inflammation or of being present in tumor tissue further enhances the reduction in systemic expression in a synergistic manner, thereby providing surprising benefits (refer to Example 7).

The invention further relates to a pharmaceutical composition comprising a genetically modified cell as described herein and a pharmaceutically acceptable carrier.

Treatment with the genetically modified cell according to the invention can in some embodiments be combined with one or more anti-cancer treatments or medicaments, preferably selected from the group of antibody therapy, vaccines, oncolytic viral therapy, chemotherapy, radiation therapy, cytokine therapy, dendritic cell therapy, gene therapy, hormone therapy, laser light therapy, immune suppression or transplantation.

The invention relates further to methods of treatment of the medical conditions described herein, comprising typically the administration of a therapeutically effective amount of the CD3 zeta deficient CAR fragment complemented with an endogenous CD3 zeta domain, or immune cell expressing said CAR, to a patient in need of said treatment.

The invention further relates to a pharmaceutical composition comprising a genetically modified cell according the invention described herein and a pharmaceutically acceptable carrier. The composition described herein may be administered to a patient subcutaneously, intradermally, intratumorally, intranodally, intramedullary, intramuscularly, by intravenous or intralymphatic injection, or intraperitoneally.

All features described in the present specification may be employed to define any other embodiment or aspect of the invention, for example, features used to describe the cell may be used to describe the construct, and vice versa. Similarly, features used to describe the methods of the invention may be used to describe the cells or constructs, and vice versa.

### DETAILED DESCRIPTION OF THE INVENTION

An important function of the immune system is to recognize and eliminate tumors. Tumor antigens are either specifically expressed on tumor cells and not found on non-pathogenic cells or abnormally expressed, e.g. at least twice above the level found in non-pathogenic cells, such as CD19. Antigens, specifically found on tumor cells, may appear foreign to the immune system and their presence may cause the immune cells to attack the transformed tumor cells.

The response of the immune system to tumors is to destroy abnormal cells with the help of killer T cells, natural killer (NK) cell, sometimes with helper T cells. Tumor antigens are presented on MHC class I molecules in a similar way to viral antigens. This enables killer T cells (CD8+ T cells) to recognize the tumor cell as abnormal. MHC class II molecules presented tumor antigens stimulate T helper cells (CD4+ T cells). NK cells also kill tumor cells in a similar way, especially if the tumor cells have fewer MHC class I molecules on their surface than normal; this is a common phenomenon in tumors. Some tumor cells also release products that inhibit the immune response, for example by secreting the cytokine TGF-β, which suppresses the activity of macrophages and lymphocytes. Cytokine-induced killer cells (CIK) are a group of immune effector cells that exhibit a hybrid T- and NK-like phenotype.

The present invention, as described herein, therefore provides means for enabling and/or enhancing an anti-tumor immune response by the expression of a chimeric antigen receptor (CAR) directed against a tumor antigen, e.g. CD19, in a genetically modified immune cell, e.g. T lymphocytes, cytokine-induced killer cell (CIK), NK cell. The expression of said CAR is controlled by a specific endogenous promoter, while abandoning TCR complexes in said genetically modified immune cell, as described herein.

Immunotherapy in the context of the present invention is to be understood to comprise any therapeutic agent employing the immune system for cancer treatment. Immunotherapy takes advantage of the fact that cancer cells have subtly different molecules on their surface, which can be recognized by the immune system. Immunotherapy encompasses, without limitation, cellular and antibody therapy. Cellular therapies typically involve the administration of immune cells isolated from the blood or from a tumor of the patient. Immune cells directed towards the tumor to be treated are activated, cultured and returned to the patient where the immune cells attack the cancer. Cell types that can be used in this way are, without limitation, natural killer cells, lymphokine-activated killer cells, cytotoxic T cells, and dendritic cells. Dendritic cell therapy provokes anti-tumor responses by causing dendritic cells to present tumor antigens. Dendritic cells present antigens to lymphocytes, which activates them, priming them to kill other cells that present the antigen.

The present invention therefore provides a strategy for immune cell therapy that uses gene editing methods to preferably integrate one or more therapeutic transgenes under the control of at least one endogenous promoters and expression termination signals enabling cell-specific and endogenously controlled expression in therapeutic immune cells. The various aspects and embodiments of the invention, in addition to their advantages over the prior art, are described above.

### Chimeric Antigen Receptors:

In various embodiments, genetically modified receptors, which redirect the cytotoxicity of immune effector cells are provided. These genetically engineered receptors are referred to herein as chimeric antigen receptors (CARs). CARs are molecules that combine antibody-based specificity for a desired antigen (e.g., CD19) with a T cell receptor-activating intracellular domain to generate a chimeric protein that exhibits a specific anti-gene cellular immune activity, e.g. against CD19. As used herein, the term, "chimeric," describes being composed of parts of different proteins or DNAs from different origins.

According to the present invention, a CAR comprises an extracellular antigen-binding domain, comprising an antibody or antibody fragment that binds a target antigen, a transmembrane domain, and an intracellular domain. CARs are typically described as comprising an extracellular ectodomain (antigen-binding domain) derived from an antibody and an endodomain comprising signaling modules originally derived from T cell signaling proteins.

In a preferred embodiment, the ectodomain preferably comprises variable regions from the heavy and light chains of an immunoglobulin configured as a single-chain variable fragment (scFv). The scFv is preferably attached to a hinge region that provides flexibility and transduces signals through an anchoring transmembrane moiety to an intracellular signaling domain. The transmembrane domains originate preferably from either CD8a or CD28. In the first generation of CARs the signaling domain consists of the zeta chain of the TCR complex. The term "generation" refers to the structure of the intracellular signaling domains. Second generation CARs are equipped with a single costimulatory domain originated from CD28 or 4-1 BB. Third generation CARs already include two costimulatory domains, e.g. CD28, 4-1 BB, ICOS or OX40, CD3 zeta. Fourth generation CARs additionally deliver immune stimulatory proteins and/or checkpoint regulatory proteins. The fifth CAR generation incorporates control elements for the regulation of CAR expression, which allows to induce and/or completely switch off the CAR expression or CAR expressing cells, e.g. by suicide genes. The present invention preferably relates to a third, fourth or fifth generation CAR.

The methods of the present invention comprise the administration of cells designed to express a CAR. In some embodiments, the ligand or extracellular ligand binding domain is selected so that the cell expressing the CAR is targeted to a cancer cell or tumor. Any cancer antigen can be a tumor antigen and any tumor antigen can be a caner antigen. The extracellular antigen-binding domain of a CAR is usually derived from a monoclonal antibody (mAb) or from receptors or their ligands.

CARs contemplated herein, comprise an extracellular domain (also referred to as a binding domain or antigen-binding domain) that binds to a preferred target protein, a transmembrane domain, and an intracellular domain, or intracellular signaling domain. "Target protein" and "target antigen" are used interchangeably. In one embodiment, the preferred target protein is CD19. Engagement of the anti-CD19 antigen binding domain of the CAR with CD19 on the surface of a target cell results in clustering of the CAR and delivers an activation stimulus to the CAR-containing cell.

In various embodiments, a CAR comprises an extracellular binding domain that comprises a humanized CD19-specific binding domain; a transmembrane domain; one or more intracellular signaling domains. In particular embodiments, a CAR comprises an extracellular binding domain that comprises a humanized anti-CD19 antigen binding fragment thereof; one or more spacer domains; a transmembrane domain; one or more intracellular signaling domains. The "extracellular antigen-binding domain" or "extracellular binding domain" are used interchangeably and provide a CAR with the ability to specifically bind to the target antigen of interest, CD19. The binding domain may be derived either from a natural, synthetic, semisynthetic, or recombinant source. Preferred in some embodiments are scFv domains or VHH (variable heavy chain only) domain fragments.

"Specific binding" is to be understood as via one skilled in the art, whereby the skilled person is clearly aware of various experimental procedures that can be used to test binding and binding specificity. Methods for determining equilibrium association or equilibrium dissociation constants are known in the art. Some cross-reaction or background binding may be inevitable in many protein- protein interactions; this is not to detract from the "specificity" of the binding between CAR and epitope. By way of example, "specific binding" describes binding of an e.g. anti-CD19 antibody or antigen binding fragment thereof (or a CAR comprising the same) to CD19 at greater binding affinity than background binding. The term "directed against" is also applicable when considering the term "specificity" in understanding the interaction between antibody and epitope.

An "antigen (Ag)" refers to a compound, composition, or substance that can stimulate the production of antibodies or a T cell response in an animal. In particular embodiments, the target antigen is an epitope of a CD19 polypeptide. An "epitope" refers to the region of an antigen to which a binding agent binds. Epitopes can be formed both from contiguous amino acids or noncontiguous amino acids juxtaposed by tertiary folding of a protein. Any suitable antigen can be selected depending on the type of cancer.

"Single-chain Fv" or "scFv" antibody fragments comprise the VH and VL domains of an antibody, wherein these domains are present in a single polypeptide chain and in either orientation {e.g., VL-VH or VH-VL). Generally, the scFv polypeptide further comprises a polypeptide linker between the VH and VL domains which enables the scFv to form the desired structure for antigen binding. In preferred embodiments, a CAR contemplated herein comprises antigen-specific binding domain that is a scFv and may be a murine, human or humanized scFv. Single chain antibodies may be cloned from the V region genes of a hybridoma specific for a desired target.

In particular embodiments, the antigen-specific binding domain that is a humanized scFv that binds a human CD19 polypeptide. An illustrative example of a variable heavy chain that is suitable for constructing anti-CD19 CARs contemplated herein include, but are not limited to the amino acid sequence set forth in SEQ ID NO: 5-7. An illustrative example of a variable light chain that is suitable for constructing anti-CD19 CARs contemplated herein include, but is not limited to the amino acid sequence set forth in SEQ ID NO: 5-7.

### Antibodies and antibody fragments:

The CAR comprises an extracellular antigen-binding domain, comprising for example an antibody or antibody fragment that binds CD19 polypeptide. Antibodies or antibody fragments of the invention therefore include, but are not limited to polyclonal, monoclonal, bispecific, human, humanized or chimeric antibodies, single chain fragments (scFv), single variable fragments (ssFv), single domain antibodies (such as VHH fragments from nanobodies), Fab fragments, F(ab')2 fragments, fragments produced by a Fab expression library, anti-idiotypic antibodies and epitope-binding fragments or combinations thereof of any of the above, provided that they retain similar binding properties of the CAR described herein, preferably comprising the corresponding CDRs, or VH and VL regions as described herein. Also mini-antibodies and multivalent antibodies such as diabodies, triabodies, tetravalent antibodies and peptabodies can be used in a method of the invention. The immunoglobulin molecules of the invention can be of any class (i.e. IgG, IgE, IgM, IgD and IgA) or subclass of immunoglobulin molecules. Thus, the term antibody, as used herein, also includes antibodies and antibody fragments comprised by the CAR of the invention, either produced by the modification of whole antibodies or synthesized de novo using recombinant DNA methodologies.

As used herein, an "antibody" generally refers to a protein consisting of one or more polypeptides substantially encoded by immunoglobulin genes or fragments of immunoglobulin genes. Where the term "antibody" is used, the term "antibody fragment" may also be considered to be referred to. The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. The basic immunoglobulin (antibody) structural unit is known to comprise a tetramer or dimer. Each tetramer is composed of two identical pairs of polypeptide chains, each pair having one "light" (L) (about 25 kD) and one "heavy" (H) chain (about 50-70 kD). The N-terminus of each chain defines a variable region of about 100 to 1 10 or more amino acids, primarily responsible for antigen recognition. The terms "variable light chain" and "variable heavy chain" refer to these variable regions of the light and heavy chains respectively. Optionally, the antibody or the immunological portion of the antibody, can be chemically conjugated to, or expressed as, a fusion protein with other proteins.

The CARs of the invention are intended to bind against mammalian, in particular human, protein targets. The use of protein names may correspond to either mouse or human versions of a protein.

Affinities of binding domain polypeptides and CAR proteins according to the present disclosure can be readily determined using conventional techniques, e.g., by competitive ELISA (enzyme-linked immunosorbent assay), or by binding association, or displacement assays using labeled ligands, or using a surface-plasmon resonance device such as the Biacore.

A variable region of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen-binding site of antibodies.

### Additional components of the CAR

In certain embodiments, the CARs contemplated herein may comprise linker residues between the various domains, added for appropriate spacing and conformation of the molecule, for example a linker comprising an amino acid sequence that connects the VH and VL domains and provides a spacer function compatible with interaction of the two sub-binding domains so that the resulting polypeptide retains a specific binding affinity to the same target molecule as an antibody that comprises the same light and heavy chain variable regions. CARs contemplated herein, may comprise one, two, three, four, or five or more linkers. In particular embodiments, the length of a linker is about 1 to about 25 amino acids, about 5 to about 20 amino acids, or about 10 to about 20 amino acids, or any intervening length of amino acids. Illustrative examples of linkers include glycine polymers; glycine-serine polymers; glycine-alanine polymers; alanine-serine polymers; and other flexible linkers known in the art, such as the Whitlow linker. Glycine and glycine-serine polymers are relatively unstructured, and therefore may be able to serve as a neutral tether between domains of fusion proteins such as the CARs described herein. In particular embodiments, the binding domain of the CAR is followed by one or more "spacers" or "spacer polypeptides," which refers to the region that moves the antigen binding domain away from the effector cell surface to enable proper cell/cell contact, antigen binding and activation. In certain embodiments, a spacer domain is a portion of an immunoglobulin, including, but not limited to, one or more heavy chain constant regions, e.g., CH2 and CH3. The spacer domain can include the amino acid sequence of a naturally occurring immunoglobulin hinge region or an altered immunoglobulin hinge region. In one embodiment, the spacer domain comprises the CH2 and CH3 domains of IgG1 or IgG4. In one embodiment the Fc-binding domain of such a spacer/hinge region is mutated in a manner that prevents binding of the CAR to Fc-receptors expressed on macrophages and other innate immune cells.

The binding domain of the CAR may in some embodiments be followed by one or more "hinge domains," which play a role in positioning the antigen binding domain away from the effector cell surface to enable proper cell/cell contact, antigen binding and activation. A CAR may comprise one or more hinge domains between the binding domain and the transmembrane domain (TM). The hinge domain may be derived either from a natural, synthetic, semi-synthetic, or recombinant source. The hinge domain can include the amino acid sequence of a naturally occurring immunoglobulin hinge region or an altered immunoglobulin hinge region. Illustrative hinge domains suitable for use in the CARs described herein include the hinge region derived from the extracellular regions of type 1 membrane proteins such as CD8 alpha, CD4, CD28, PD1, CD 152, and CD7, which may be wild-type hinge regions from these molecules or may be altered. In another embodiment, the hinge domain comprises a PD1, CD 152, or CD8 alpha hinge region.

The "transmembrane domain" is the portion of the CAR that fuses the extracellular binding portion and intracellular signaling domain and anchors the CAR to the plasma membrane of the immune effector cell. The TM domain may be derived either from a natural, synthetic, semi-synthetic, or recombinant source. The TM domain may be derived from the alpha, beta or zeta chain of the T cell receptor, CD3s, O'Ω3ζ, CD4, CD5, CD8 alpha, CD9, CD16, CD22, CD27, CD28, CD33, CD37, CD45, CD64, CD80, CD86, CD134, CD137, CD152, CD154, and PD1 . In one embodiment, the CARs contemplated herein comprise a TM domain derived from CD8 alpha or CD28.

In particular embodiments, CARs contemplated herein comprise an intracellular signaling domain. An "intracellular signaling domain," refers to the part of a CAR that participates in transducing the message of effective anti-CD19 CAR binding to a human CD19 polypeptide into the interior of the immune effector cell to elicit effector cell function, e.g., activation, cytokine production, proliferation and cytotoxic activity, including the release of cytotoxic factors to the CAR-bound target cell, or other cellular responses elicited with antigen binding to the extracellular CAR domain. The term "effector function" refers to a specialized function of an immune effector cell. Effector function of the T cell, for example, may be cytolytic activity or help or activity including the secretion of a cytokine. Thus, the term "intracellular signaling domain" refers to the portion of a protein which transduces the effector function signal and that directs the cell to perform a specialized function. CARs contemplated herein comprise one or more co-stimulatory signaling domains to enhance the efficacy, expansion and/or memory formation of T cells expressing CAR receptors. As used herein, the term, "co-stimulatory signaling domain" refers to an intracellular signaling domain of a costimulatory molecule. Co-stimulatory molecules are cell surface molecules other than antigen receptors or Fc receptors that provide a second signal required for efficient activation and function of T lymphocytes upon binding to an antigen. The costimulatory molecule is a cell surface molecule other than an antigen receptor or its ligand that contributes to an efficient immune response. Costimulatory molecules include MHC class I molecules, BTLA and Toll ligand receptors, OX40, CD27, CD28, CDS, ICAM-1, LFA-1 (CD11a / CD18), ICOS (CD278) and 4-1BB (CD137). Including, but not limited to, further examples of such costimulatory molecules are CDS, ICAM-1, GITR, BAFFR, HVEM (LIGHTTR), SLAMF7, NKp80 (KLRF1), NKp44, NKp30, NKp46, CD160, CD19, CD4, CD8 alpha, CD8 beta , IL2Rbeta, IL2Rgamma, IL7Ralpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE / RANKL, DNAM1 (CD 26), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG / Cbp, CD19a and ligands that specifically bind to CD83,.

A costimulatory intracellular signaling domain can be the intracellular portion of a costimulatory molecule. In one embodiment, the CAR comprises an intracellular domain, which comprises a costimulatory domain and a signaling (activation) domain. The CAR construct may therefore include an intracellular signaling domain (CD3 zeta) of the native T cell receptor complex and one or more co- stimulatory domains that provide a second signal to stimulate full T cell activation. Costimulatory domains are thought to increase CAR-T cell cytokine production and facilitate T cell replication and T cell persistence. Co-stimulatory domains have also been shown to potentially prevent CAR-T cell exhaustion, increase T cell anti-tumor activity, and enhance survival of CAR-T cells in patients. As a non-limiting example, CAR constructs with the 4-1 BB co-stimulatory domain have been associated with gradual, sustained expansion and effector function, increased persistence, and enriched central memory cells (TCM) in the T cell subset composition in preclinical studies. 4-1 BB is a member of the tumor necrosis factor (TNF) superfamily, and it is an inducible glycoprotein receptor in vivo that is primarily expressed on antigen-activated CD4 and CD8 T cells. As a non-limiting example, CD28 is member of the immunoglobulin (Ig) superfamily. It is constitutively expressed on resting and activated CD4 and CD8 T cells and plays a critical role in T cell activation by stimulating the PI3K- AKT signal transduction pathway. In one embodiment, the intracellular domain comprises both 4- 1 BB and CD28 co-stimulatory domains. Other costimulatory domains comprise ICOS and OX40 that can be combined with the CD3 zeta signaling (activation) domain.

In some non-limiting embodiments, an intracellular domain of a CAR may additionally comprise at least one co-stimulating signal domain. Such a co-stimulatory signal domain can result in increased activation of a T cell.

The polypeptide domains described herein may be useful in an anti-cancer CAR to mediate a desired function such as that of a signal peptide, antigen, binding domain, transmembrane domain, intracellular signal domain and/or co-stimulating domain. Potential desirable functions may comprise, but are not limited to, providing a signal peptide and/or transmembrane domain.

The cytokines described herein may relate to any mammalian cytokine corresponding to the cytokine named herein. Preferably, the cytokines relate to the human cytokines, or mouse cytokines. Cancer immunotherapy attempts to stimulate the immune system to reject and destroy tumors. Initially, immunotherapy treatments involved administration of cytokines such as "Interleukin", as described herein.

Checkpoint inhibitors, also known as immune checkpoint modulators, are designed to lessen the effectiveness of checkpoint proteins. They may have a variety of mechanisms of action, but if effective, they enable the immune system to recognize other molecules on the surface of the cancer cells. The checkpoint inhibitor of the immune response is selected from the group consisting of: PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta.

CAR-regulating proteins can also be proteins that induce or completely switch off a CAR expression or a cell expressing a CAR. In another embodiment, the CAR-T cells or CAR-NK cells additionally comprise one or more immune suppression defeating proteins and/or an inducible suicide gene that provokes CAR-T cell or CAR-NK cell death allowing their selective destruction. In some cases, it may be desirable to provide a safety mechanism that allows selective deletion of the administered genetically modified cells, as the manipulated cells can spread and persist for years after administration. Therefore, the method of the invention in some embodiments may include the transformation of preferably the T cells, NK cells or Tregs with a recombinant suicide gene. This recombinant suicide gene can be used to preferably reduce the risk of direct toxicity and/or uncontrolled proliferation of these T cells, NK cells or Tregs after administration to a subject. Suicide genes also enable the selective deletion of transformed cells in vivo. In particular, the suicide gene has the ability to convert a non-toxic prodrug into a cytotoxic drug or to express the toxic gene expression product. In other words, "suicide gene" is preferably a nucleic acid that codes for a product, whereby the product itself or in the presence of other compounds causes cell death.

### TCR, CD3 and CD3 zeta

A "T cell receptor" (TCR) is a heterodimer of the TCR alpha and TCR beta chains. The TCR complex is formed by TCR and CD3 gamma, CD3 delta, CD3 epsilon and CD3 zeta. The interruption or decreased expression of one or more of the chains T cell receptor alpha, T cell receptor beta, CD3 gamma, CD3 delta, CD3 epsilon or CD3 zeta can be used to reduce or prevent the formation of a functional T cell receptor (TCR) complex. By reducing or preventing the formation of a functional TCR complex, the T cell no longer mediates an immune response via its TCR complex. In an embodiment, a nucleic acid encoding a CAR is integrated at a site inside the cell's genome that interrupts or reduces the expression of a T cell receptor alpha chain, T cell receptor beta chain, CD3 gamma chain, CD3 delta chain, CD3 epsilon chain or CD3 zeta chain. While the reduction of one of the proteins of the TCR complex may be sufficient, it is understood that more than one component of the TCR complex can be reduced if desired.

Antigen-presenting cells activate the T cell receptor (TCR) and TCR-mediated signals are transmitted by the CD3 chains zeta, delta, epsilon, and gamma across the cell membrane. All CD3 chains contain immune receptor tyrosine-based activation motifs (ITAMs) in their cytoplasmic domain. CD3 zeta is part of the TCR-CD3 complex, which is present on the surface of T lymphocyte cells and plays an essential role in the adaptive immune response. CD3 zeta is encoded by the CD247 gene. Only endogenous CD3-zeta comprises three ITAMs and transmits an activation signal to the cell, e.g. a cell of the lymphatic line, such as a T-cell, after binding to the antigen. In some embodiments, a CAR comprises an intracellular CD3-zeta signal domain that can activate or stimulate a T-cell. The expression of the CD3 zeta/CD427 gene is the rate-limiting factor in the formation of the TCR complex and the CD247 expression is determining for the TCR complex assembly rate and function. It is understood that a CD3 zeta nucleic acid molecule refers to a polynucleotide encoding a CD3 zeta polypeptide.

### Polypeptides

"Peptide" "polypeptide", "polypeptide fragment" and "protein" are used interchangeably, unless specified to the contrary, and according to conventional meaning, i.e., as a sequence of amino acids. Polypeptides are not limited to a specific length, e.g., they may comprise a full length protein sequence or a fragment of a full length protein, and may include post-translational modifications of the polypeptide, for example, glycosylations, acetylations, phosphorylations and the like, as well as other modifications known in the art, both naturally occurring and non-naturally occurring.

In various embodiments, the CAR polypeptides contemplated herein comprise a signal (or leader) sequence at the N-terminal end of the protein, which co-translationally or post-translationally directs transfer of the protein. Polypeptides can be prepared using any of a variety of well-known recombinant and/or synthetic techniques. Polypeptides contemplated herein specifically encompass the CARs of the present disclosure, or sequences that have deletions from, additions to, and/or substitutions of one or more amino acid of a CAR as disclosed herein.

An "isolated peptide" or an "isolated polypeptide" and the like, as used herein, refer to in vitro isolation and/or purification of a peptide or polypeptide molecule from a cellular environment, and from association with other components of the cell, i.e., it is not significantly associated with in vivo substances. Similarly, an "isolated cell" refers to a cell that has been obtained from an in vivo tissue or organ and is substantially free of extracellular matrix.

### Fusion protein, fusion gene

"Fusion proteins" or "chimeric proteins" as used herein, comprise proteins formed by joining two or more nucleic acid segments that originally encoded separate proteins, polypeptides or protein fragments. "Fusion gene", "gene fusion" or "fused gene" are according to the invention preferably joined nucleic acid sequence segments, wherein at least one nucleic acid sequence is also an exogenous nucleic acid sequence and at least one other nucleic acid sequence is also an endogenous nucleic acid sequence. In preferred embodiments, the exogenous nucleic acid is integrated into and fused to an endogenous nucleic acid sequence, e.g. an endogenous gene. In some embodiments, the integrated exogenous nucleic acid sequence to be fused may have several combined gene structures and/or polypeptide-encoding sequences separated by one or more protein separation sites.

The translation of a fusion gene results in a single or multiple polypeptides with functional properties derived from each of the original proteins or protein fragments. In a further embodiment, a fused gene may contain one or more nucleic acid sequences encoding a polypeptide, wherein the polypeptides may be present as individual polypeptides or proteins after translation, with separation being mediated by a protein separation site.

Chimera or chimeras refer to hybrid proteins made up of polypeptides with different functions or attributes. In some embodiments, fusion proteins combine whole peptides and therefore contain all functional domains of the original proteins. In certain embodiments, fusion proteins combine only parts of coding sequences and therefore only retain parts of the original functions of the genes from which they were formed.

A gene fusion can also cause regulatory changes that alter when, to what extent, and where these genes are expressed. In some embodiments, the rearrangement of different active sites and/or binding domains can additionally lead to the formation of proteins with new functions and/or specificities. A nucleic acid sequence encoding a CD3 zeta-deficient CAR fragment and a protein separation site may be fused within an endogenous gene, downstream of a promoter of an endogenous gene, within or at the junction site of an endogenous exon or within or at the junction site of an endogenous intron.

### Nucleic acids

As used herein, the terms "polynucleotide" or "nucleic acid molecule" refers to messenger RNA (mRNA), RNA, genomic RNA (gRNA), plus strand RNA (RNA(+)), minus strand RNA (RNA(-)), genomic DNA (gDNA), complementary DNA (cDNA) or recombinant DNA. Polynucleotides include single and double stranded polynucleotides. Preferably, polynucleotides of the invention include polynucleotides or variants having at least about 50%, 55%, 60%, 65%, 70%, 75%, 80%, 85%, 90%, 95%, 96%, 97%, 98%, 99% or 100%) sequence identity to any of the reference sequences described herein, typically where the variant maintains at least one biological activity of the reference sequence.

In various illustrative embodiments, the present invention contemplates, in part, polynucleotides comprising expression vectors, viral vectors, and transfer plasmids, and compositions, and cells comprising the same. Polynucleotides can be prepared, manipulated and/or expressed using any of a variety of well- established techniques known and available in the art. In order to express a desired polypeptide, a nucleotide sequence encoding the polypeptide, can be inserted into appropriate vector. Examples of vectors are plasmid, autonomously replicating sequences, and transposable elements. Additional exemplary vectors include, without limitation, plasmids, phagemids, cosmids, artificial chromosomes such as yeast artificial chromosome (YAC), bacterial artificial chromosome (BAC), or PI-derived artificial chromosome (PAC), bacteriophages such as lambda phage or MI 3 phage, and animal viruses. Examples of categories of animal viruses useful as vectors include, without limitation, retrovirus (including lentivirus), adenovirus, adeno-associated virus, herpesvirus {e.g., herpes simplex virus), poxvirus, baculovirus, papillomavirus, and papovavirus {e.g., SV40). Examples of expression vectors are pClneo vectors (Promega) for expression in mammalian cells; pLenti4/V5- DEST^{™}, pLenti6/V5-DEST^{™}, and pLenti6.2/V5-GW/lacZ (Invitrogen) for lentivirus-mediated gene transfer and expression in mammalian cells. In particular embodiments, the coding sequences of the chimeric proteins disclosed herein can be ligated into such expression vectors for the expression of the chimeric protein in mammalian cells. The "control elements" or "regulatory sequences" present in an expression vector are those non-translated regions of the vector - origin of replication, selection cassettes, promoters, enhancers, translation initiation signals (Shine Dalgarno sequence or Kozak sequence) introns, a polyadenylation sequence, 5' and 3' untranslated regions - which interact with host cellular proteins to carry out transcription and translation. Such elements may vary in their strength and specificity. Depending on the vector system and host utilized, any number of suitable transcription and translation elements, including ubiquitous promoters and inducible promoters may be used.

### Vectors

In particular embodiments, a cell (e.g., an immune effector cell, such as a T cell) is transfected with a no-viral vector, a retroviral vector, e.g., a lentiviral vector, preferably a non-viral vector encoding a CAR. For example, an immune effector cell is transfected with a vector encoding a CAR that comprises a humanized anti-CD19 antibody or antigen binding fragment that binds a CD19 polypeptide, with a transmembrane and intracellular signaling domain, such that these transduced cells can elicit a CAR-mediated cytotoxic response.

The term "vector" is used herein to refer to a nucleic acid molecule capable transferring or transporting another nucleic acid molecule. The transferred nucleic acid is generally linked to, e.g., inserted into, the vector nucleic acid molecule. A vector may include sequences that direct autonomous replication in a cell, or may include sequences sufficient to allow integration into host cell DNA. Useful vectors include, for example, plasmids (e.g., DNA plasmids or RNA plasmids), transposons, cosmids, bacterial artificial chromosomes, and viral vectors. Useful viral vectors include, e.g., replication defective retroviruses and lentiviruses. In further embodiments of the invention, a gene editing technique, preferably CRISPR-Cas, mediated integration of the CD19 CAR encoding nucleic acid into a host genome may be employed. Appropriate vectors for CRISPR/Cas and TALEN-mediated insertion are known to a skilled person. In certain embodiments, the gene editing techniques include zinc finger nucleases (ZFNs), integrases, site specific recombinases, meganucleases, homing endonucleases, or TALENs, more preferably a CRISPR-Cas9 derived from *Streptococcus pyogenes.*

As will be evident to one of skill in the art, the term "viral vector" is widely used to refer either to a nucleic acid molecule (e.g., a transfer plasmid) that includes virus-derived nucleic acid elements that typically facilitate transfer of the nucleic acid molecule or integration into the genome of a cell or to a viral particle that mediates nucleic acid transfer. Viral particles will typically include various viral components and sometimes also host cell components in addition to nucleic acid(s).

In a preferred embodiment the invention therefore relates to a method for transfecting cells with an expression vector encoding a CAR. For example, in some embodiments, the vector comprises additional sequences, such as sequences that facilitate expression of the CAR, such a promoter, enhancer, poly-A signal, and/or one or more introns. In preferred embodiments, the CAR-coding sequence is flanked by transposon sequences, such that the presence of a transposase allows the coding sequence to integrate into the genome of the transfected cell.

In some embodiments, the genetically transformed cells are further transfected with a transposase that facilitates integration of a CAR coding sequence into the genome of the transfected cells. In some embodiments the transposase is provided as DNA expression vector. However, in preferred embodiments, the transposase is provided as an expressible RNA or a protein such that long-term expression of the transposase does not occur in the transgenic cells. For example, in some embodiments, the transposase is provided as an mRNA (e.g., an mRNA comprising a cap and poly- A tail). Any transposase system may be used in accordance with the embodiments of the present invention. However, in some embodiments, the transposase is salmonid-type Tel -like transposase (SB). For example, the transposase can be the so called "Sleeping beauty" transposase, see e.g., U.S. Patent 6,489,458, incorporated herein by reference. In some embodiments, the transposase is an engineered enzyme with increased enzymatic activity. Some specific examples of transposases include, without limitation, SB 10, SB 1 1 or SB 100X transposase (see, e.g., Mates et al, 2009, Nat Genet. 41 (6):753-61, or US9228180, herein incorporated by reference). For example, a method can involve electroporation of cells with an mRNA encoding an SB 10, SB 1 1 or SB 100X transposase.

### Gene Editing and transfer

A gene editing technique can be used to interfere with the endogenous expression of any expressed structure on a genome, preferably the TCR complex component, CD5, CD7, CD52, MHC and/or a molecule involved in a control point gene. "Endogenous" as used herein is particularly used to define a gene expression that originates from within a system such as a host, organism, tissue, or cell whereas "exogenous" particularly defines a genetic sequence introduced from externally into a cell by one or more genetic, biochemical or other methods.

Gene editing is a type of genetic engineering in which DNA is inserted, deleted or replaced in the genome of a living organism by manipulated nucleases, or "molecular Scissors". These nucleases generate site-specific double-strand breaks (DSBs) at desired sites in the genome. The induced double-strand breaks are repaired by non-homologous end compound (HEJ) or homologous recombination (HR), resulting in targeted mutations ("edits"). Five families of manipulated nucleases can be used for gene editing: (a) meganucleases, (b) zinc finger nuclei (ZFNs), (c) transcription activator-like effector-based nucleases (TALENs), (d) Mega-TALENs, and (e) the CRISPR-Cas system.

In certain embodiments, CRISPR-Cas9 is preferably used for gene editing. A "targeting sequence", "target sequence" or "target site" mainly refers to a chromosomal or extrachromosomal nucleic acid sequence that defines a part of a nucleic acid to which a binding molecule binds and/or cleaves, provided there are sufficient conditions for binding and/or cleavage. In any of these gene editing approaches inducing double-strand breaks, the target sequence comprises an identical sequence to a recognition site of the endogenous DNA sequence into which an integration is intended, preferably selected from the group consisting of a homology arm, a guide RNA target site, a restriction enzyme recognition site, a ZFN recognition site, a ZFN cleaving site, a TALEN DNA-binding site, a recombinase recognition site, an integrase site and/or a homing nuclease recognition site Techniques using each of these artificial nucleases are well known among experts. For example, when cells are edited with a genome editing complex (e.g., a TALEN, CRISPR/Cas9, ZFN, Mega-TALENs, or meganucleases) to introduce a functional gene or to knock out a gene, nucleases (e.g., Fokl or Cas9) induce a double strand break at the site of modification. Upon induction of the double strand break, a protein, such as a DNA repair protein, e.g., phosphorylated (serl778) 53BP1 (p53BPI) or gH2AC can accumulate at the site of the double strand break and is indicative of a DNA damage response.

One or more of these techniques can be used alone or in any combination to disrupt endogenous expression of a TCR-complex component in the method of the invention. When the gene editing technique is used to disrupt the expression of a TCR complex component, the gene-editing technology can particularly target one or more of the following: (i) a CD3 zeta locus, (ii) a safe harbor locus, (iii) a TRAC locus; (ii) a locus controlling TCR-beta expression; (iii) a locus controlling TCR gamma expression; (iv) a locus that controls TCR delta expression; (v) a locus that controls the expression of a CD3 chain. CD3 chains are described above. The CD3 chain can be CD3 zeta, CD3 epsilon, CD3 delta or CD3 gamma. In certain embodiments, genome loci harboring genetic information for expressing at least one ITAM domain are preferably target for gene editing.

In some embodiments, a genetically modified cell or animal comprising an inactivated genomic sequence may be termed a "knock-out" or a "conditional knock-out." Similarly, a genetically modified cell or animal comprising an integrated sequence may be termed a "knock-in" or a "conditional knock-in".

### Protein separation site

The term "protein separation site", as used herein, comprise nucleic acid sequence elements that trigger separation of a bi-cistronic or a multi-cistronic expression of protein sequences in order to obtain distinctively expressed proteins. A protein separation site can, without limitation, refer to a self-cleavage peptide, an internal ribosome entry site (IRES), and/or a proteolytic cleavage site. In a preferred aspect of the invention, the nucleic acid construct can optionally be designed to include a protein separation site, preferably upstream of the nucleic acid sequences encoding a CAR or a CAR fragment. In particular embodiments, nucleic acid sequences considered herein, comprise one or more nucleic acid sequences regions of interest that encode one or more peptides and/or proteins. To achieve efficient translation of each of the numerous proteins, nucleic acid sequences regions can be separated by one or more IRES equivalents or nucleic acid sequences encoding for a self-cleaving peptide or a proteolytic cleavage site.

A "self-cleavage peptide" or "self-cleaving peptide", e.g. P2A, can mediate ribosomal skipping during protein translation in a cell.

The term "internal ribosome entry site" or "IRES", as used herein, refers to an element that promotes direct internal ribosome entry into the initiation codon, such as ATG, of a cistron (a protein coding region), resulting in RNA cap-independent translation of the gene. Examples of IRES generally used by professionals are those described in U.S. Pat. Pat. No. 6,692,736. Examples of "IRES" that are known to the scientific community include IRES that can be obtained from picornaviruses and IRES that can be obtained from viral or cellular mRNA sources, such as the immunoglobulin heavy chain binding protein (BiP), vascular endothelial growth factor (VEGF), fibroblast growth factor 2 (FGF-2) and insulin-like growth factor (IGFII), translational initiation factor elF4G and yeast transcription factors TFIID and HAP4, encephalomy-carditis virus (EMCV) commercially available from Novagen, and VEGF IRES IRES have also been detected in viral genomes of Picomaviridae, Dicistroviridae and Flaviviridae species as well as in HCV, Friend murine leukemia virus (FrMLV) and Moloney murine leukemia virus (MoMLV).

The term "proteolytic cleavage site" refers to the recognition nucleic acid sequence for an intracellular proteolytic cleavage enzyme breaking the peptide bonds between amino acids in proteins.

### TCR complex component promoter

The term "promoter" is used for a DNA sequence that initiates and regulates transcription from the DNA downstream of it through binding of proteins. The transcribed RNA may encode a protein. Promoters are preferably located near the transcription start sites of genes, upstream of the coding DNA (towards the 5' region of the sense strand).

In some embodiments, it may be preferable to use a cell-type, cell lineage or tissue-specific expression control sequence to achieve cell-type, lineage or tissue-specific expression of a desired polynucleotide sequence (e.g. to express a particular nucleic acid encoding a peptide only in a subset of cell types, cell lines or tissues or during certain stages of development).

In aspects of the invention, a method is provided in which the regulation of an integrated transgene is placed under an endogenous promoter, whereby a nucleic acid construct of the transgene to be integrated is preferably promoter-deficient. In some embodiments, the nucleic acid construct of the transgene to be integrated encoding for a CAR or a CAR fragment, is preferably promoter-deficient. One such construct enables the integration of the transgene, encoding a CAR or a CAR fragment, into a location within the genome such that the integrated nucleic acid sequence, i.e. the transgene, is under the control of an endogenous promoter. In some embodiments, the endogenous promoter is a promoter of a TCR complex component. In some embodiments, the said endogenous promoter is preferably a promoter of genes encoding proteins of the TCR complex, e.g. a CD3 zeta, CD3 epsilon, CD3 delta, CD3 gamma, TCR alpha chain or TCR beta chain, preferably a gene encoding protein comprising at least one intracellular ITAM domain.

### Expression termination signal

"Expression termination signal" comprises any structural elements at RNA and/or DNA level that specifies an end of transcription and/or translation, and/or a polyadenylation of the heterologous nucleic acid transcripts. The transcriptional stop is usually defined by a terminator, a nucleic acid sequence that defines the end of a transcription (e.g. of a gene) and initiates the release of the newly synthesized RNA. Terminators are located downstream of the gene to be transcribed and occur directly after 3' regulatory elements, such as the poly-A signal. The term "poly-A site", "poly-A signal" or "poly-A sequence", as used herein, refers to a nucleic acid sequence that signals both, termination and polyadenylation, of the nascent RNA transcript by RNA polymerase II. Polyadenylation sequences can enhance mRNA stability by adding a poly-A tail at the 3' end of the coding sequence, thus contributing to increased translation efficiency. Efficient polyadenylation of the recombinant transcript is preferable because transcripts without a poly-A tail are unstable and degrade rapidly. A "stop codon" is also a sequence of three consecutive nucleotides (termed triplet) within the messenger RNA that signals the termination of a protein translation process at the ribosome. In aspects of the invention, the CAR transgene preferably uses endogenous expression termination signals, including a poly-A signal, a stop codon for RNA translation into protein, and a transcriptional stop signal.

### Integrated sequence

An "integrated sequence" or "integrating sequence" particularly refers to a nucleic acid construct including coding and/or non-coding elements to be inserted into the genome of the transfected cell. In some embodiments, an edited chromosomal sequence may comprise an integrated sequence. The integrated sequence may code for an endogenous protein, an exogenous or heterologous protein, a wild-type protein, a modified protein, a fusion protein or similar. In some embodiments, the integrated sequence is also a transgene. In a specific aspect of the invention, different transgenes are integrated at the different integration sites. An "integration site" or "fusion site" as used herein is the exact nucleic acid position within a genome where a double-strand break is generated and a nucleic acid construct is incorporated into the genome by homology directed repair at the position of the double-strand break. An endogenous sequence can be interrupted by integrating an exogenous sequence, so that the exogenous sequence is under the control of the endogenous promoter. In some embodiments the integrated exogenous sequence and/or the integrated exogenous sequence would be expressed together with an endogenous sequence. An integrated protein coding sequence can also be placed under the control of an endogenous promoter and/or fused in-frame with an endogenous protein coding sequence. In addition, the integrated sequence can also serve as a control element. Accordingly, the integrated sequence may be endogenous or exogenous to the cell. The exogenous sequence may be a homologue of or related to an endogenous sequence. The exogenous sequence can also be a human sequence. An animal or cell with such an integrated sequence can be called a "knock-in". In certain embodiments, a sequence can be integrated to change the expression pattern of a protein of interest. For example, a conditional knock-out system can be created. In aspects of the invention, an integrated sequence can be a nucleic acid sequence encoding a CAR, a CAR fragment, a protein separation site, a CAR regulatory protein and/or an immune stimulatory protein. In some embodiments, the nucleic acid construct integrated into the genome of the transfected cell comprise a CAR fragment and a protein separation site upstream of said CAR fragment.

### Nucleic acids and transfer methods

Methods for introducing exogenous molecules, e.g. nucleic acid sequences, into cells are well known to experts and include lipid-mediated transfer (i.e. liposomes, including neutral and cationic lipids), electroporation, direct injection, cell fusion, particle bombardment, biopolymer nanoparticles, calcium phosphate co-precipitation, DEAE-dextran-mediated transfer and viral vector-mediated transfer. "Gene transfer", "nucleic acid transfer", gene sequence transfer", "transgene transfer" are used interchangeably. In some embodiments, nucleic acid constructs are preferably transferred into cells by electroporation.

A "gene" refers to a DNA region that encodes a gene product including regions regulating the production of the gene product, regardless of whether these sequences are adjacent to coding and/or transcribed sequences. A gene comprises, but is not limited to, promoter sequences, terminators, translational regulatory sequences such as ribosome binding sites and internal ribosome entry sites, enhancers, silencers, insulators, boundary elements, replication origins, matrix attachment sites, and locus control regions.

"Gene expression" is particularly the conversion of the information encoded by a gene into a gene product. A gene product can be the direct transcription product of a gene (e.g., mRNA, tRNA, rRNA, antisense RNA, ribozyme, structural RNA or any other type of RNA) or a protein produced by translation of a mRNA. Gene products also include RNAs modified by processes such as capping, polyadenylation, methylation, and editing, as well as proteins modified by processes such as methylation, acetylation, phosphorylation, ubiquitination, ADP-ribosylation, myristoylation, and glycosylation.

### Sequence Variation:

Sequence variants of the claimed nucleic acids, proteins, antibodies, antibody fragments and/or CARs, for example those defined by % sequence identity, that maintain similar binding properties of the invention are also included in the scope of the invention. Such variants, which show alternative sequences, but maintain essentially the same binding properties, such as target specificity, as the specific sequences provided are known as functional analogues, or as functionally analogous.

Sequence identity relates to the percentage of identical nucleotides or amino acids when carrying out a sequence alignment. The recitation "sequence identity" as used herein refers to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Phe, Tyr, Trp, Lys, Arg, His, Asp, Glu, Asn, Gin, Cys and Met) occurs in both sequences to yield the number of matched positions, dividing the number of matched positions by the total number of positions in the window of comparison (i.e., the window size), and multiplying the result by 100 to yield the percentage of sequence identity. Included are nucleotides and polypeptides having at least about 50%, 55%, 60%, 65%, 70%, 75%, 80%, 85%, 90%, 95%, 96%, 97%, 98%, 99% or 100% sequence identity to any of the reference sequences described herein, typically where the polypeptide variant maintains at least one biological activity of the reference polypeptide.

It will be appreciated by those of ordinary skill in the art that, as a result of the degeneracy of the genetic code, there are many nucleotide sequences that encode a polypeptide as described herein. Some of these polynucleotides bear minimal homology or sequence identity to the nucleotide sequence of any native gene. Nonetheless, polynucleotides that vary due to differences in codon usage are specifically contemplated by the present invention. Deletions, substitutions and other changes in sequence that fall under the described sequence identity are also encompassed in the invention.

Protein sequence modifications, which may occur through substitutions, are also included within the scope of the invention. Substitutions as defined herein are modifications made to the amino acid sequence of the protein, whereby one or more amino acids are replaced with the same number of (different) amino acids, producing a protein which contains a different amino acid sequence than the primary protein. Substitutions may be carried out that preferably do not significantly alter the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function.

In general, the non-polar amino acids Gly, Ala, Val, lie and Leu; the non-polar aromatic amino acids Phe, Trp and Tyr; the neutral polar amino acids Ser, Thr, Cys, Gin, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

Substitution variants have at least one amino acid residue in the antibody molecule removed and a different residue inserted in its place. The sites of greatest interest for substitutional mutagenesis include the hypervariable regions, but framework alterations are also contemplated. If such substitutions result in a change in biological activity, then more substantial changes, denominated "exemplary substitutions" in the table immediately below, or as further described below in reference to amino acid classes, may be introduced and the products screened.

**Potential Amino Acid Substitutions:**

| **Original residue** | **Preferred conservative substitutions** | **Examples of exemplary substitutions** |
|---|---|---|
| Ala (A) | Val | Val; Leu; Ile |
| Asg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn, Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain.

Conservative amino acid substitutions are not limited to naturally occurring amino acids, but also include synthetic amino acids. Commonly used synthetic amino acids are omega amino acids of various chain lengths and cyclohexyl alanine which are neutral non-polar analogues; citrulline and methionine sulfoxide which are neutral non-polar analogues, phenylglycine which is an aromatic neutral analog; cysteic acid which is a negatively charged analog and ornithine which is a positively charged amino acid analogue. Like the naturally occurring amino acids, this list is not exhaustive, but merely exemplary of the substitutions that are well known in the art.

### Genetically modified cells and Immune cells

The present invention contemplates, in particular embodiments, cells genetically modified to express the CARs contemplated herein, for use in the treatment of medical conditions. As used herein, the term "genetically engineered" or "genetically modified" refers to the addition of extra genetic material in the form of DNA or RNA into the total genetic material in a cell. Genetic modifications can be carried out selectively or not at a specific location in the genome of a cell. In an embodiment, the genetic change is site-specific. In an embodiment, the genetic modification is not site-specific. The terms, "genetically modified cells"," genetically modified immune cells", "modified cells," and, "redirected cells," are used interchangeably. As used herein, the term "gene therapy" refers to the introduction-permanently or transiently- of extra genetic material in the form of DNA or RNA into the total genetic material in a cell that restores, corrects, or modifies expression of a gene, or for the purpose of expressing a therapeutic polypeptide, e.g., a CAR. In particular embodiments, the CARs contemplated herein are introduced and expressed in immune effector cells so as to redirect their specificity to a target antigen of interest, e.g., a CD19 polypeptide.

An "immune cell" or "immune effector cell" is any cell of the immune system that has one or more effector functions (e.g., cytotoxic cell killing activity, secretion of cytokines, induction of ADCC and/or CDC). An immune effector cell can be also differentiated from iPSCs (induced pluripotent stem cells) or derived from peripheral human blood and/or human cord blood.

Immune effector cells of the invention can be autologous/autogeneic ("self") or non-autologous ("non- self," e.g., allogeneic, syngeneic or xenogeneic). "Autologous", as used herein, refers to cells from the same subject, and represent a preferred embodiment of the invention. "Allogeneic," as used herein, refers to cells of the same species that differ genetically to the cell in comparison.

"Syngeneic," as used herein, refers to cells of a different subject that are genetically identical to the cell in comparison. "Xenogeneic," as used herein, refers to cells of a different species to the cell in comparison. In preferred embodiments, the cells of the invention are autologous or allogeneic. Illustrative immune effector cells used with the CARs contemplated herein include T lymphocytes. The terms "T cell" or "T lymphocyte" are art-recognized and are intended to include thymocytes, immature T lymphocytes, mature T lymphocytes, resting T lymphocytes, cytokine-induced killer cells (CIK cells) or activated T lymphocytes. Cytokine-induced killer (CIK) cells are typically CD3- and CD56-positive, non-major histocompatibility complex (MHC)- restricted, natural killer (NK)-like T lymphocytes. A T cell can be a T helper (Th; CD4+ T cell) cell, for example a T helper (Th) cell, such as a TH1, TH2, TH3, TH17, TH9 or TFH cell. The T cell can be a cytotoxic T cell (CTL; CD8+ T cell), CD4+CD8+ T cell, CD4 CD8 T cell, or a regulatory T cell (reg) or any other subset of T cells. The T cell can be a naive, effector, memory, effector storage, central storage, memory stem T cell. The T cell can be an umbilical cord blood cell. The T cell can be a peripheral lymphocyte. The T-cell may be expanded from PBMNCs. The T-cell may be autologous with respect to an individual to whom it is to be administered. The T-cell may be allogeneic with respect to an individual to whom it is to be administered. The T-cell may be partially HLA incompatible with an individual to whom it is to be administered.

"Immortalized", as used herein, refers to immortalized cells as a population of cells that would not normally reproduce for an unlimited number of cell cycles. Due to a mutation, immortal cells escape from a normal cellular senescence and instead keep undergoing the cell proliferation. The mutation can occur spontaneously or induced by UV light, genetic manipulation, or entry by a virus, toxin or bacteria into the cell. The cells can therefore be cultivated in vitro over a longer period of time for experimental, therapeutic or medical purposes. Said immortalized immune cells include K13, αβ T cells, γδ T cells, NK cells, NKT cells, NK-92 and YT cells, stem cells, or stem cell-derived cells including cells of the aforementioned immune system, preferably NKT cells, NK-92 cells, YT cells, and NK cells. An immortalized T cell line may retains its lytic function.

Other illustrative populations of T cells suitable for use in particular embodiments include naive T cells and memory T cells and stem cell-like memory cells TSCM).

For example, when reintroduced back to patients after autologous cell transplantation, the T cells modified with the CAR of the invention as described herein may recognize and kill tumor cells. CIK cells may have enhanced cytotoxic activity compared to other T cells, and therefore represent a preferred embodiment of an immune cell of the present invention.

As would be understood by the skilled person, other cells may also be used as immune effector cells with the CARs as described herein. In particular, immune effector cells also include NK cells, NKT cells, neutrophils, and macrophages. Immune effector cells also include progenitors of effector cells wherein such progenitor cells can be induced to differentiate into an immune effector cells in vivo or in vitro. Progenitors can be iPSCs that become immune effector cells under defined culture conditions. The present invention provides methods for making the immune effector cells which express the CAR contemplated herein. In one embodiment, the method comprises transfecting or transducing immune effector cells isolated from an individual such that the immune effector cells express one or more CAR as described herein. In certain embodiments, the immune effector cells are isolated from an individual and genetically modified without further manipulation in vitro. Such cells can then be directly re-administered into the individual. In further embodiments, the immune effector cells are first activated and stimulated to proliferate in vitro prior to being genetically modified to express a CAR. In this regard, the immune effector cells may be cultured before and/or after being genetically modified (i.e., transduced or transfected to express a CAR contemplated herein).

In particular embodiments, prior to in vitro manipulation or genetic modification of the immune effector cells described herein, the source of cells is obtained from a subject. In particular embodiments, the CAR-modified immune effector cells comprise T cells. T cells can be obtained from a number of sources including, but not limited to, peripheral blood mononuclear cells, bone marrow, lymph nodes tissue, cord blood, thymus issue, tissue from a site of infection, ascites, pleural effusion, spleen tissue, and tumors. In certain embodiments, T cells can be obtained from a unit of blood collected from a subject using any number of techniques known to the skilled person, such as sedimentation, e.g., FICOLL^{™} separation, antibody-conjugated bead-based methods such as MACS^{™} separation (Miltenyi). In one embodiment, cells from the circulating blood of an individual are obtained by apheresis. The apheresis product typically contains lymphocytes, including T cells, monocytes, granulocyte, B cells, other nucleated white blood cells, red blood cells, and platelets. In one embodiment, the cells collected by apheresis may be washed to remove the plasma fraction and to place the cells in an appropriate buffer or media for subsequent processing. The cells can be washed with PBS or with another suitable solution that lacks calcium, magnesium, and most, if not all other, divalent cations. As would be appreciated by those of ordinary skill in the art, a washing step may be accomplished by methods known to those in the art, such as by using a semiautomated flow through centrifuge. For example, the Cobe 2991 cell processor, the Baxter CytoMate, or the like. After washing, the cells may be resuspended in a variety of biocompatible buffers or other saline solution with or without buffer. In certain embodiments, the undesirable components of the apheresis sample may be removed in the cell directly resuspended culture media.

In certain embodiments, T cells are isolated from peripheral blood mononuclear cells (PBMCs) by lysing the red blood cells and depleting the monocytes, for example, by centrifugation through a PERCOLL^{™} gradient. A specific subpopulation of T cells can be further isolated by positive or negative selection techniques. One method for use herein is cell sorting and/or selection via negative magnetic immunoadherence or flow cytometry that uses a cocktail of monoclonal antibodies directed to cell surface markers present on the cells negatively selected.

PBMC may be directly genetically modified to express CARs using methods contemplated herein. In certain embodiments, after isolation of PBMC, T lymphocytes are further isolated and in certain embodiments, both cytotoxic and helper T lymphocytes can be sorted into naive, memory, and effector T cell subpopulations either before or after genetic modification and/or expansion. CD8+ cells can be obtained by using standard methods. In some embodiments, CD8+ cells are further sorted into naive, central memory, and effector cells by identifying cell surface antigens that are associated with each of those types of CD8+ cells.

In some embodiments, the immune cell of the present invention, for example the T cells described herein, can be obtained from inducible pluripotent stem cells (iPSCs) using methods known to a skilled person. Accepted approaches for producing CAR-T cells rely on the genetic modification and expansion of mature circulating T cells. Such processes utilize autologous T cells and reduce risk of graft-versus- host (GvHD) disease from allogeneic T cells through endogenous TCR expression as well as rejection through MHC incompatibility. As an alternative, direct in vitro differentiation of engineered T cells from pluripotent stem cells, such as inducible pluripotent stem cells, provides an essentially unlimited source of cells that can be genetically modified to express the CAR of the present invention. In some embodiments, a so-called master iPSC line can be maintained, which represents a renewable source for consistently and repeatedly manufacturing homogeneous cell products. In some embodiments, the transformation of a master iPSC cell line with the CAR encoding nucleic acid is contemplated, prior to expansion and differentiation to the desired immune cell, preferably T cell or NK cell. T lymphocytes can for example be generated from iPSCs, such that iPSCs could be modified with the CAR or the CD3 zeta-deficient CAR fragment encoding nucleic acids and subsequently expanded and differentiated to T cells for administration to the patient. Differentiation to the appropriate immune cell, such a T cell, could also be conducted from the iPSCs before transformation with the CAR or the CD3 zeta-deficient CAR encoding nucleic acids and expansion prior to administration. All possible combinations of iPSC expansion, genetic modification and expansion to provide suitable numbers of cells for administration are contemplated in the invention.

The immune effector cells, such as T cells, NK cells, CIK cells or Tregs, can be genetically modified following isolation using known methods, or the immune effector cells can be activated and expanded (or differentiated in the case of progenitors) in vitro prior to being genetically modified. In a particular embodiment, the immune effector cells, such as T cells, NK cells, CIK cells or Tregs, are genetically modified with the CD3 zeta-deficient CAR fragment contemplated herein (e.g., transfected with a nucleic acid and pre-complexed Cas9 enzyme or transduced with a viral vector comprising a nucleic acid encoding a CD3 zeta-deficient CAR fragment) and then are activated and expanded in vitro. In various embodiments, T cells can be activated and expanded before or after genetic modification to express a CAR or a CD3 zeta-deficient CAR fragment, using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

In a further embodiment, a mixture of, e.g., one, two, three, four, five or more, different expression vectors can be used in genetically modifying a donor population of immune effector cells wherein each vector encodes a different chimeric antigen receptor protein as contemplated herein. The resulting modified immune effector cells forms a mixed population of modified cells, with a proportion of the modified cells expressing more than one different CAR protein.

In one embodiment, the invention provides a method of storing genetically modified murine, human or humanized CAR protein expressing immune effector cells which target a CD19 protein, comprising cryopreserving the immune effector cells such that the cells remain viable upon thawing. A fraction of the immune effector cells expressing the CAR proteins can be cryopreserved by methods known in the art to provide a permanent source of such cells for the future treatment of patients afflicted with the B cell, T cell, NK cell, dendritic cell (DC), cytotoxic induced killer cell (CIK) related condition. When needed, the cryopreserved transformed immune effector cells can be thawed, grown and expanded for more such cells.

### Upstream/ downstream

"Upstream" and "downstream" as used herein, are employed in the context of the 5'→3' direction of a nucleic acid strand. Upstream refers to nucleotides towards the 5' end and downstream towards the 3' end of any given nucleic acid.

### In-frame, out-of-frame

A fusion product is defined as 'in-frame' when an open reading frame remains intact in the 3'-region downstream of the nucleic acid integration site, regardless of the number of inserted or deleted amino acids at the fusion junction point. A shift in the open reading frame of a protein coding gene sequence refers to "out-of-frame".

### Compositions and Formulations

The compositions contemplated herein may comprise one or more polypeptides, polynucleotides, vectors comprising same, genetically modified immune effector cells, etc., as contemplated herein. Compositions include, but are not limited to, pharmaceutical compositions.

A "pharmaceutical composition" refers to a composition formulated in pharmaceutically-acceptable or physiologically- acceptable solutions for administration to a cell or an animal, either alone, or in combination with one or more other modalities of therapy. It will also be understood that, if desired, the compositions of the invention may be administered in combination with other agents as well, such as, e.g., cytokines, growth factors, hormones, small molecules, chemotherapeutics, prodrugs, drugs, antibodies, or other various pharmaceutically-active agents. There is virtually no limit to other components that may also be included in the compositions, provided that the additional agents do not adversely affect the ability of the composition to deliver the intended therapy.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein "pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, surfactant, or emulsifier which has been approved by the United States Food and Drug Administration as being acceptable for use in humans or domestic animals.

Plasmids, nucleic acids, nucleic acids complexed with a Cas enzyme, AAV vectors, lentiviral vectors and/or other compositions, agents, drugs, biologics (proteins) can be incorporated into pharmaceutical compositions, e.g., pharmaceutically acceptable carriers or excipients. Such pharmaceutical compositions are particularly useful for administration and delivery to a subject in vivo or ex vivo.

In particular embodiments, compositions of the present invention comprise an amount of CAR-expressing immune effector cells contemplated herein. As used herein, the term "amount" refers to "an amount effective" or "an effective amount" of a genetically modified therapeutic cell, e.g., T cell, NK cell, CIK cell, or Treg cell, to achieve a beneficial or desired prophylactic or therapeutic result, including clinical results.

A "prophylactically effective amount" refers to an amount of a genetically modified therapeutic cell effective to achieve the desired prophylactic result. Typically but not necessarily, since a prophylactic dose is used in subjects prior to or at an earlier stage of disease, the prophylactically effective amount is less than the therapeutically effective amount. The term prophylactic does not necessarily refer to a complete prohibition or prevention of a particular medical disorder. The tern prophylactic also refers to the reduction of risk of a certain medical disorder occurring or worsening in its symptoms.

A "therapeutically effective amount" of a genetically modified immune cell may vary according to factors such as the disease state, age, sex, and weight of the individual, and the ability of the stem and progenitor cells to elicit a desired response in the individual. A therapeutically effective amount is also one in which any toxic or detrimental effects of the virus or transduced or transfected therapeutic cells are outweighed by the therapeutically beneficial effects. The term "therapeutically effective amount" includes an amount that is effective to "treat" a subject {e.g., a patient). When a therapeutic amount is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells or CIK cells or NK cells or Treg cells described herein may be administered at a dosage of 10² to 10¹⁰ cells/kg body weight, preferably 10⁵ to 10⁷ cells/kg body weight, including all integer values within those ranges. The number of cells will depend upon the ultimate use for which the composition is intended as will the type of cells included therein. For uses provided herein, the cells are generally in a volume of a liter or less, can be 500 mL or less, even 250 mL or 100 mL or less. Hence the density of the desired cells is typically greater than 10⁶ cells/ml and generally is greater than 10⁷ cells/mL, generally 10⁸ cells/mL or greater. The clinically relevant number of immune cells can be apportioned into multiple infusions that cumulatively equal or exceed 10⁵, 10⁶, 10⁷, 10⁸, 10⁹, 10¹⁰, 10¹¹, or 10¹² cells. In some aspects of the present invention, particularly since all the infused cells will be redirected to a particular target antigen, lower numbers of cells may be administered. CAR expressing cell compositions may be administered multiple times at dosages within these ranges. The cells may be allogeneic, syngeneic, xenogeneic, or autologous to the patient undergoing therapy.

Generally, compositions comprising the cells activated and expanded as described herein may be utilized in the treatment and prevention of diseases that arise in individuals who are immunocompromised. In particular, compositions comprising the CAR-modified T cells, CAR-modified NK cells, CAR-modified CIK cells or CAR-modified Treg cells contemplated herein are used in the treatment of cancer, preferably solid or liquid, more preferably solid malignancies, more preferably rectal cancer, lung cancer, breast cancer, liver cancer, pancreatic cancer, stomach cancer, and ovarian cancer, more preferably metastatic tumor cells positive for CD19. The CAR-modified T cells, CAR-modified NK cells, CAR-modified CIK cells or CAR-modified Treg cells of the present invention may be administered either alone, or as a pharmaceutical composition in combination with carriers, diluents, excipients, and/or with other components such as interleukins or other immune response stimulating cytokines e.g. IL-15 and/or checkpoint inhibitory molecules, e.g. PD1 polypeptide or a PD1-antibody, or cell populations.

In particular embodiments, pharmaceutical compositions contemplated herein comprise an amount of genetically modified T cells, in combination with one or more pharmaceutically or physiologically acceptable carriers, diluents or excipients. In particular embodiments, pharmaceutical compositions contemplated herein comprise an amount of genetically modified NK cells, genetically modified CIK cells or genetically modified Treg cells in combination with one or more pharmaceutically or physiologically acceptable carriers, diluents or excipients.

Pharmaceutical compositions of the present invention comprising a CAR-expressing immune effector cell population, such as T cells or NK cells or CIK cells or Treg cells, may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for parenteral administration, e.g., intravascular (intravenous or intraarterial), intraperitoneal or intramuscular administration.

The liquid pharmaceutical compositions, whether they are solutions, suspensions or other like form, may include one or more of the following: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. An injectable pharmaceutical composition is preferably sterile.

In a particular embodiment, compositions contemplated herein comprise an effective amount of CAR-expressing immune effector cells, alone or in combination with one or more therapeutic agents. Thus, the CAR-expressing immune effector cell compositions may be administered alone or in combination with other known cancer treatments, such as radiation therapy, chemotherapy, transplantation, immunotherapy, hormone therapy, photodynamic therapy, etc. The compositions may also be administered in combination with antibiotics. Such therapeutic agents may be accepted in the art as a standard treatment for a particular disease state as described herein, such as a particular cancer. Exemplary therapeutic agents contemplated include cytokines, growth factors, steroids, NSAIDs, DMARDs, anti-inflammatories, chemotherapeutics, radiotherapeutics, therapeutic antibodies, or other active and ancillary agents.

A combined immunotherapy encompasses simultaneous treatment, co-treatment or joint treatment, and includes the administration of genetically modified immune cells expressing a nucleic acid construct encoding a CAR or a CD3 zeta-deficient CAR fragment combined with immunotherapies, such as checkpoint inhibitors and/or immune stimulatory cytokines, whereby treatment may occur within minutes of each other, in the same hour, on the same day, in the same week or in the same month as one another. A combination medicament, comprising one or more of said genetically modified immune cells with another immunotherapeutic, may also be used in order to co-administer the various components in a single administration or dosage. A "medicament" particularly refers to a drug including an immune-therapeutic cell or genetically modified cell used to cure, treat, or prevent a disease.

As used herein, the term "tumor microenvironment" relates to the cellular environment in which any given tumor exists, including the tumor stroma, surrounding blood vessels, immune cells, fibroblasts, other cells, signaling molecules, and the ECM.

### Therapeutic Methods

The genetically modified immune effector cells contemplated herein provide improved methods of adoptive immunotherapy for use in the treatment of medical disorders associated with the presence of pathogenic cells expressing CD19 that include, but are not limited to, immunoregulatory conditions and/or hematological and/or solid malignancies.

As used herein, "medical disorders associated with the presence of pathogenic cells expressing CD19 refer to medical conditions, such as a cancer or an autoimmune disease, in which the cells involved in pathophysiology of the disease demonstrate expression of CD19, and preferably presentation of CD19 on the cell surface. The expression of CD19 can be determined by various methods known to a skilled person, for example by isolating cells from a patient and assessing these by PCR using primers directed CD19 transcripts, immuno-staining with anti CD19 antibodies, or by analysis by flow cytometry. Such pathogenic cells may typically be pathogenic mature B cells and/or memory B cells and/or pathogenic T cells and/or T follicular helper cells and/or tumor stem cells and/or solid tumor cells and/or liquid tumors and/or metastatic cancer cells and/or NK cells and/or CIK cells, and/or Treg cells, and/or dendritic cells.

"Cancer", as used herein, is a disease characterized by the uncontrolled growth of abnormal cells. Cancer refers to any type of cancerous growth or carcinogenic process, metastatic tissue or malignant transformed cells, tissues or organs, regardless of histopathological type or invasive stage. Cancer cells can spread locally or to other parts of the body via the bloodstream and lymphatic system. Cancer cells spreading to other parts to the body are termed "metastatic cells" or "metastatic tumor cells". The terms "tumor" and "cancer", used herein, are utilized interchangeably, e.g. both terms include solid and liquid, e.g. general or circulating tumors, premalignant and malignant cancers and tumors. Examples of liquid cancers include, but not limited to, acute lymphocytic leukemia (ALL), chronic lymphocytic leukemia (CLL), acute myeloid Leukemia (acute myelogenous leukemia (AML), chronic bone marrow cancer, chronic myelogenous leukemia (CML), Hodgkin lymphoma, non-Hodgkin lymphoma, and myeloma. Examples of solid tumors are liver, lung, breast, lymphatic system, digestive organs (e.g. colon), urogenital organs (e.g. kidney, urothelial cells), prostate and throat, malignant organ systems including tumors such as sarcomas, adenocarcinomas and cancer. Examples for breast cancer that can be treated with the are ductal carcinoma in situ (DCIS), lobular carcinoma in situ (LCIS), invasive ductal carcinoma (IDC), invasive ductal carcinoma including tubular, medullary, mucinous, papillary, and cribriform carcinomas, invasive lobular carcinoma (ILC), inflammatory breast cancer, male breast cancer, Paget's Disease of the nipple, phyllodes tumors of the breast, recurrent and/or metastatic breast cancer. Adenocarcinoma includes most malignant tumors such as colon cancer, rectal cancer, renal cell cancer, liver cancer, non-small cell lung cancer, small intestine cancer and esophageal cancer. In certain forms the cancer is a melanoma, e.g. an advanced stage melanoma. Metastatic lesions of the cancer can also be treated or prevented with the methods and compositions of the invention. Examples of other types of cancer that can be treated are bone cancer, pancreatic cancer, skin cancer, head and neck cancer, skin or intraocular melanoma, uterine cancer, ovarian cancer, rectal cancer, anal cancer, stomach cancer, testicular cancer, faropius duct cancer, Endometrial cancer, cervical cancer, vaginal cancer, vulvar cancer, Hodgkin's disease, non-Hodgkin's lymphoma, esophageal cancer, small intestine cancer, endocrine cancer, thyroid cancer, parathyroid cancer, adrenal cancer, soft tissue sarcoma, urethral cancer, penile cancer, acute myeloid leukemia, chronic myelocytic leukemia, acute lymphoblastic leukemia, chronic or acute leukemia including chronic lymphatic leukemia, solid tumor in childhood, lymphatic lymphoma, bladder cancer, kidney or ureter cancer, renal pelvis cancer, neoplasm of the central nervous system (CNS) primary CNS lymphoma, tumor angiogenesis, spinal axis tumor, brainstem glioma, pituitary adenoma, Kaposi's sarcoma, epidermoid carcinoma, squamous cell carcinoma, asbestos-induced T cell lymphoma Including a combination of environmental cancer and cancer including things Treatment of metastatic cancer, e.g. metastatic cancer that expresses PD-L1 (Iwai et al. (2005) Int. Immunol. 17: 133-144) can be performed with the inhibitory molecules described in this invention.

"Cancer-associated antigen" or "tumor antigen" is expressed interchangeably on the surface of cancer cells, either completely or as a fragment (e.g. MHC / peptide), and the drug targets cancer cells preferentially. Molecules (usually proteins, carbohydrates or lipids) that are useful for. Tumor antigen refers to an antigen that is commonly found in a specific hyperproliferative disease. In one aspect, the antigen of hyperproliferative disease of the present invention is a primary or metastatic melanoma, thymoma, lymphoma, sarcoma, lung cancer, liver cancer, non-Hodgkin's lymphoma, Hodgkin's lymphoma, Leukemia, uterine cancer, cervical cancer, bladder cancer, derived from cancers such as kidney and breast cancer, prostate cancer, ovarian cancer, adenocarcinoma such as pancreatic cancer and the like. In certain embodiments, the tumor antigen is a marker expressed on both normal and cancer cells, such as a marker of cell lineage, like CD19 on B cells. In certain embodiments, the tumor antigen is overexpressed in cancer cells compared to normal cells, e.g. 1-fold overexpression, 2-fold overexpression, 3-fold overexpression or more overexpression compared to normal cells. Molecules of the cell surface. In certain forms, a tumor antigen is a cell surface molecule that is inadequately synthesised in cancer cells, e.g. a molecule that contains deletions, additions or mutations compared to a molecule expressed in normal cells. In certain embodiments, the tumor antigen is expressed exclusively, completely or as a fragment (e.g. MHC / peptide) on the cell surface of cancer cells and is not synthesised or expressed on the surface of normal cells. In certain embodiments, a CAR of the invention comprises a CAR containing an antigen binding domain (e.g. an antibody or antibody fragment) which binds to an MHC-presenting peptide. Normally, peptides derived from endogenous proteins fill the pockets of major histocompatibility complex (MHC) class I molecules and are recognized by the T cell receptor (TCR) on CD8 + T lymphocytes. Class I MHC complexes are constitutively expressed by all nucleated cells. In cancer, virus- and/or tumor-specific peptides / MHC complexes represent a unique class of cell surface targets for immunotherapy. TCR-like antibodies have been described which target peptides of virus or tumor antigens associated with human leukocyte antigen (HLA) - A1 or HLA-A2 (see e.g. Sastry et al., J Virol. 2011 85 (5) : 1935-1942; Sergeeva et al, Blood, 2011 117 (16): 4262-4272; Verma et al., J Immunol 2010 184 (4): 2156-2165; Willemsen et al., Gene Ther 2001 8(21): 1601-1608; Dao et al., Sci Transl Med 2013 5 (176): 176ra33; Tassev et al., Cancer Gene Ther 2012 19 (2): 84-100). For example, TCR-like antibodies can be identified by searching a library such as a human scFv phage display library.

In particular embodiments, compositions comprising CAR-modified T cells contemplated herein are used in the treatment of cancer, including but not limited to solid or liquid/hematological malignancies, such as, for example, rectal cancer, lung cancer, breast cancer, liver cancer, pancreatic cancer, stomach cancer, ovarian cancer, and metastatic tumor cells positive for CD19, or hematological malignancies such as, leukemia and lymphoma or myeoloproliferative disorders/neoplasms with or without a leukemic tumor cell dissemination, preferably solid tumors and multiple myeloma.

As used herein "treatment" or "treating," includes any beneficial or desirable effect on the symptoms or pathology of a disease or pathological condition, and may include even minimal reductions in one or more measurable markers of the disease or condition being treated. Treatment can involve optionally either the reduction or amelioration of symptoms of the disease or condition, or the delaying of the progression of the disease or condition. "Treatment" does not necessarily indicate complete eradication or cure of the disease or condition, or associated symptoms thereof.

As used herein, "prevent," and similar words such as "prevented," "preventing" or "prophylactic" etc., indicate an approach for preventing, inhibiting, or reducing the likelihood of the occurrence or recurrence of, a disease or condition. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

In one embodiment, a method of treating a cancer related condition in a subject in need thereof comprises administering an effective amount, e.g., therapeutically effective amount of a composition comprising genetically modified immune effector cells contemplated herein. The quantity and frequency of administration will be determined by such factors as the condition of the patient, and the type and severity of the patient's disease, although appropriate dosages may be determined by clinical trials.

The administration of the compositions contemplated herein may be carried out in any convenient manner, including by aerosol inhalation, injection, ingestion, transfusion, implantation or transplantation. In a preferred embodiment, compositions are administered parenterally. The phrases "parenteral administration" and "administered parenterally" as used herein refers to modes of administration other than enteral and topical administration, usually by injection, and includes, without limitation, intravascular, intravenous, intramuscular, intraarterial, intrathecal, intracapsular, intraorbital, intratumoral, intracardiac, intradermal, intraperitoneal, transtracheal, subcutaneous, subcuticular, intraarticular, subcapsular, subarachnoid, intraspinal and intrasternal injection and infusion. In one embodiment, the compositions contemplated herein are administered to a subject by direct injection into a tumor, lymph node, or site of infection.

### SEQUENCES

**Preferred amino acid sequences of the invention:**

| **Seq _ID NO** | **Homology-Directed DNA Repair (HDR) Template** | **Info HDR-Template Part** | **Amino acid sequence** |
|---|---|---|---|
| 1 | CD247-exon2_CD19CAR (truncated) | Full Sequence | DIQMTQTTSSLSASLGDRVTISCRASQDISKYLNWYQQKPDGTVKLLIYH TSRLHSGVPSRFSGSGSGTDYSLTISNLEQEDIATYFCQQGNTLPYTFG GGTKLEIT |
| 2 | | Left Homology Arm | aqsfglldpklcylldgilfiyrviltalflr |
| 3 | | P2A | ATNFSLLKQAGDVEENPGP |
| 4 | | Membrane Export Signal (Leader) | MALPVTALLLPLALLLHAARP |
| 5 | | FMC63VL (Light Chain, Variable Part) | DIQMTQTTSSLSASLGDRVTISCRASQDISKYLNWYQQKPDGTVKLLIYH TSRLHSGVPSRFSGSGSGTDYSLTISNLEQEDIATYFCQQGNTLPYTFG GGTKLEIT |
| 6 | | (G4S)3 Linker | GGGGSGGGGSGGGGS |
| 7 | | FMC63VH (Heavy Chain, Variable Part) | EVKLQESGPGLVAPSQSLSVTCTVSGVSLPDYGVSWIRQPPRKGLEWL GVIWGSETTYYNSALKSRLTIIKDNSKSQVFLKMNSLQTDDTAIYYCAKH YYYGGSYAMDYWGQGTSVTVSS |
| 8 | | IgG4 Hinge | ESKYGPPCPPCP |
| 9 | | IgG1 CH3 Domain (Hinge) | GQPREPQVYTLPPSRDELTKNQVSLTCLVKGFYPSDIAVEWESNGQPE NNYKTTPPVLDSDGSFFLYSKLTVDKSRWQQGNVFSCSVMHEALHNAY TQKSLSLSPGK |
| 10 | | Linker | KDPK |
| 11 | | CD28 Transmembran e Domain | FWVLVVVGGVLACYSLLVTVAFIIFWV |
| 12 | | CD28 Intracellular Domain | RSKRSRLLHSDYMNMTPRRPGPTRKHYQPYAPPRDFAAYRS |
| 13 | | Right Homology Arm | rvk |
| 14 | CD247-exon2_CD19CAR (full) | Full Sequence | aqsfglldpklcylldgilfiyrviltalflrATNFSLLKQAGDVEENPGPMALPVTALLLPL ALLLHAARPDIQMTQTTSSLSASLGDRVTISCRASQDISKYLNWYQQKP DGTVKLLIYHTSRLHSGVPSRFSGSGSGTDYSLTISNLEQEDIATYFCQQ GNTLPYTFGGGTKLEITGGGGSGGGGSGGGGSEVKLQESGPGLVAPS QSLSVTCTVSGVSLPDYGVSWIRQPPRKGLEWLGVIWGSETTYYNSAL KSRLTIIKDNSKSQVFLKMNSLQTDDTAIYYCAKHYYYGGSYAMDYWGQ GTSVTVSSESKYGPPCPPCPGQPREPQVYTLPPSRDELTKNQVSLTCL VKGFYPSDIAVEWESNGQPENNYKTTPPVLDSDGSFFLYSKLTVDKSR WQQGNVFSCSVMHEALHNAYTQKSLSLSPGKKDPKFWVLVVVGGVLA CYSLLVTVAFIIFWVRSKRSRLLHSDYMNMTPRRPGPTRKHYQPYAPPR DFAAYRSRVKFSRSADAPAYQQGQNQLYNELNLGRREEYDVLDKRRG RDPEMGGKPRRKNPQEGLYNELQKDKMAEAYSEIGMKGERRRGKGH DGLYQGLSTATKDTYDALHMQALPPR* |
| 15 | CD247-exon3_CD19CAR (truncated) | Full Sequence | fsrsadapayATNFSLLKQAGDVEENPGPMALPVTALLLPLALLLHAARPDIQ MTQTTSSLSASLGDRVTISCRASQDISKYLNWYQQKPDGTVKLLIYHTS RLHSGVPSRFSGSGSGTDYSLTISNLEQEDIATYFCQQGNTLPYTFGGG TKLEITGGGGSGGGGSGGGGSEVKLQESGPGLVAPSQSLSVTCTVSG VSLPDYGVSWIRQPPRKGLEWLGVIWGSETTYYNSALKSRLTIIKDNSKS QVFLKMNSLQTDDTAIYYCAKHYYYGGSYAMDYWGQGTSVTVSSESKY GPPCPPCPGQPREPQVYTLPPSRDELTKNQVSLTCLVKGFYPSDIAVE WESNGQPENNYKTTPPVLDSDGSFFLYSKLTVDKSRWQQGNVFSCSV MHEALHNAYTQKSLSLSPGKKDPKFWVLVVVGGVLACYSLLVTVAFIIF WVRSKRSRLLHSDYMNMTPRRPGPTRKHYQPYAPPRDFAAYRSRVKF SRSAdapayqqgqnqlyn |
| 16 | | Left Homology Arm | fsrsadapay |
| 17 | | CAR body | SEQ ID NO 3 - SEQ ID 12 |
| 18 | | truncated CD3-zeta | RVKFSRSA |
| 19 | | Right Homology Arm | dapayqqgqnqlyn |
| 20 | CD247-exon3_CD19CAR (full) | Full Sequence | fsrsadapayATNFSLLKQAGDVEENPGPMALPVTALLLPLALLLHAARPDIQ MTQTTSSLSASLGDRVTISCRASQDISKYLNWYQQKPDGTVKLLIYHTS RLHSGVPSRFSGSGSGTDYSLTISNLEQEDIATYFCQQGNTLPYTFGGG TKLEITGGGGSGGGGSGGGGSEVKLQESGPGLVAPSQSLSVTCTVSG VSLPDYGVSWIRQPPRKGLEWLGVIWGSETTYYNSALKSRLTIIKDNSKS QVFLKMNSLQTDDTAIYYCAKHYYYGGSYAMDYWGQGTSVTVSSESKY GPPCPPCPGQPREPQVYTLPPSRDELTKNQVSLTCLVKGFYPSDIAVE WESNGQPENNYKTTPPVLDSDGSFFLYSKLTVDKSRWQQGNVFSCSV MHEALHNAYTQKSLSLSPGKKDPKFWVLVVVGGVLACYSLLVTVAFIIF WVRSKRSRLLHSDYMNMTPRRPGPTRKHYQPYAPPRDFAAYRSRVKF SRSADAPAYQQGQNQLYNELNLGRREEYDVLDKRRGRDPEMGGKPR RKNPQEGLYNELQKDKMAEAYSEIGMKGERRRGKGHDGLYQGLSTAT KDTYDALHMQALPPR* |
| 21 | CD3-zeta Intracellular Domain + STOP | | RVKFSRSADAPAYQQGQNQLYNELNLGRREEYDVLDKRRGRDPEMGG KPRRKNPQEGLYNELQKDKMAEAYSEIGMKGERRRGKGHDGLYQGLS TATKDTYDALHMQALPPR* |
| 22 | eGFP | | MVSKGEELFTGVVPILVELDGDVNGHKFSVSGEGEGDATYGKLTLKFIC TTGKLPVPWPTLVTTLTYGVQCFSRYPDHMKQHDFFKSAMPEGYVQER TIFFKDDGNYKTRAEVKFEGDTLVNRIELKGIDFKEDGNILGHKLEYNYN SHNVYIMADKQKNGIKVNFKIRHNIEDGSVQLADHYQQNTPIGDGPVLLP DNHYLSTQSALSKDPNEKRDHMVLLEFVTAAGITLGMDELYK* |
| 23 | TRAC-Charité | Left homology arm | iqnpdpavyqlrdskssdksvclft |

**Preferred nucleic acid sequences of the invention:**

| **SEQ ID NO** | **Homology-Directed DNA Repair (HDR) Template** | **Info HDR-Template Part** | **Nucleic acid sequence 5' - 3'** |
|---|---|---|---|
| 24 | CD247-exon2_CD19CA R(truncated) | Full Sequence | GGCAGGAGCTCTGTGCACAGAGAACAGCCTCATCTGCTCGCCTTGTTTC CACCTCCCCTCCCATTGCCCCAGGTTCTTTGGCCCCACAGCGGCCACAT CTGCCGTTGGTGCCAATAGGTTTTCCAGGAGCTGGTTGAGGTGGGAGG GAGGGAGAGGGTTGTGATCAGGCTGAGGCATGGGGATTGGATATAGTC TCCGTGTCATGATTTATTTGGTCAGTCAGTCCTAGTGCCACCCTGGGGTA ATGGGGATGTGTTCTCGTCACCTTGGGCCTGGCTGACCAGCTTTATCTC TTGGCACAGAGGCACAGAGCTTTGGCCTGCTGGATCCCAAACTCTGCTA CCTGCTGGATGGAATCCTCTTCATCTATCGTGTCATTCTCACTGCCTTGT TCCTGAGAGCCACCAACTTCTCTCTTTTGAAGCAGGCCGGAGATGTGGA AGAAAATCCTGGGCCCATGGCTCTTCCTGTGACTGCCCTTCTGCTGCCC CTGGCTCTCCTGCTCCATGCTGCCCGGCCAGACATCCAGATGACACAGA CAACCAGCAGCCTCAGCGCCAGCCTGGGGGACAGAGTGACCATTAGCT GCCGGGCCTCTCAGGACATCAGCAAATACCTGAACTGGTACCAGCAGAA ACCAGATGGCACTGTCAAGCTGCTGATTTACCACACCTCCAGGCTCCAC AGCGGCGTGCCCAGTCGCTTCAGCGGCAGTGGGAGCGGGACAGATTAT TCCCTCACAATCTCCAACCTGGAGCAGGAAGATATTGCCACATACTTCTG CCAGCAAGGCAACACCCTGCCATACACATTTGGAGGCGGCACCAAATTG |
| | Arm | | GAGATCACCGGCGGTGGTGGATCTGGAGGAGGGGGCAGCGGAGGTGG CGGCTCTGAGGTGAAACTGCAGGAGAGTGGCCCTGGCCTGGTGGCTCC CAGCCAGAGCCTTTCTGTCACCTGCACCGTGTCTGGGGTGTCCCTGCCT GACTATGGAGTCTCCTGGATCCGGCAGCCTCCAAGAAAAGGACTGGAAT GGCTGGGCGTCATCTGGGGAAGTGAGACCACCTACTATAATTCAGCCCT CAAGTCCCGGCTCACCATCATTAAGGACAACTCCAAATCCCAGGTGTTC CTGAAGATGAATTCTCTCCAGACTGATGACACAGCCATCTACTACTGTGC CAAGCATTATTATTACGGCGGGTCCTATGCCATGGACTACTGGGGCCAG GGGACCAGTGTCACTGTTTCTTCTGAAAGCAAGTATGGCCCCCCATGCC CTCCCTGCCCCGGACAGCCGCGGGAGCCCCAGGTCTACACCCTCCCAC CCTCCAGAGATGAGCTGACCAAGAACCAGGTTTCACTGACATGCCTGGT GAAGGGCTTCTACCCCTCTGACATTGCTGTGGAGTGGGAGAGCAATGG GCAGCCAGAGAACAACTACAAGACCACACCTCCTGTCCTGGACAGCGAC GGCTCCTTCTTCCTCTACAGTAAGCTCACTGTGGACAAGAGCCGCTGGC AGCAGGGCAATGTCTTCTCCTGCAGCGTGATGCACGAGGCCCTGCACA ATGCCTATACCCAGAAGTCACTCTCTCTGAGCCCTGGAAAGAAGGATCC CAAGTTTTGGGTCCTCGTGGTGGTCGGGGGTGTGCTGGCCTGCTACAG CTTGCTGGTCACAGTGGCCTTCATCATCTTCTGGGTGCGCTCCAAGAGG AGCCGGCTGCTTCACAGTGATTACATGAACATGACCCCCAGGAGGCCAG GACCCACCAGGAAGCACTACCAGCCCTACGCTCCCCCGCGGGACTTTG CTGCTTACCGCAGCAGAGTGAAGGTGGGTACCACTGGGCTTTGGAGG AGGGCACGGGGTCCCCCACTTGATGGATGTTCAGAGGGGCCTTGGTCT TGGAAGGTCTCAAGCTCGGGTGGTGCCTGGGGCTTGGTATCCAGGAGC AAAGCAAGGACCAGCCAAGTGTGTGCCTTGAGTGGGCTGAGGAGGAGG TGGCAGTGTCTGGCTGAGATGGACAGGGTAGGAGGGAGAGCCTGGTGC TAGGCACCTCCATGACAAGCCGTACAAATGTGTGCACATCAGAGTGTCC CAGGGAAGGCGATGCTACTGGTGACAAAGGGGCTTACACTCAGGCAGA GGTCCTTCTTTCCAAGTGTGAATGAAGGCCATGTTAGCCTTTCTCTTGAA AAGGCCCTTTCCTCATCTGTAACTGGGGA |
| 25 | | Left Homology Arm | GGCAGGAGCTCTGTGCACAGAGAACAGCCTCATCTGCTCGCCTTGTTTC CACCTCCCCTCCCATTGCCCCAGGTTCTTTGGCCCCACAGCGGCCACAT CTGCCGTTGGTGCCAATAGGTTTTCCAGGAGCTGGTTGAGGTGGGAGG GAGGGAGAGGGTTGTGATCAGGCTGAGGCATGGGGATTGGATATAGTC TCCGTGTCATGATTTATTTGGTCAGTCAGTCCTAGTGCCACCCTGGGGTA ATGGGGATGTGTTCTCGTCACCTTGGGCCTGGCTGACCAGCTTTATCTC TTGGCACAGAGGCACAGAGCTTTGGCCTGCTGGATCCCAAACTCTGCTA CCTGCTGGATGGAATCCTCTTCATCTATCGTGTCATTCTCACTGCCTTGT TCCTGAGA |
| 26 | | P2A | GCCACCAACTTCTCTCTTTTGAAGCAGGCCGGAGATGTGGAAGAAAATC CTGGGCCC |
| 27 | | Membrane Export Signal (Leader) | ATGGCTCTTCCTGTGACTGCCCTTCTGCTGCCCCTGGCTCTCCTGCTCC ATGCTGCCCGGCCA |
| 28 | | FMC63VL (Light Chain, Variable Part) | GACATCCAGATGACACAGACAACCAGCAGCCTCAGCGCCAGCCTGGGG GACAGAGTGACCATTAGCTGCCGGGCCTCTCAGGACATCAGCAAATACC TGAACTGGTACCAGCAGAAACCAGATGGCACTGTCAAGCTGCTGATTTA CCACACCTCCAGGCTCCACAGCGGCGTGCCCAGTCGCTTCAGCGGCAG TGGGAGCGGGACAGATTATTCCCTCACAATCTCCAACCTGGAGCAGGAA GATATTGCCACATACTTCTGCCAGCAAGGCAACACCCTGCCATACACATT TGGAGGCGGCACCAAATTGGAGATCACC |
| 29 | | (G4S)3 Linker | GGCGGTGGTGGATCTGGAGGAGGGGGCAGCGGAGGTGGCGGCTCT |
| 30 | | FMC63VH (Heavy Chain, Variable Part) | GAGGTGAAACTGCAGGAGAGTGGCCCTGGCCTGGTGGCTCCCAGCCAG AGCCTTTCTGTCACCTGCACCGTGTCTGGGGTGTCCCTGCCTGACTATG GAGTCTCCTGGATCCGGCAGCCTCCAAGAAAAGGACTGGAATGGCTGG GCGTCATCTGGGGAAGTGAGACCACCTACTATAATTCAGCCCTCAAGTC CCGGCTCACCATCATTAAGGACAACTCCAAATCCCAGGTGTTCCTGAAG ATGAATTCTCTCCAGACTGATGACACAGCCATCTACTACTGTGCCAAGCA TTATTATTACGGCGGGTCCTATGCCATGGACTACTGGGGCCAGGGGACC AGTGTCACTGTTTCTTCT |
| 31 | | IgG4 Hinge | GAAAGCAAGTATGGCCCCCCATGCCCTCCCTGCCCC |
| 32 | | IgG1 CH3 Domain (Hinge) | GGACAGCCGCGGGAGCCCCAGGTCTACACCCTCCCACCCTCCAGAGAT GAGCTGACCAAGAACCAGGTTTCACTGACATGCCTGGTGAAGGGCTTCT ACCCCTCTGACATTGCTGTGGAGTGGGAGAGCAATGGGCAGCCAGAGA ACAACTACAAGACCACACCTCCTGTCCTGGACAGCGACGGCTCCTTCTT CCTCTACAGTAAGCTCACTGTGGACAAGAGCCGCTGGCAGCAGGGCAA TGTCTTCTCCTGCAGCGTGATGCACGAGGCCCTGCACAATGCCTATACC CAGAAGTCACTCTCTCTGAGCCCTGGAAAG |
| 33 | Arm | Linker | AAGGATCCCAAG |
| 34 | | CD28 Transmembra ne Domain | TTTTGGGTCCTCGTGGTGGTCGGGGGTGTGCTGGCCTGCTACAGCTTG CTGGTCACAGTGGCCTTCATCATCTTCTGGGTG |
| 35 | | CD28 Intracellular Domain | CGCTCCAAGAGGAGCCGGCTGCTTCACAGTGATTACATGAACATGACCC CCAGGAGGCCAGGACCCACCAGGAAGCACTACCAGCCCTACGCTCCCC CGCGGGACTTTGCTGCTTACCGCAGC |
| 36 | | Right Homology Arm | AGAGTGAAGGTGGGTACCACTGGGCTTTGGGAGGAGGGCACGGGGTC CCCCACTTGATGGATGTTCAGAGGGGCCTTGGTCTTGGAAGGTCTCAAG CTCGGGTGGTGCCTGGGGCTTGGTATCCAGGAGCAAAGCAAGGACCAG CCAAGTGTGTGCCTTGAGTGGGCTGAGGAGGAGGTGGCAGTGTCTGGC TGAGATGGACAGGGTAGGAGGGAGAGCCTGGTGCTAGGCACCTCCATG ACAAGCCGTACAAATGTGTGCACATCAGAGTGTCCCAGGGAAGGCGATG CTACTGGTGACAAAGGGGCTTACACTCAGGCAGAGGTCCTTCTTTCCAA GTGTGAATGAAGGCCATGTTAGCCTTTCTCTTGAAAAGGCCCTTTCCTCA TCTGTAACTGGGGA |
| 37 | CD247-exon2_CD19CA R(full) | Full Sequence | GGCAGGAGCTCTGTGCACAGAGAACAGCCTCATCTGCTCGCCTTGTTTC CACCTCCCCTCCCATTGCCCCAGGTTCTTTGGCCCCACAGCGGCCACAT CTGCCGTTGGTGCCAATAGGTTTTCCAGGAGCTGGTTGAGGTGGGAGG GAGGGAGAGGGTTGTGATCAGGCTGAGGCATGGGGATTGGATATAGTC TCCGTGTCATGATTTATTTGGTCAGTCAGTCCTAGTGCCACCCTGGGGTA ATGGGGATGTGTTCTCGTCACCTTGGGCCTGGCTGACCAGCTTTATCTC TTGGCACAGAGGCACAGAGCTTTGGCCTGCTGGATCCCAAACTCTGCTA CCTGCTGGATGGAATCCTCTTCATCTATCGTGTCATTCTCACTGCCTTGT TCCTGAGAGCCACCAACTTCTCTCTTTTGAAGCAGGCCGGAGATGTGGA AGAAAATCCTGGGCCCATGGCTCTTCCTGTGACTGCCCTTCTGCTGCCC CTGGCTCTCCTGCTCCATGCTGCCCGGCCAGACATCCAGATGACACAGA CAACCAGCAGCCTCAGCGCCAGCCTGGGGGACAGAGTGACCATTAGCT GCCGGGCCTCTCAGGACATCAGCAAATACCTGAACTGGTACCAGCAGAA ACCAGATGGCACTGTCAAGCTGCTGATTTACCACACCTCCAGGCTCCAC AGCGGCGTGCCCAGTCGCTTCAGCGGCAGTGGGAGCGGGACAGATTAT TCCCTCACAATCTCCAACCTGGAGCAGGAAGATATTGCCACATACTTCTG CCAGCAAGGCAACACCCTGCCATACACATTTGGAGGCGGCACCAAATTG GAGATCACCGGCGGTGGTGGATCTGGAGGAGGGGGCAGCGGAGGTGG CGGCTCTGAGGTGAAACTGCAGGAGAGTGGCCCTGGCCTGGTGGCTCC CAGCCAGAGCCTTTCTGTCACCTGCACCGTGTCTGGGGTGTCCCTGCCT GACTATGGAGTCTCCTGGATCCGGCAGCCTCCAAGAAAAGGACTGGAAT GGCTGGGCGTCATCTGGGGAAGTGAGACCACCTACTATAATTCAGCCCT CAAGTCCCGGCTCACCATCATTAAGGACAACTCCAAATCCCAGGTGTTC CTGAAGATGAATTCTCTCCAGACTGATGACACAGCCATCTACTACTGTGC CAAGCATTATTATTACGGCGGGTCCTATGCCATGGACTACTGGGGCCAG GGGACCAGTGTCACTGTTTCTTCTGAAAGCAAGTATGGCCCCCCATGCC CTCCCTGCCCCGGACAGCCGCGGGAGCCCCAGGTCTACACCCTCCCAC CCTCCAGAGATGAGCTGACCAAGAACCAGGTTTCACTGACATGCCTGGT GAAGGGCTTCTACCCCTCTGACATTGCTGTGGAGTGGGAGAGCAATGG GCAGCCAGAGAACAACTACAAGACCACACCTCCTGTCCTGGACAGCGAC GGCTCCTTCTTCCTCTACAGTAAGCTCACTGTGGACAAGAGCCGCTGGC AGCAGGGCAATGTCTTCTCCTGCAGCGTGATGCACGAGGCCCTGCACA ATGCCTATACCCAGAAGTCACTCTCTCTGAGCCCTGGAAAGAAGGATCC CAAGTTTTGGGTCCTCGTGGTGGTCGGGGGTGTGCTGGCCTGCTACAG CTTGCTGGTCACAGTGGCCTTCATCATCTTCTGGGTGCGCTCCAAGAGG AGCCGGCTGCTTCACAGTGATTACATGAACATGACCCCCAGGAGGCCAG GACCCACCAGGAAGCACTACCAGCCCTACGCTCCCCCGCGGGACTTTG CTGCTTACCGCAGCAGGGTCAAATTTTCTAGATCTGCAGATGCGCCGGC CTATCAACAAGGCCAGAACCAGCTCTATAACGAGCTCAATCTAGGACGA AGAGAGGAGTACGATGTTTTGGACAAGAGACGTGGCCGGGACCCTGAG ATGGGGGGAAAGCCGAGAAGGAAGAACCCTCAGGAAGGCCTGTACAAT GAACTGCAGAAAGATAAGATGGCGGAGGCCTACAGTGAGATTGGGATG AAAGGCGAGCGCCGGAGGGGCAAGGGGCACGATGGCCTTTACCAGGG TCTCAGTACAGCCACCAAGGACACCTACGACGCCCTTCACATGCAGGCC CTGCCCCCTCGCTAATAAGAATTCTAACTAGAGCTCGCTGATCAGCCTC GACTGTGCCTTCTAGTTGCCAGCCATCTGTTGTTTGCCCCTCCCCCGTG CCTTCCTTGACCCTGGAAGGTGCCACTCCCACTGTCCTTTCCTAATAAAA TGAGGAAATTGCATCGCATTGTCTGAGTAGGTGTCATTCTATTCTGGGG GGTGGGGTGGGGCAGGACAGCAAGGGGGAGGATTGGGAAGAGAATAG CAGGCATGCTGGGGAGCGGCCGCAGAGTGAAGGTGGGTACCACTGGG CTTTGGGAGGAGGGCACGGGGTCCCCCACTTGATGGATGTTCAGAGGG GCCTTGGTCTTGGAAGGTCTCAAGCTCGGGTGGTGCCTGGGGCTTGGT |
| | | | ATCCAGGAGCAAAGCAAGGACCAGCCAAGTGTGTGCCTTGAGTGGGCT GAGGAGGAGGTGGCAGTGTCTGGCTGAGATGGACAGGGTAGGAGGGA GAGCCTGGTGCTAGGCACCTCCATGACAAGCCGTACAAATGTGTGCACA TCAGAGTGTCCCAGGGAAGGCGATGCTACTGGTGACAAAGGGGCTTAC ACTCAGGCAGAGGTCCTTCTTTCCAAGTGTGAATGAAGGCCATGTTAGC CTTTCTCTTGAAAAGGCCCTTTCCTCATCTGTAACTGGGGA |
| 38 | CD247-exon3_CD19CA R(truncated) | Full Sequence | ATTGTCCTCAGAGTCCCCGGAGGAAGGGATCCTAGAGAATACTCATGTC CCCGGGGGCGCAGACCAAGAACCTTATACCTCTGCGCACGCGCGAGGG CGCTAGCCCGGGAAGAATAAACTCCAGCGGGTCCTGGGCCAGCGTGAT GAATGCACGTGTCACAGGCGGGAGGAAAAGGACAACTGGGCTCAGAA AAAGGGGCTGCCACGCCCCGGCCCCTGCAAGGGCTCAGCCCCAGGAG GCAGGGCAGCATTGTTAGTTGCCAAGGAGCGGAGTAGGGCTGTCCCTC GGGTCGCCGCGCGCCCTGGCACCCCGGCTCCCCTGCGCGCCCTCGCG GAGCAGTAACGCGCTTTGCTTTCTGTGTTGCAGTTCAGCAGGAGCGCAG ACGCCCCCGCGTACGCCACCAACTTCTCTCTTTTGAAGCAGGCCGGAGA TGTGGAAGAAAATCCTGGGCCCATGGCTCTTCCTGTGACTGCCCTTCTG CTGCCCCTGGCTCTCCTGCTCCATGCTGCCCGGCCAGACATCCAGATGA CACAGACAACCAGCAGCCTCAGCGCCAGCCTGGGGGACAGAGTGACCA TTAGCTGCCGGGCCTCTCAGGACATCAGCAAATACCTGAACTGGTACCA GCAGAAACCAGATGGCACTGTCAAGCTGCTGATTTACCACACCTCCAGG CTCCACAGCGGCGTGCCCAGTCGCTTCAGCGGCAGTGGGAGCGGGAC AGATTATTCCCTCACAATCTCCAACCTGGAGCAGGAAGATATTGCCACAT ACTTCTGCCAGCAAGGCAACACCCTGCCATACACATTTGGAGGCGGCAC CAAATTGGAGATCACCGGCGGTGGTGGATCTGGAGGAGGGGGCAGCG GAGGTGGCGGCTCTGAGGTGAAACTGCAGGAGAGTGGCCCTGGCCTG GTGGCTCCCAGCCAGAGCCTTTCTGTCACCTGCACCGTGTCTGGGGTGT CCCTGCCTGACTATGGAGTCTCCTGGATCCGGCAGCCTCCAAGAAAAGG ACTGGAATGGCTGGGCGTCATCTGGGGAAGTGAGACCACCTACTATAAT TCAGCCCTCAAGTCCCGGCTCACCATCATTAAGGACAACTCCAAATCCC AGGTGTTCCTGAAGATGAATTCTCTCCAGACTGATGACACAGCCATCTAC TACTGTGCCAAGCATTATTATTACGGCGGGTCCTATGCCATGGACTACTG GGGCCAGGGGACCAGTGTCACTGTTTCTTCTGAAAGCAAGTATGGCCCC CCATGCCCTCCCTGCCCCGGACAGCCGCGGGAGCCCCAGGTCTACACC CTCCCACCCTCCAGAGATGAGCTGACCAAGAACCAGGTTTCACTGACAT GCCTGGTGAAGGGCTTCTACCCCTCTGACATTGCTGTGGAGTGGGAGA GCAATGGGCAGCCAGAGAACAACTACAAGACCACACCTCCTGTCCTGGA CAGCGACGGCTCCTTCTTCCTCTACAGTAAGCTCACTGTGGACAAGAGC CGCTGGCAGCAGGGCAATGTCTTCTCCTGCAGCGTGATGCACGAGGCC CTGCACAATGCCTATACCCAGAAGTCACTCTCTCTGAGCCCTGGAAAGA AGGATCCCAAGTTTTGGGTCCTCGTGGTGGTCGGGGGTGTGCTGGCCT GCTACAGCTTGCTGGTCACAGTGGCCTTCATCATCTTCTGGGTGCGCTC CAAGAGGAGCCGGCTGCTTCACAGTGATTACATGAACATGACCCCCCAGG AGGCCAGGACCCACCAGGAAGCACTACCAGCCCTACGCTCCCCCGCGG GACTTTGCTGCTTACCGCAGCAGGGTCAAATTTTCTAGATCTGCAGATGC GCCGGCCTATCAACAAGGCCAGAACCAGCTCTATAACGTAAGTCAGCCT CGCCGTAGACCCTCCGGAAAGGAAGGGCAGCCTCCTCCTCGGACCTAG GGAACGCGCCCGCCAGAGTCTTGGGTTTAGGTTTGGTCCCTAGCTATCA CCTCTGCGACCCGGCTGCAACTTGTTTAACCTCAGTTTTCTAACCCACAA AACAGGTGTAGTAATTGTACCTGTCTCAAAGGGCGCCATAAAGATTACAT GCTTTAATGTCTTGATGCATGGAACGAGCTTAGTCTAGTGCCTGCCACTA GTGAGCCCCCAAGCCATCAGCAATTTGCAAAGGCTAGAGGGGGGTGGA GAAGGAATGTATTAGAATCTTCCCAGAGGAGGGGAAAGCGATTTCACAC TCCCTGTGCAATGGGACTG |
| 39 | | Left Homology Arm | ATTGTCCTCAGAGTCCCCGGAGGAAGGGATCCTAGAGAATACTCATGTC CCCGGGGGCGCAGACCAAGAACCTTATACCTCTGCGCACGCGCGAGGG CGCTAGCCCGGGAAGAATAAACTCCAGCGGGTCCTGGGCCAGCGTGAT GAATGCACGTGTCACAGGCGGGAGGAAAAAGGACAACTGGGCTCAGAA AAAGGGGCTGCCACGCCCCGGCCCCTGCAAGGGCTCAGCCCCAGGAG GCAGGGCAGCATTGTTAGTTGCCAAGGAGCGGAGTAGGGCTGTCCCTC GGGTCGCCGCGCGCCCTGGCACCCCGGCTCCCCTGCGCGCCCTCGCG GAGCAGTAACGCGCTTTGCTTTCTGTGTTGCAGTTCAGCAGGAGCGCAG ACGCCCCCGCGTAC |
| 40 | | CAR body | SEQ ID NO 26-35 |
| 41 | | truncated CD3-zeta | AGGGTCAAATTTTCTAGATCTGCA |
| 42 | | Right Homology Arm with | GATGCGCCGGCCTATCAACAAGGCCAGAACCAGCTCTATAACGTAAGTC AGCCTCGCCGTAGACCCTCCGGAAAGGAAGGGCAGCCTCCTCCTCGGA CCTAGGGAACGCGCCCGCCAGAGTCTTGGGTTTAGGTTTGGTCCCTAG |
| | | Silent Mutations to Prevent Cas9 Re-Cutting | CTATCACCTCTGCGACCCGGCTGCAACTTGTTTAACCTCAGTTTTCTAAC CCACAAAACAGGTGTAGTAATTGTACCTGTCTCAAAGGGCGCCATAAAG ATTACATGCTTTAATGTCTTGATGCATGGAACGAGCTTAGTCTAGTGCCT GCCACTAGTGAGCCCCCAAGCCATCAGCAATTTGCAAAGGCTAGAGGG GGGTGGAGAAGGAATGTATTAGAATCTTCCCAGAGGAGGGGAAAGCGA TTTCACACTCCCTGTGCAATGGGACTG |
| 43 | CD247-exon3_ | Right Homology Arm | CAAGGCCAGAACCAGCTCTATAACGTAAGTCAGCCTCGCCGTAGACCCT CCGGAAAGGAAGGGCAGCCTCCTCCTCGGACCTAGGGAACGCGCCCG CCAGAGTCTTGGGTTTAGGTTTGGTCCCTAGCTATCACCTCTGCGACCC GGCTGCAACTTGTTTAACCTCAGTTTTCTAACCCACAAAACAGGTGTAGT AATTGTACCTGTCTCAAAGGGCGCCATAAAGATTACATGCTTTAATGTCT TGATGCATGGAACGAGCTTAGTCTAGTGCCTGCCACTAGTGAGCCCCCA AGCCATCAGCAATTTGCAAAGGCTAGAGGGGGGTGGAGAAGGAATGTAT TAGAATCTTCCCAGAGGAGGGGAAAGCGATTTCACACTCCCTGTGCAAT GGGACTG |
| 44 | CD3-zeta Intracellular Domain + STOP | | AGGGTCAAATTTTCTAGATCTGCAGATGCGCCGGCCTATCAACAAGGCC AGAACCAGCTCTATAACGAGCTCAATCTAGGACGAAGAGAGGAGTACGA TGTTTTGGACAAGAGACGTGGCCGGGACCCTGAGATGGGGGGAAAGCC GAGAAGGAAGAACCCTCAGGAAGGCCTGTACAATGAACTGCAGAAAGAT AAGATGGCGGAGGCCTACAGTGAGATTGGGATGAAAGGCGAGCGCCGG AGGGGCAAGGGGCACGATGGCCTTTACCAGGGTCTCAGTACAGCCACC AAGGACACCTACGACGCCCTTCACATGCAGGCCCTGCCCCCTCGCTAA |
| 45 | bGH Poly-A Signal (Extended) | | TAAGAATTCTAACTAGAGCTCGCTGATCAGCCTCGACTGTGCCTTCTAGT TGCCAGCCATCTGTTGTTTGCCCCTCCCCCGTGCCTTCCTTGACCCTGG AAGGTGCCACTCCCACTGTCCTTTCCTAATAAAATGAGGAAATTGCATCG CATTGTCTGAGTAGGTGTCATTCTATTCTGGGGGGTGGGGTGGGGCAG GACAGCAAGGGGGAGGATTGGGAAGAGAATAGCAGGCATGCTGGGGA GCGGCCGC |
| 46 | eGFP | | ATGGTATCCAAGGGTGAAGAATTGTTCACTGGCGTAGTTCCTATCTTGGT GGAGCTCGACGGAGATGTCAATGGACACAAGTTTTCAGTCAGTGGCGAA GGTGAGGGTGATGCAACTTACGGGAAGCTTACACTGAAATTCATTTGTA CTACTGGCAAGTTGCCGGTGCCATGGCCCACGCTGGTAACGACCCTTAC CTACGGTGTCCAATGTTTTTCTCGATACCCCGATCATATGAAACAGCATG ACTTTTTTAAATCTGCTATGCCCGAGGGATACGTACAGGAAAGGACTATA TTTTTTAAGGACGATGGTAACTATAAGACCCGCGCCGAAGTCAAGTTTGA GGGAGATACTCTTGTTAATCGAATCGAGCTCAAGGGGATAGATTTTAAG GAGGATGGCAACATTCTCGGCCACAAGCTGGAGTACAATTACAATAGTC ACAATGTTTACATAATGGCTGATAAGCAAAAAAACGGCATCAAAGTTAAT TTTAAAATTAGGCATAATATAGAAGACGGTTCAGTTCAACTCGCCGACCA CTACCAGCAGAATACCCCTATTGGCGATGGGCCGGTTCTCCTGCCTGAC AACCATTACCTCTCAACACAGTCTGCTTTGAGTAAAGACCCTAACGAGAA GCGAGATCATATGGTCTTGCTCGAGTTCGTAACGGCCGCCGGTATAACC CTGGGCATGGATGAGCTCTACAAATAA |
| 47 | **TRAC-Charité** | Left Homology Arm | CCTATTAAATAAAAGAATAAGCAGTATTATTAAGTAGCCCTGCATTTCAGG TTTCCTTGAGTGGCAGGCCAGGCCTGGCCGTGAACGTTCACTGAAATCA TGGCCTCTTGGCCAAGATTGATAGCTTGTGCCTGTCCCTGAGTCCCAGT CCATCACGAGCAGCTGGTTTCTAAGATGCTATTTCCCGTATAAAGCATGA GACCGTGACTTGCCAGCCCCACAGAGCCCCGCCCTTGTCCATCACTGG CATCTGGACTCCAGCCTGGGTTGGGGCAAAGAGGGAAATGAGATCATGT CCTAACCCTGATCCTCTTGTCCCACAGATATCCAGAACCCTGACCCTGC CGTGTACCAGCTGAGAGACTCTAAATCCAGTGACAAGTCTGTCTGCCTA TTCACC |
| 48 | | Right Homology Arm | ATTTTGATTCTCAAACAAATGTGTCACAAAGTAAGGATTCTGATGTGTATA TCACAGACAAAACTGTGCTAGACATGAGGTCTATGGACTTCAAGAGCAA CAGTGCTGTGGCCTGGAGCAACAAATCTGACTTTGCATGTGCAAACGCC TTCAACAACAGCATTATTCCAGAAGACACCTTCTTCCCCAGCCCAGGTAA GGGCAGCTTTGGTGCCTTCGCAGGCTGTTTCCTTGCTTCAGGAATGGCC AGGTTCTGCCCAGAGCTCTGGTCAATGATGTCTAAAACTCCTCTGATTGG TGGTCTCGGCCTTATCCATTGCCACCAAAACCCTCTTTTTACTAAGAAAC AGTGAGCCTTGTTCTGGCAGTCCAGAGAATGACACGGGAAAAAAGCAGA TG |
| 49 | **AAVS1_EF1a-LTR** | Left Homology Arm | ACCGTTTTTCTGGACAACCCCAAAGTACCCCGTCTCCCTGGCTTTAGCC ACCTCTCCATCCTCTTGCTTTCTTTGCCTGGACACCCCGTTCTCCTGTGG ATTCGGGTCACCTCTCACTCCTTTCATTTGGGCAGCTCCCCTACCCCCCT TACCTCTCTAGTCTGTGCTAGCTCTTCCAGCCCCCTGTCATGGCATCTTC |
| | | | CAGGGGTCCGAGAGCTCAGCTAGTCTTCTTCCTCCAACCCGGGCCCCTA TGTCCACTTCAGGACAGCATGTTTGCTGCCTCCAGGGATCCTGTGTCCC CGAGCTGGGACCACCTTATATTCCCAGGGCCGGTTAATGTGGCTCTGGT TCTGGGTACTTTTATCTGTCCCCTCCACCCCACAGTGGGGCCACTAGGG ACAG |
| 50 | | EF1 a-LTR Promotor | GGCAGGAGCTCTGTGCACAGAGAACAGCCTCATCTGCTCGCCTTGTTTC CACCTCCCCTCCCATTGCCCCAGGTTCTTTGGCCCCACAGCGGCCACAT CTGCCGTTGGTGCCAATAGGTTTTCCAGGAGCTGGTTGAGGTGGGAGG GAGGGAGAGGGTTGTGATCAGGCTGAGGCATGGGGATTGGATATAGTC TCCGTGTCATGATTTATTTGGTCAGTCAGTCCTAGTGCCACCCTGGGGTA ATGGGGATGTGTTCTCGTCACCTTGGGCCTGGCTGACCAGCTTTATCTC TTGGCACAGAGGCACAGAGCTTTGGCCTGCTGGATCCCAAACTCTGCTA CCTGCTGGATGGAATCCTCTTCATCTATCGTGTCATTCTCACTGCCTTGT TCCTGAGAGCCACCAACTTCTCTCTTTTGAAGCAGGCCGGAGATGTGGA AGAAAATCCTGGGCCCATGGCTCTTCCTGTGACTGCCCTTCTGCTGCCC CTGGCTCTCCTGCTCCATGCTGCCCGGCCAGACATCCAGATGACACAGA CAACCAGCAGCCTCAGCGCCAGCCTGGGGGACAGAGTGACCATTAGCT GCCGGGCCTCTCAGGACATCAGCAAATACCTGAACTGGTACCAGCAGAA ACCAGATGGCACTGTCAAGCTGCTGATTTACCACACCTCCAGGCTCCAC AGCGGCGTGCCCAGTCGCTTCAGCGGCAGTGGGAGCGGGACAGATTAT TCCCTCACAATCTCCAACCTGGAGCAGGAAGATATTGCCACATACTTCTG CCAGCAAGGCAACACCCTGCCATACACATTTGGAGGCGGCACCAAATTG GAGATCACCGGCGGTGGTGGATCTGGAGGAGGGGGCAGCGGAGGTGG CGGCTCTGAGGTGAAACTGCAGGAGAGTGGCCCTGGCCTGGTGGCTCC CAGCCAGAGCCTTTCTGTCACCTGCACCGTGTCTGGGGTGTCCCTGCCT GACTATGGAGTCTCCTGGATCCGGCAGCCTCCAAGAAAAGGACTGGAAT GGCTGGGCGTCATCTGGGGAAGTGAGACCACCTACTATAATTCAGCCCT CAAGTCCCGGCTCACCATCATTAAGGACAACTCCAAATCCCAGGTGTTC CTGAAGATGAATTCTCTCCAGACTGATGACACAGCCATCTACTACTGTGC CAAGCATTATTATTACGGCGGGTCCTATGCCATGGACTACTGGGGCCAG GGGACCAGTGTCACTGTTTCTTCTGAAAGCAAGTATGGCCCCCCATGCC CTCCCTGCCCCGGACAGCCGCGGGAGCCCCAGGTCTACACCCTCCCAC CCTCCAGAGATGAGCTGACCAAGAACCAGGTTTCACTGACATGCCTGGT GAAGGGCTTCTACCCCTCTGACATTGCTGTGGAGTGGGAGAGCAATGG GCAGCCAGAGAACAACTACAAGACCACACCTCCTGTCCTGGACAGCGAC GGCTCCTTCTTCCTCTACAGTAAGCTCACTGTGGACAAGAGCCGCTGGC AGCAGGGCAATGTCTTCTCCTGCAGCGTGATGCACGAGGCCCTGCACA ATGCCTATACCCAGAAGTCACTCTCTCTGAGCCCTGGAAAGAAGGATCC CAAGTTTTGGGTCCTCGTGGTGGTCGGGGGTGTGCTGGCCTGCTACAG CTTGCTGGTCACAGTGGCCTTCATCATCTTCTGGGTGCGCTCCAAGAGG AGCCGGCTGCTTCACAGTGATTACATGAACATGACCCCCAGGAGGCCAG GACCCACCAGGAAGCACTACCAGCCCTACGCTCCCCCGCGGGACTTTG CTGCTTACCGCAGCAGAGTGAAGGTGGGTACCACTGGGCTTTGGGAGG AGGGCACGGGGTCCCCCACTTGATGGATGTTCAGAGGGGCCTTGGTCT TGGAAGGTCTCAAGCTCGGGTGGTGCCTGGGGCTTGGTATCCAGGAGC AAAGCAAGGACCAGCCAAGTGTGTGCCTTGAGTGGGCTGAGGAGGAGG TGGCAGTGTCTGGCTGAGATGGACAGGGTAGGAGGGAGAGCCTGGTGC TAGGCACCTCCATGACAAGCCGTACAAATGTGTGCACATCAGAGTGTCC CAGGGAAGGCGATGCTACTGGTGACAAAGGGGCTTACACTCAGGCAGA GGTCCTTCTTTCCAAGTGTGAATGAAGGCCATGTTAGCCTTTCTCTTGAA AAGGCCCTTTCCTCATCTGTAACTGGGGA |
| 51 | | Right Homology Arm | GATTGGTGACAGAAAAGCCCCATCCTTAGGCCTCCTCCTTCCTAGTCTC CTGATATTGGGTCTAACCCCCACCTCCTGTTAGGCAGATTCCTTATCTGG TGACACACCCCCATTTCCTGGAGCCATCTCTCTCCTTGCCAGAACCTCTA AGGTTTGCTTACGATGGAGCCAGAGAGGATCCTGGGAGGGAGAGCTTG GCAGGGGGTGGGAGGGAAGGGGGGGATGCGTGACCTGCCCGGTTCTC AGTGGCCACCCTGCGCTACCCTCTCCCAGAACCTGAGCTGCTCTGACG CGGCCGTCTGGTGCGTTTCACTGATCCTGGTGCTGCAGCTTCCTTACAC TTCCCAAGAGGAGAAGCAGTTTGGAAAAACAAAATCAGAATAAGTTGGTC CTGAGTTCT |

Other sequences as used in specific embodiments and presented in the Examples share the nucleic acid and amino acid sequences as presented below. A full Sequence for a particular construct can be assembled according to the Sequence IDs given.

| | Preferred amino acid sequences | Preferred nucleic acid sequences |
|---|---|---|
| **CD247-exon2_CD19CAR(full)** | SEQ ID NO 2-12, 21 | SEQ ID NO 25-35, 44, 45, 36 |
| **CD247-exon2_CD19CAR(short/truncated)** | SEQ ID NO 1 (complete) or: SEQ ID NO 2-13 (individual components) | SEQ ID NO 24 (complete) or SEQ ID NO 25-36 (individual components) |
| CD247-exon2_eGFP | SEQ ID NO 2, 3, 22 | SEQ ID NO 25, 26, 46, 45, 36 |
| **CD247-exon3_CD19CAR(full)** | SEQ ID NO 16, 3-12, 21 | SEQ ID NO 39, 26-35, 44, 45, 43 |
| **CD247-exon3_CD19CAR(short/truncated)** | SEQ ID NO 15 (complete) or: SEQ ID NO 16, 3-12, 18-19 (inidivudal components) | SEQ ID NO 38 (complete) or: SEQ ID NO 39, 26-35, 41, 42 (individual components) |
| CD247-exon3_eGFP | SEQ ID NO 16, 3, 22 | SEQ ID NO 39, 26, 46, 45, 43 |
| **TRAC-Charité_CD19CAR(full)** | SEQ ID NO 23, 3-12, 21 | SEQ ID NO 47, 26-35, 44, 45, 48 |
| **TRAC-Charité_eGFP** | SEQ ID 23, 3, 22 | SEQ ID NO 47, 26, 46, 45, 48 |
| **AAVS1_EF1a-LTR_CD19CAR(full)** | SEQ ID NO 3-12, 21 | SEQ ID NO 49, 50, 27-35, 44, 45, 51 |

In specific embodiments, CAR nucleic acid sequence constructs of the prior art used in the Examples for comparison purposes, i.e. nucleic acid constructs encoding the TRAC-CD19CAR(full) or TRAC- eGFP polypeptide published by Roth et al. 2018 have been designed according to PMID: 29995861 and the respective SEQ IDs for the CD19CAR and eGFP listed above.

### FIGURES

The invention is demonstrated by way of the example through the figures disclosed herein. The figures provided represent particular, non-limiting embodiments and are not intended to limit the scope of the invention.

### Short description of the figures:

Figure 1: New strategy fuses zeta-deficient CAR transgenes to endogenous CD3 zeta on genetic level
Figure 2: Knock-out of TRAC or CD247 prevent formation of TCR-CD3 complex on the surface of primary human T cells
Figure 3: In-frame insertion of GFP into different T cell receptor complex genes leads to loss of CD3-TCR complex surface expression
Figure 4: GFP intensity is lower when expressed under control of the CD247 promotor compared to the TRAC promotor
Figure 5: In NK cells, transgenes can be expressed under control of CD247 but not TRAC
Figure 6: Insertion of complete and zeta-deficient CAR transgenes into CD247 gene induce CAR protein surface expression
Figure 7: Introducing shorter insert size tends to increase absolute CAR T cell generation after non-viral gene insertion
Figure 8: CAR expression level is regulated most strictly under control of the CD247 gene
Figure 9: Short CAR CD247 ki CAR T cell lyse CD19+ target cells in antigen-specific and dose-dependent fashion (as well as at a comparable rate to TRAC/CD247-integrated full CARs)
Figure 10: CD247 short CAR KI T cells perform comparable to full CAR knock-in in serial tumor challenge
Figure 11: Reduced cytokine production after antigen-engagement by truncated CARs integrated in the CD247 gene vs full CAR in the TRAC locus
Figure 12: CD247 KI abolishes allo-reactivity of CAR T comparable to TRAC KI
Figure 13: Integration of truncated CARs into CD247 gene can be used to generate CAR+ regulatory T cells

### Detailed description of the figures:

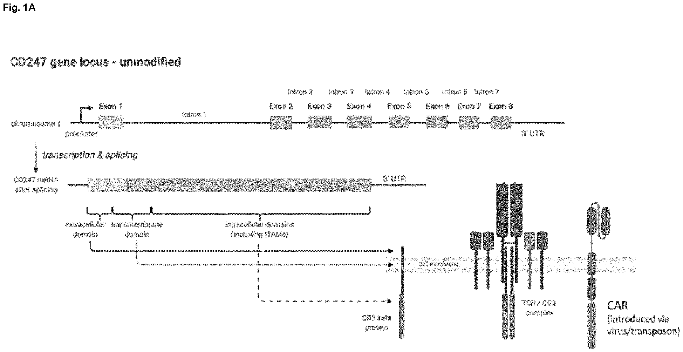

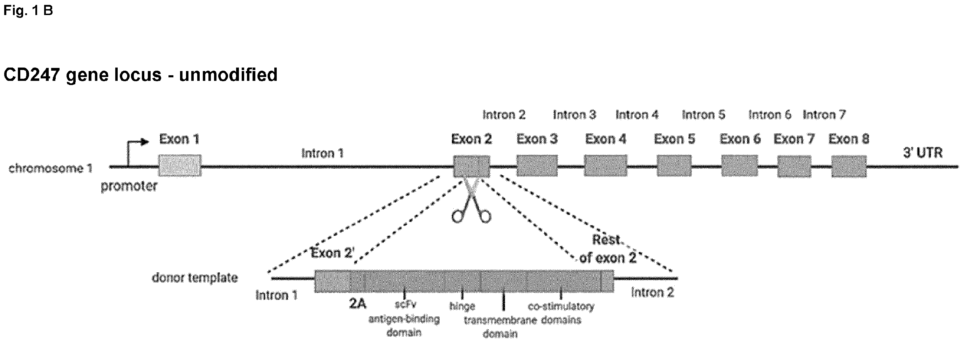

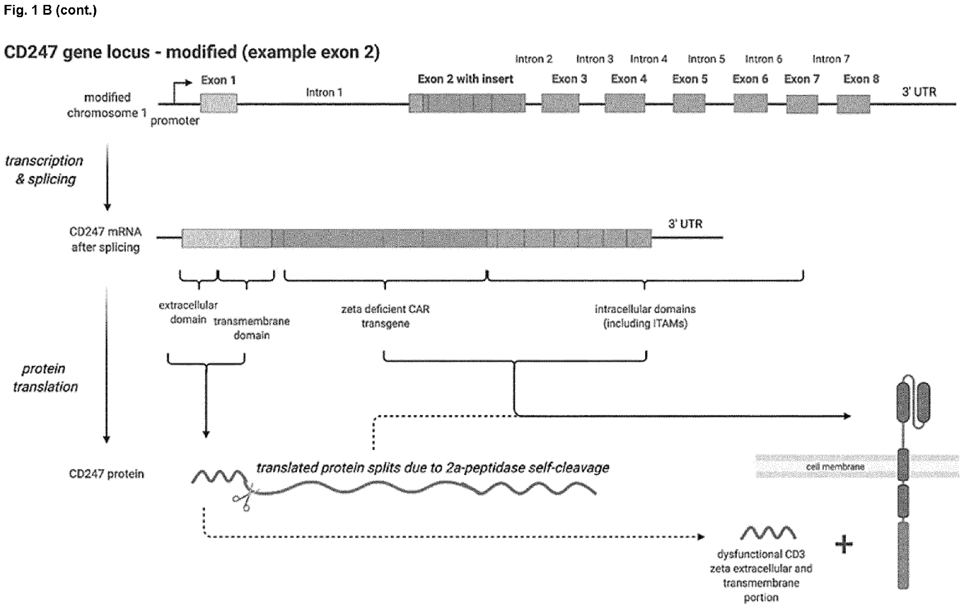

**Figure 1****: New strategy fuses zeta-deficient CAR transgenes to endogenous CD3 zeta on genetic level:** (A) CD3-zeta protein expression form the CD247 gene. In the process of RNA transcription, CD247 exons are spliced together to form a mature CD247 messenger RNA, which serves as a template for protein translation by the ribosome. The formed CD3-zeta protein assembles with other proteins to form a fully functioning TCR/CD3 complex. The intracellular domain of the CD3-zeta protein within the TCR/CD3 complex is identical to the CD3-zeta domain of conventional Chimeric Antigen Receptors. (B) The sketch also illustrates this invention. A truncated, CD3-zeta deficient CAR transgene (preferably but not exclusively encoding a self-cleaving 2A-peptide, an antigen binding domain (e.g. an scFv), a hinge/spacer domain, a transmembrane domain and one or more co-stimulatory or regulatory domains but no Immunoreceptor Tyrosine-based Activation Motif (ITAM) containing signaling domain) is inserted into the CD247 gene. The modified CD247 gene is transcribed into mRNA encoding a full CAR with an endogenous CD3-zeta domain. During protein translation, the N-terminus of the expressed fusion protein is cleaved off the CAR protein via self-cleavage of the 2A peptide. While a functional CAR Protein is expressed on the surface of edited immune cells, the cleaved N-terminus of the CD3-zeta gene is non-functional and cannot serve for TCR/CD3 complex formation. As a result, successfully edited immune cells can express a CAR protein but are unable to express a TCR.

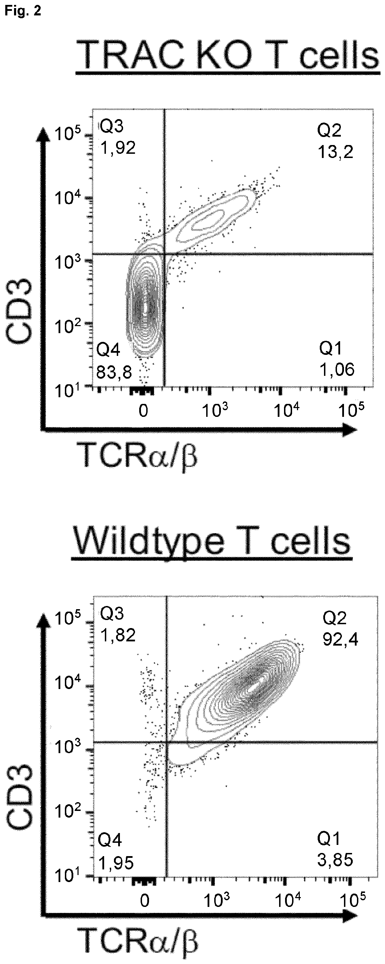

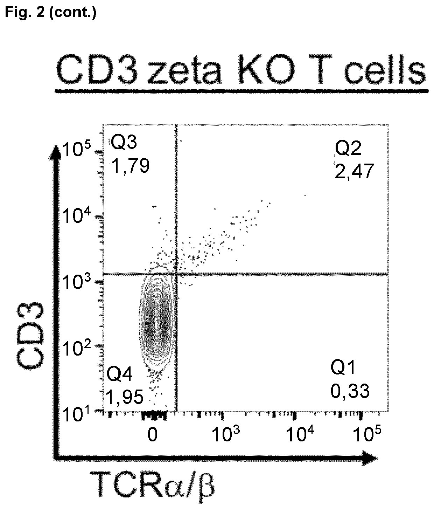

**Figure 2****: Knock-out of TRAC or CD247 prevent formation of TCR-CD3 complex on the surface of primary human T cells:** Healthy human T cells were isolated from venous blood and polyclonally stimulated on anti-CD3-/anti-CD28-antibody coated tissue culture plates in the presence of IL-7 and IL-15 (each at 10 ng/ml). Two days after activation, T cells were electroporated with sgRNAs targeting either the constant region of the T cell receptor alpha chain gene (TRAC, left panel) or the CD247 gene (CD3-zeta, right panel). After four days of subsequent expansion in cytokine containing medium, cells were analyzed for CD3 and TCRα/β expression via flow cytometry. [KI = knock-in; KO = knock-out]

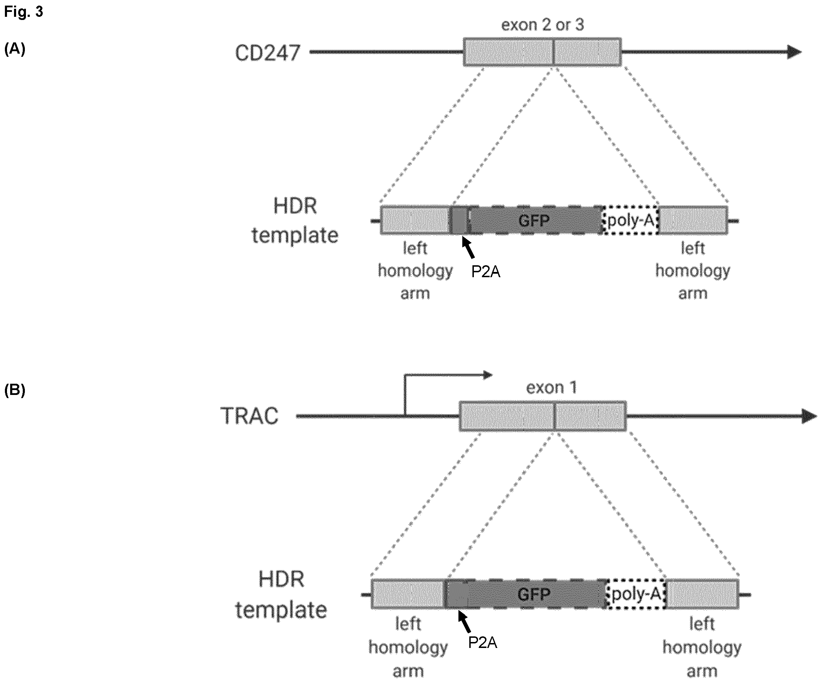

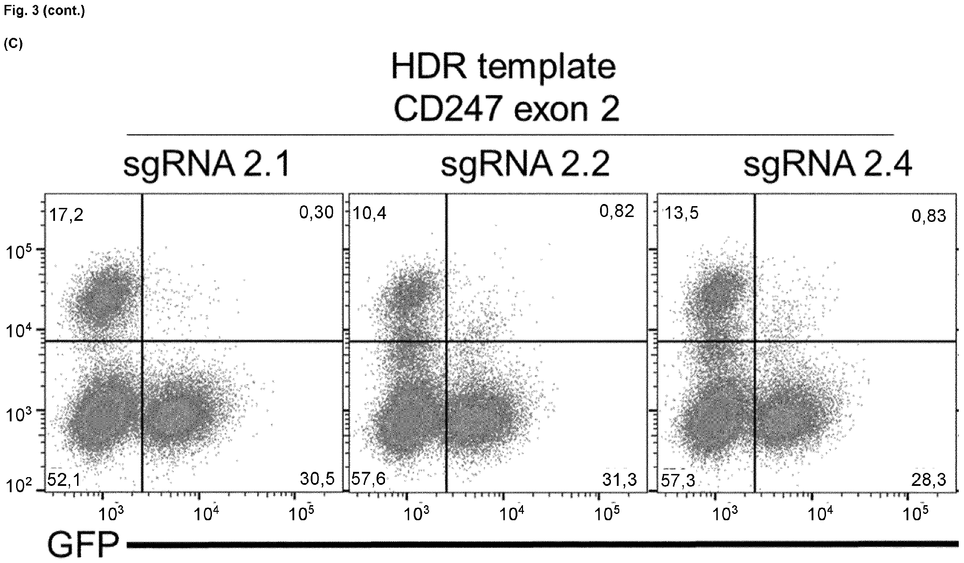

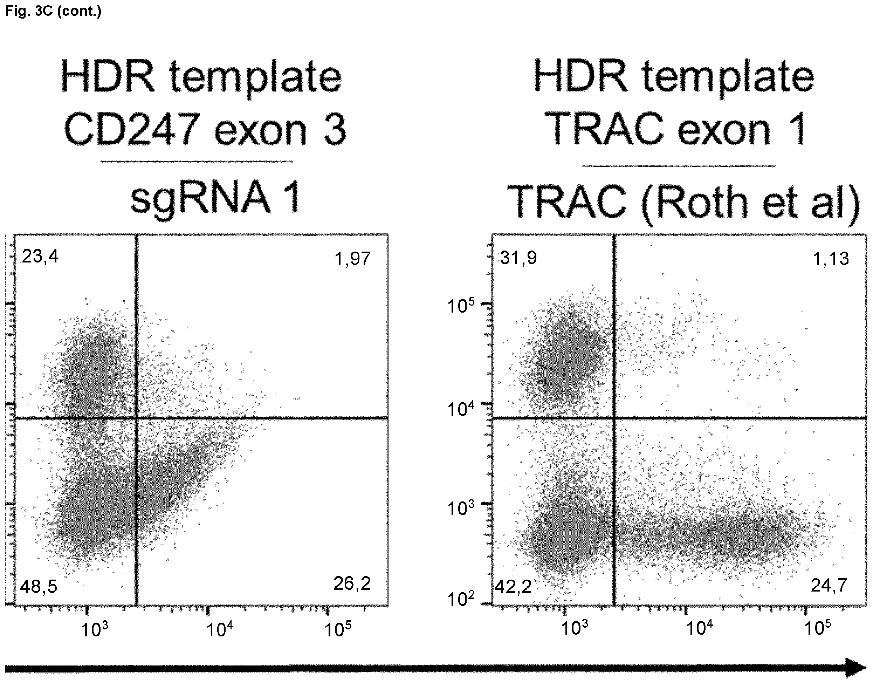

**Figure 3****: In-frame insertion of GFP into different T cell receptor complex genes leads to loss of CD3-TCR complex surface expression:** In this experiment, nuclease-assisted gene insertion of green fluorescent protein (GFP) reporter transgenes into the protein-coding reading frame of either the CD247 gene (encoding for CD3 zeta) or the TRAC gene (encoding for TCR alpha chain) was performed to investigate the effect on CD3-TCR surface expression. As indicated by flow cytometric protein analysis, most GFP expression was observed in the CD3-TCR negative population (Y-axis). Multiple Cas9 nucleases with different sgRNAs were used for CD247 exon 2 and 3 achieving similar results. Insertion of GFP into TRAC gene mirrored results seen in CD247, despite higher maximum GFP intensity.

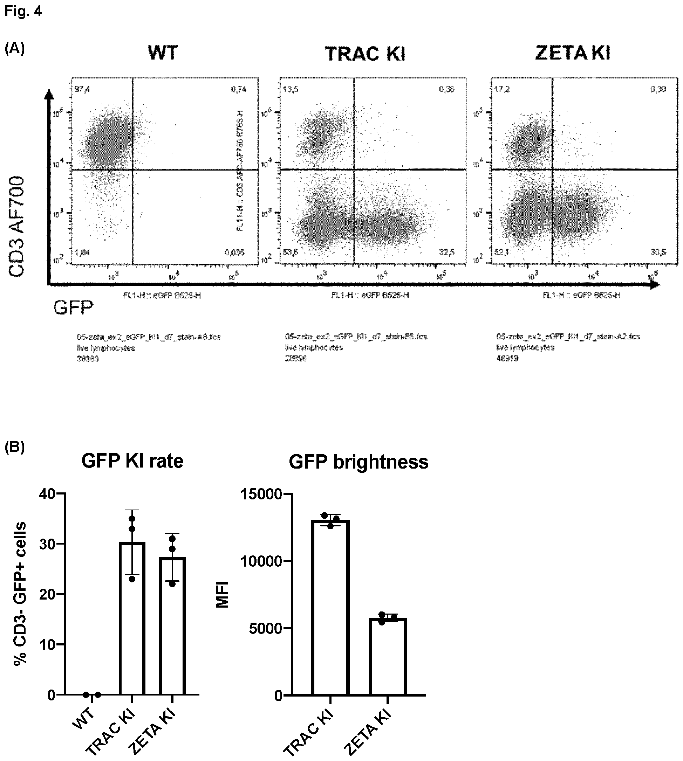

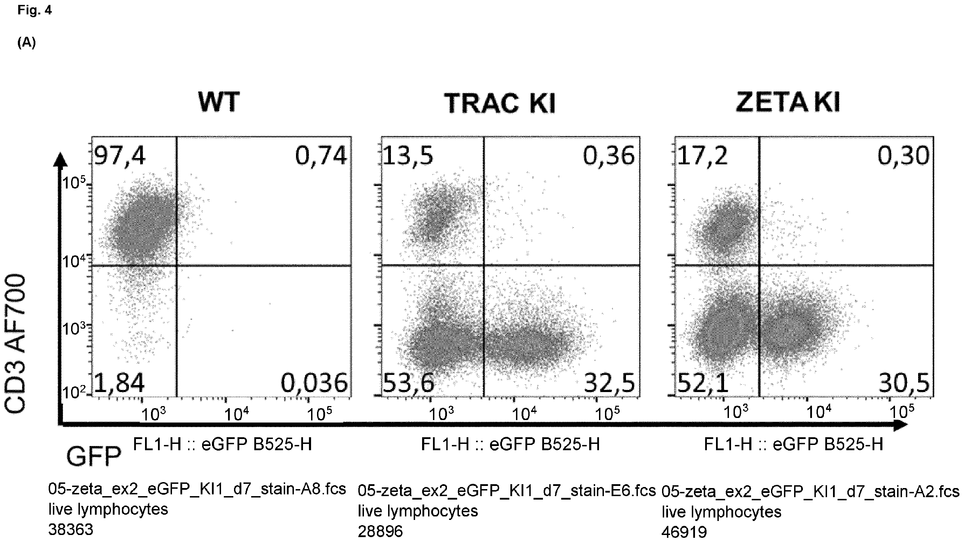

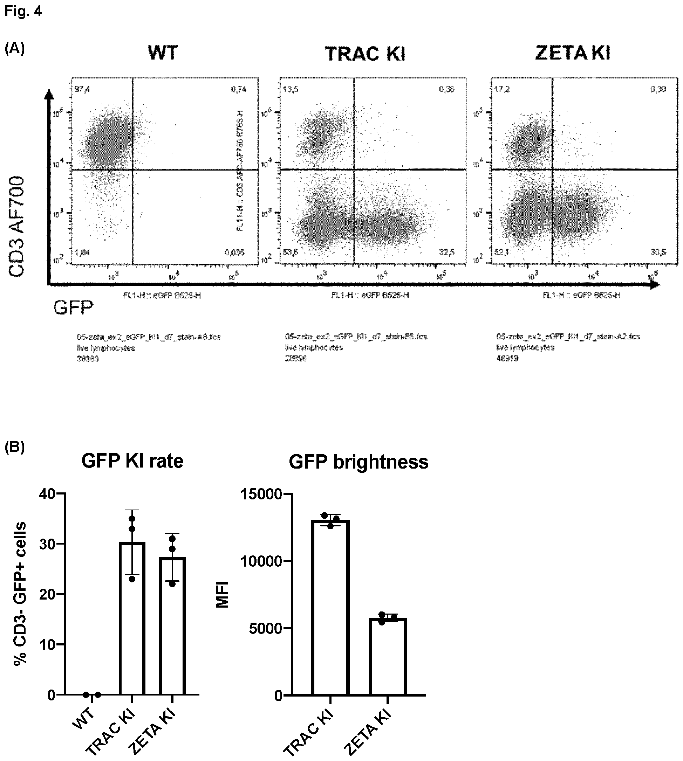

**Figure 4****: GFP intensity is lower when expressed under control of the CD247 promoter compared to the TRAC promoter:** This subanalysis from the previous experiment (Fig. 3) compares GFP knock-in (KI) rates and fluorescence intensity in three replicates summarized for the preferred conditions (CD247: HDR template exon 2 + sgRNA 2.1; TRAC: exon 1 Charité + TRAC sg1). While relative KI did not differ significantly between TRAC and CD247, GFP expressed under control of the CD247 promotor leads to a lower median fluorescence intensity (MFI). [KI = knock-in; KO = knock-out]

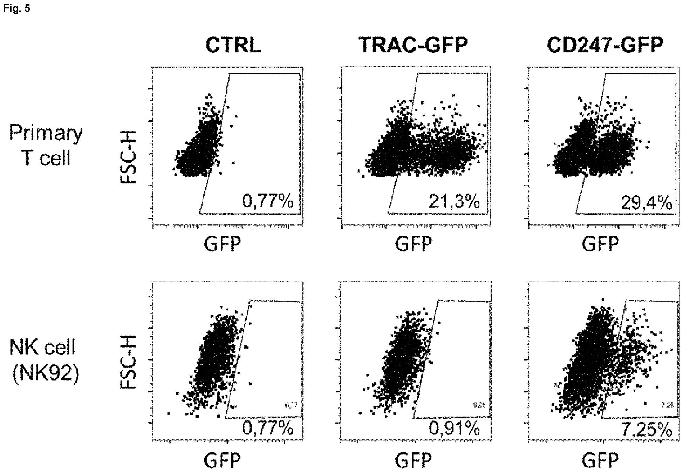

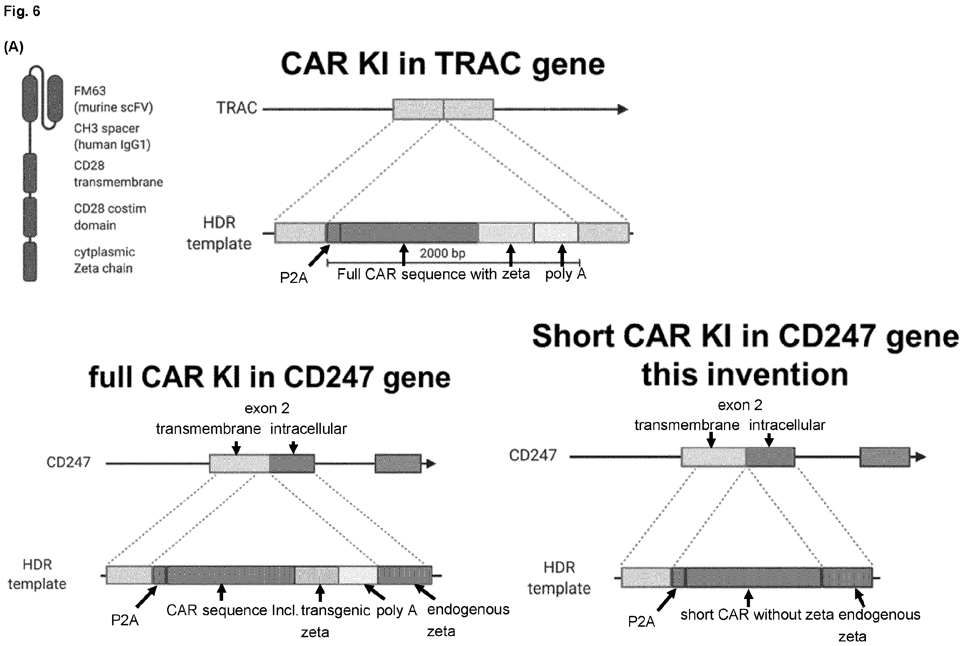

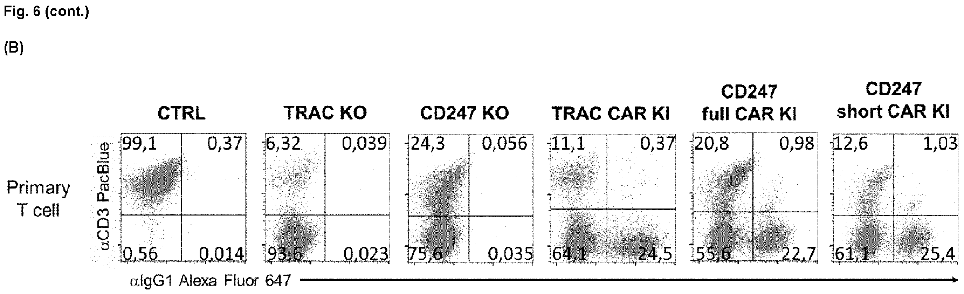

**Figure 5****: In NK cells, transgenes can be expressed under control of CD247 but not TRAC:** For this experiment, nuclease assisted gene insertion was repeated in Natural Killer (NK) cells (modeled by NK92 cell line) using preferred HDR templates and sgRNA-nuclease as before (Fig. 6, CD247: HDR template exon 2 + sgRNA 2.1; TRAC: exon 1 Charité + TRAC sg 1). To this end, NK92 cells were expanded in RPMI1640 medium supplemented with 10% FCS, 1% penicillin/streptomycin (Biochrom) and IL-2 (500IU/ml). Four days prior to electroporation, NK92 were seeded in antibiotic free medium. In preparation, RNPs and HDRTs as well as cells were prepared as previously described (Fig. 3). Nucleofection was performed with program CA137. NK92 cells were subsequently cultured for 2 days. Flow cytometric analysis was performed as before, but without CD3 antibody. Here, representative flow cytometric analysis are shown and compared to results from experiments with primary T cells (Fig. 4).

**Figure 6****: Insertion of complete and CD3 zeta-deficient CAR transgenes into CD247 gene induce CAR protein surface expression:** Nuclease assisted gene insertion was used to introduce complete or CD3 zeta-deficient (truncated) CAR transgenes into the CD247 gene. To perform this experiment, new HDR templates were designed. We rationally designed a 2nd generation CAR consisting of a murine CD19-specific single chain variable fragment (scFv), clone FMC63, an intermediate hinge CH3 spacer (derived from human Fc-IgG1), a CD28 transmembrane domain, a CD28 costimulatory intracellular domain and the CD3 cytoplasmic zeta chain. This CAR was cloned into HDR templates presented before (Fig. 3-5, Table 1 in Example 5). For CD247 HDR templates, a full CAR expression module with CD3 zeta and poly-A terminator sequence as well as a short CAR sequence lacking a poly-A terminator sequence were generated to test the hypothesis of this invention. Nucleofection was performed as described in Figure 3. Flow cytometry was performed 7 days after nucleofection. To stain for CAR via its IgG1 hinge, cells were stained with LIVE/dead and anti-Fc Alexa Fluor 647 (Jackson) first. After washing, a second surface stain with anti-CD3 PacBlue (Biolegend) was done. *(Note: for this experiment CD247 sg2.2 was used. For all subsequent experiment CD247 sg2.1 is used)*

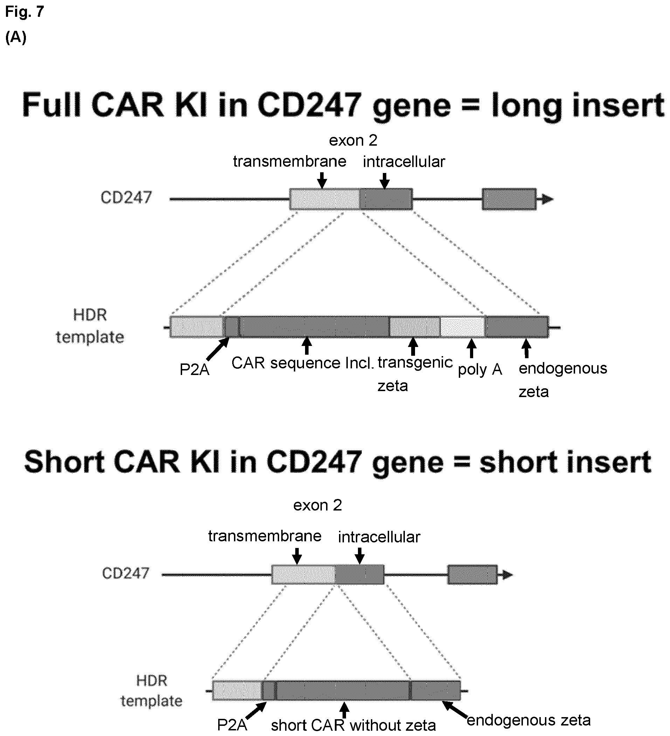

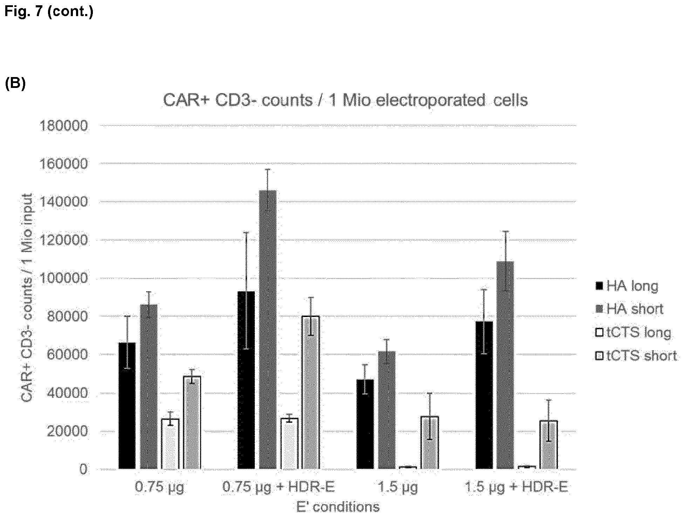

**Figure 7****: Introducing shorter insert size tends to increase absolute CAR T cell generation after non-viral gene insertion:** The effect of insert size (full CAR vs short zeta-less CARs) on absolute CAR+ T cell counts was compared after nuclease assisted gene insertion with two different non-viral dsDNA HDR template variants. HDR templates were either generated with regular homology arms (HA) (Fig. 3, previously described in PMID: 29995861) or with homology arms modified with truncated Cas9 targeting sequences (tCTS) (16 bp, previously described in PMID: 31819258). After Electroporation, cells were either cultured in T cell medium or in T cell medium supplemented with 15 uM (0.5%) Alt-R HDR-Enhancer (Integrated DNA Technologies, Catalog #1081073). Two HDR template concentrations (0.75 or 1.50 ug / 20 ul Electroporation) were compared. Regardless of the HDR template format used, smaller insert size due to the zeta-less CAR transgenes lead to improved CAR+ T cell recovery 7 days after transfection, thereby indicating an advantage for CAR T cell generation using this approach.

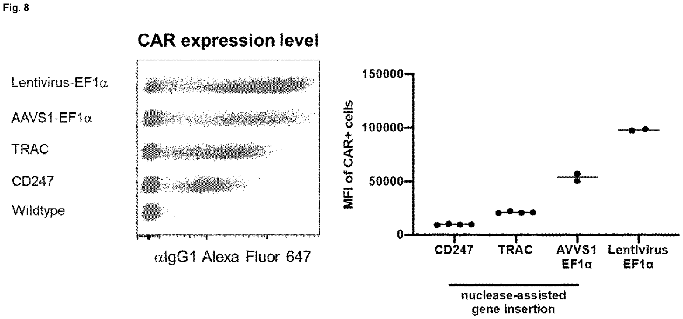

**Figure 8****: CAR expression level is regulated most strictly under control of the CD247 gene:** In this experiment, CAR expression levels were compared between CAR T cells generated by different strategies. Representative flow cytometry plots and summary of median fluorescence intensity of CAR+ CD3- cells are shown. CAR T cells generated through insertion of truncated CAR transgenes into the CD247 exon 2 (sg 2.1) display the lowest MFI compared to TRAC-integrated CARs or other controls. For overexpression controls, the CAR presented in Fig. 6 was cloned into an expression cassette commonly used for generation of CAR T cells via lentiviruses or via transposase-transposon technology. It includes an EF1-alpha promoter and a BGH poly-A terminator sequence. The expression cassette was cloned into an HDR template for insertion into the human safe harbor locus hAAVS1. Additionally, the CAR was cloned into the SIN epHIV7 lentiviral vector plasmid for virus preparation and lentiviral transduction was performed as previously described (PMID: 22707919).

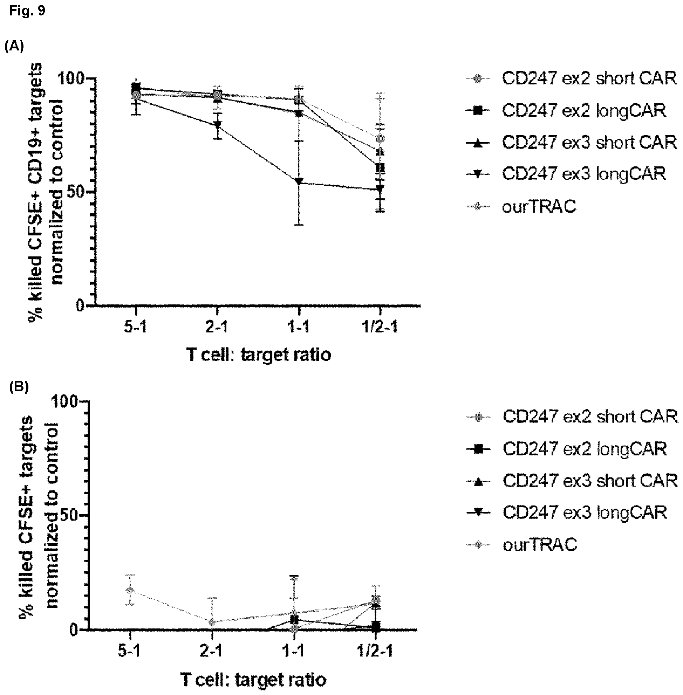

**Figure 9****: Short CAR CD247 KI CART cell lyse CD19+ target cells in antigen-specific and dose-dependent fashion (as well as at a comparable rate to TRAC/CD247-integrated full CARs):** CAR T cells generated either by short (truncated) or full CAR transgenes lysed target cells in an antigen- and dose-dependent fashion and with similar efficacy as TRAC-integrated CAR T cells. The only CAR T cell condition with reduced killing capacity at 2-1 and 1-1 T cell : target ratios was generated with the HDR template CD247 exon 3 inserting a full CAR expression cassette. To test the cytolytic activity of CAR T cells generated through transgene insertion into the CD247 locus, overnight co-culture assays with tumor cells were performed. CAR T cells generated either with nuclease assisted gene insertion into the CD247 exon 2, CD247 exon 3 or TRAC exon 1 were tested. (A) CD19+ tumor cells (Nalm6) or (B) CD19- tumor cells (Jurkat) were stained with CFSE to allow discrimination between targets (tumor cells) and effectors (CAR T cells). Co-cultures were performed in 96-U-Bottom-Well plates with 25.000 tumor cells per well. Depending on desired T cell : target ratio, respective numbers of CAR T cells were added to the specific wells. As controls, tumor cells were cultured without any T cells. On the following day, the cells were washed with PBS, centrifuged at 400 G for 10 minutes and stained with LIVE/Dead dye to exlude apoptotic cells. Subsequently, surviving cells were quantified by volume-based flow cytometric acquisition (Cytoflex LX, Beckman). The percentage of killing was calculated by normalizing to the negative control. TRAC-integrated CAR T cells served as a positive control. [KI = knock-in]

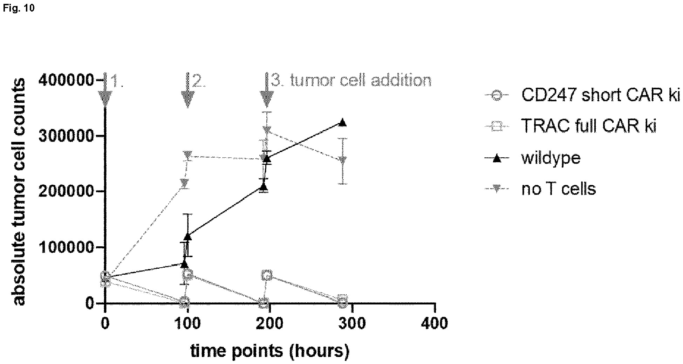

**Figure 10****: CD247 short CAR KI T cells perform comparable to full CAR KI in serial tumor challenge:** CAR T cells reprogrammed by insertion of truncated (zeta-deficient) CARs into the CD247 gene were able to eliminate tumor cells repeatedly. At the day of assay setup, 25.000 T cells were mixed with 50.000 CD19+ tumor cells (Nalm6) in cytokine-free medium (RPMI1640 + 10% FCS). Per condition, four wells were prepared. The first well was analysed at setup day to control absolute tumor cell counts. Every third day, one well per condition was analysed and 50.000 CD19+ tumor cells (Nalm6) are added to the remaining wells. In summary, absolute tumor cell counts are presented. In negative controls (conditions with no T cells or with wildtype T cells), tumor cells grew uncontrolled. Both TRAC-integrated full-CAR equipped and CD247-integrated short CAR-reprogrammed T cells repeatedly eliminated tumor cells. [KI = knock-in]

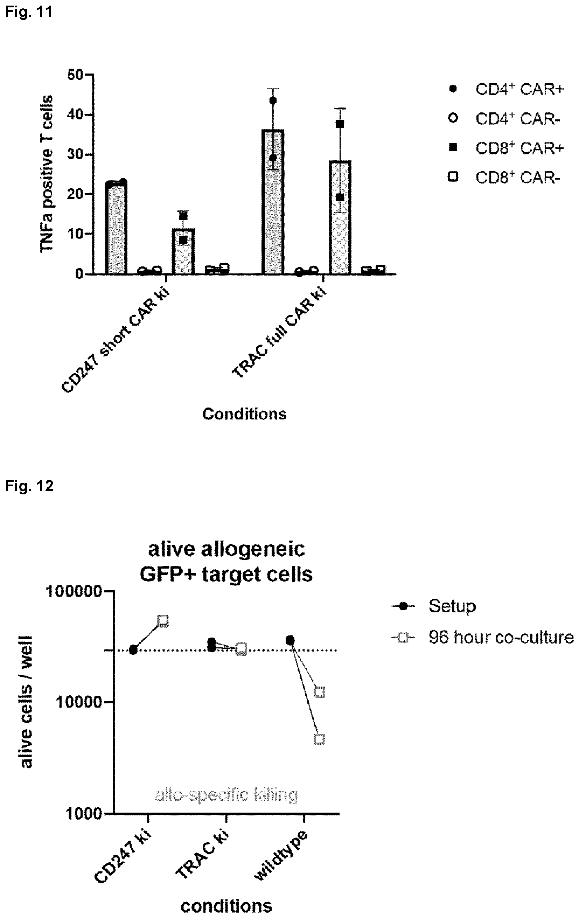

**Figure 11****: Reduced cytokine production after antigen-engagement by truncated CARs integrated in the CD247 gene vs full CAR in the TRAC locus:** As presented before (e.g. Fig. 8), CD247 integrated zeta-deficient CAR-reprogrammed T cells have a lower CAR expression level. While cytolytic activity remained unaffected by lower CAR surface density (Fig. 9,10), secretion of inflammatory cytokine TNF-alpha was reduced after challenge with CD19+ tumor cells (Nalm6) in both CD4+ and CD8+ T cells. One hour after start of co-culture, brefeldin A (Sigma) was added at 10 µg/ml to prevent cytokine secretion into the medium. After 6 hour total incubation, the reaction was stopped by washing in cold PBS. Then, surface staining with LIVE/dead Blue Cell Dye (1:100, Thermo Fisher) was performed to exclude dead cells. Fixation and permeabilization was performed with Foxp3 Transcription Staining Kit according to the manufacturer's recommendations (Ebioscience, ThermoFisher). Subsequent intracellular staining was performed in two steps; first anti-Fc Alexa Fluor 647 (1:50, Jackson) was stained at 4°C for 20 minutes. After wash with Permbuffer (same kit as before), a second intracellular staining was performed with antibodies for CD4, CD8 and TNF-alpha.

**Figure 12****: CD247 KI abolishes allo-reactivity of CAR T comparable to TRAC KI:** As presented before (e.g. Fig. 6), insertion of (truncated) CARs into the TRAC as well as the CD247 gene eliminates the CD3-TCR from the T cell surface. To test whether this eliminates allogeneic cell killing, we performed co-culture assay with human T cells from two unmatched healthy donors with strong allo-reactivity against the CD19-negative cell line MM1S expressing a GFP reporter. After four days, unmodified wildtype T cells eliminated a significant fraction of the tumor cells. In contrast the amount of tumor cells remained unchanged for TRAC-inserted CAR T cells or they increased in the presence of the CD247-integrated CAR T cells, indicating undectable rate of allo-activity.

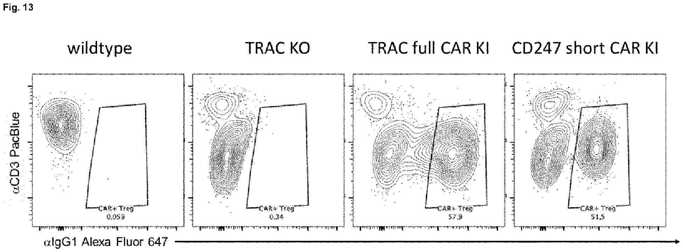

**Figure 13****: Integration of truncated CARs into CD247 gene can be used to generate CAR+ regulatory T cells:** CD4+ Treg were generated from PBMCs as described before (Fig. 3). On the day of PBMC isolation, CD8+ cells were eliminated using CD8+ microbead depletion (Miltenyi Biotec). The following day, CD25+ cells were enriched (CD25 microbeads, Miltenyi Biotec) from the CD8-negative cell fraction to isolate CD4+ CD25+ T cells which contain >70% regulatory T cells. Subsequently, the cells activated with anti-CD3/28-coated beads and expanded in X-Vivo cell medium (Lonza) supplemented with 10% FCS, high doses of IL-2 (500IU/ml) and Rapamycin (Treg medium) as described before (PMID: 33087345). On day 7 after PBMC isolation, regulatory T cells were re-stimulated with anti-CD3/28-coated beads. Next day, the beads were magnetically depleted and nucleofected as described before (Fig. 3). Rescue was performed with the Treg medium. After resting one more day, the nucleofected T cells were re-stimulated with irradiated CD19+ target cells (Nalm-6) and sub-cultured for additional 7 days prior to flow cytometric analysis.

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

The constructs employed in the examples are disclosed in the sequence tables above.

### Example 1: Novel strategy fuses zeta-deficient CAR transgenes to endogenous CD3 zeta at gene level

In one embodiment, sketches in Figure 1 present a comparison between CAR-T cells generated by conventional methods (Fig. 1A) and a preferred strategy provided by this invention (Fig. 1B). As shown in Figure 1A, CD3-zeta protein expression from the CD247 gene. In the process of RNA transcription, CD247 exons are spliced together to form a mature CD247 messenger RNA, which serves as a template for protein translation by the ribosome. The formed CD3-zeta protein assembles with other proteins to form a fully functioning TCR/CD3 complex. The intracellular domain of the CD3-zeta protein within the TCR/CD3 complex is identical to the CD3-zeta domain of conventional Chimeric Antigen Receptors. Figure 1B illustrates the strategy of a preferred embodiment of the invention. A truncated, CD3-zeta-deficient CAR transgene (preferably but not exclusively encoding a self-cleaving 2A-peptide, an antigen binding domain (e.g. an scFv), a hinge/spacer domain, a transmembrane domain and one or more co-stimulatory or regulatory domains but no Immunoreceptor Tyrosine-based Activation Motif (ITAM) containing signaling domain) is inserted into the CD247 gene. The modified CD247 gene is transcribed into mRNA encoding a full CAR with an endogenous CD3-zeta domain. During protein translation, the N-terminus of the expressed fusion protein is cleaved off the CAR protein via self-cleavage of the 2A peptide. While a functional CAR Protein is expressed on the surface of edited immune cells, the cleaved N-terminus of the CD3-zeta gene is non-functional and cannot serve for TCR/CD3 complex formation. As a result, successfully edited immune cells can express a CAR protein but are unable to express a TCR.

### Example 2: In-frame insertion of GFP into different T cell receptor complex genes leads to loss of CD3-TCR complex surface expression

In these experiments as shown in Figure 2 and 3, nuclease-assisted gene insertion of green fluorescent protein (GFP) reporter transgenes into the protein-coding reading frame of either the CD247 gene (encoding for CD3 zeta) or the TRAC gene (encoding for TCR alpha chain) was performed to investigate the effect on CD3-TCR surface expression. As indicated by flow cytometric protein analysis, most GFP expression was observed in the CD3-TCR negative population (Y-axis). Multiple Cas9 nucleases with different sgRNAs were used for CD247 exon 2 and 3 achieving similar results. Insertion of GFP into TRAC gene mirrored results seen in CD247, despite higher maximum GFP intensity.

**Generation of dsDNA templates for homology-directed DNA repair:** To this end, three different homology-directed repair (HDR) templates were designed to insert the transgene sequence in-frame into the exons 2 or 3 of the CD247 gene and the TRAC exon 1 via nuclease-assisted gene insertion. For TRAC locus, two templates were generated: one as previously published by Roth et al (PMID: 29995861), one self-designed template (TRAC exon 1 Charité) for use with the sgRNA TRAC sg1, which has fewer predicted off-target (PMID: 32241852). To allow for independent protein expression, the insert was designed to include a P2A-peptidase sequence and a bovine-growth hormone (bgh) poly-adenylation sequence. Synthesized homology arms (ca. 400 bp each) and inserts were cloned into pUC19 plasmid backbone using In-Fusion system according to the manufacturer's protocols (Clontech, Takara). Plasmids were purified using column-based plasmid purification (Zymo Research, ZymoPure Miniprep Kit) and sequences were validated using Sanger Sequencing. For generation of highly-purified HDR templates made from double stranded DNA (dsDNA), polymerase chain reaction (PCR) with high-fidelity enzymes (KAPA HiFi HS Polymerase, 2x Readymix; Roche) was performed at large volumes (typically 500-1000ul reactions) using 10-20 ng plasmid template. HDR templates were purified using Ampure XP magnetic bead purification (1 volume beads : 1 volume PCR product) according to the manufacturer's manual (Beckman Coulter). DsDNA HDR templates were resuspended in nuclease-free water; their purity was validated by agarose gel electrophoresis. HDR template concentration was measured using NanoDrop 1000 Spectrophotometer (ThermoFisher). Subsequently, HDR templates were aliquoted at 1000 ng/microliter or 2000 ng/microliter and cryopreserved at -20°C until further use.

**Nuclease formulation:** CRISPR-Cas9 nuclease system was used to induce DNA double strand breaks and provoke DNA repair at the targeted locus. To this end, ribonucleoproteins (RNP) were formulated freshly for each experiment. Per 20 ul nucleofection volume, 0.5 µl poly-L-glutamic acid (15-50kDa, 100mg/mL, Sigma), 0.48 ul of respective sgRNA (100uM, Synthego) and 0.4 µl of Sp.Cas9 (10mg/ml, IDT) were mixed in this order and incubated for 15 minutes at room temperature in nuclease-free PCR tubes. Instantly prior to nucleofection, RNP were mixed with dsDNA HDR templates.

**Cell culture and electroporation:** Peripheral blood mononuclear cells (PBMCs) were isolated from heparinized venous blood of healthy human adults after informed consent. T cells were enriched using CD3+ microbeads and magnetic columns enrichment (MACS, Miltenyi Biotec). T cells were cultured in RPMI1640 (PAN) with 10% FCS (Biochrom) supplemented with IL-2 (100IU/ml, Novartis), IL-7 (10ng/ml, Cellgenix) and IL-15 (5ng/ml, Cellgenix). After 48 hours polyclonal stimulation on anti-CD3/28-coated tissue culture well plates, activated T cells were harvested and washed twice by resuspension in 1x phosphate buffered saline (PBS) and centrifugation at 100G for 10 minutes. Per 20ul nucleofection, 1-1.5 x 10e6 T cells were used. Dried pellets were resuspended with nucleofection buffer P3 primary cell (Lonza) and mixed with RNP-HDR template suspenion, and electroporated using the program EH-115 (4D-Nucleofector, Lonza). Subsequently, nucleofected cells are rescued with pre-warmed T cell medium and transferred into tissue culture wells containing T cell medium supplemented with 15µM HDR enhancer (IDT). 16 hours post-electroporation, medium was changed to remove HDR enhancer. T cells were split every other day.

**Flow cytometric analysis:** (2-) 7 days after nucleofection, T cells were harvested, washed with PBS, centrifuged at 400 G for 4 minutes. Supernatants were discarded and cell pellets resuspended prior to staining with LIVE/DEAD^{™} Fixable Blue Dead Cell Stain Kit (1:100, Molecular Probes, now ThermoFisher) and CD3 APC-AF700 (1:100, Beckman Coulter) for 15 minutes at 4C in the dark. Subsequently, cells were washed again and resuspended for analysis on Cytoflex LX, flow cytometer. Data was analysed with FlowJo 10 software (BD). Representative plots were selected displaying alive single lymphocytes in the dimensions CD3 (TCR complex) in y-axis and GFP (successful gene insertion).

### Example 3: GFP intensity is lower when expressed under control of the CD247 promotor compared to the TRAC promotor

This subanalysis shown in Figure 4 from the previous experiment (Fig. 3) compares GFP knock-in (KI) rates and fluorescence intensity in three replicates summarized for the preferred conditions (CD247: HDR template exon 2 + sgRNA 2.1; TRAC: exon 1 Charité + TRAC sg1). While relative KI did not differ significantly between TRAC and CD247, GFP expressed under control of the CD247 promotor leads to a lower median fluorescence intensity (MFI). [KI = knock-in]

### Example 4: In NK cells, transgenes can be expressed under control of CD247 but not TRAC

In another experiment as shown in Figure 5, nuclease assisted gene insertion was repeated in Natural Killer (NK) cells (modeled by NK92 cell line) using preferred HDR templates and sgRNA-nuclease as before (Fig. 4, CD247: HDR template exon 2 + sgRNA 2.1; TRAC: exon 1 Charité + TRAC sg 1). To this end, NK92 cells were expanded in RPMI1640 medium supplemented with 10% FCS, 1% penicillin/streptomycin (Biochrom) and IL-2 (500IU/ml). Four days prior to electroporation, NK92 were seeded in antibiotic free medium. In preparation, RNPs and HDRTs as well as cells were prepared as previously described (Fig. 3). Nucleofection was performed with program CA137. NK92 cells were subsequently cultured for 2 days. Flow cytometric analysis was performed as before, but without CD3 antibody. Here, representative flow cytometric analysis are shown and compared to results from experiments with primary T cells (Figure 4).

### Example 5: Insertion of complete and zeta-deficient CAR transgenes into CD247 gene induce CAR protein surface expression

Nuclease assisted gene insertion was used to introduce complete or zeta-deficient (truncated) CAR transgenes into the CD247 gene (Fig. 6). To perform this experiment, new HDR templates were designed. We rationally designed a 2nd generation CAR consisting of a murine CD19-specific single chain variable fragment (scFv), clone FMC63, an intermediate hinge CH3 spacer (derived from human Fc-IgG1), a CD28 transmembrane domain, a CD28 costimulatory intracellular domain and the CD3 cytoplasmic zeta chain. This CAR was cloned into HDR templates presented before (Fig. 3-5, Table 1). For CD247 HDR templates, a full CAR expression module with CD3 zeta and poly-A terminator sequence as well as a short CAR sequence lacking a poly-A terminator sequence were generated to test the hypothesis of this invention. Nucleofection was performed as described in Figure 3. Flow cytometry was performed 7 days after nucleofection. To stain for CAR via its IgG1 hinge, cells were stained with LIVE/dead and anti-Fc Alexa Fluor 647 (Jackson) first. After washing, a second surface stain with anti-CD3 PacBlue (Biolegend) was done. (Note: *for this experiment CD247 sg2.2 was used. For all subsequent experiment CD247 sg2.1 is used)*

**Table 1: sgRNA sequences and respective locations/ donor templates. [sg = single guide RNA, AAVS1 = Adeno-associated virus integration site 1]]**

| **Location & corresponding donor template names:** | **CD247 Exon 2** | **CD247 Exon 3** | **Ctrl: TRAC Exon 1 - Marson Lab** | **Ctrl: TRAC Exon 1 - Charité** | **Ctrl: AAVS1** |
|---|---|---|---|---|---|
| **SpCas9 sgRNAs** | gaUggaaUc cUcUUcaUc Ua (SEQ ID NO 52) CD247 sg2.1: | gacgcccccg cgUaccagca (SEQ ID NO 53) CD247 sg1: | AGAGUCU CUCAGCU GGUACA (SEQ ID NO 54) TRAC (Roth et al 2018): | TRAC sg 1 GGGAAUC AAAAUCGG UGAAU (SEQ ID NO 55) | AAVS1 (Mali et al 2013) GGGGCCA CUAGGGA CAGGAU (SEQ ID NO 56) |
| | UGGUACC CACCUUCA CUCUC (SEQ ID NO 57) CD247 sg2.2: | | | | |
| | UgccUUgU UccUgagag Uga (SEQ ID NO 58) CD247 sg2.4: | | | | |

### Example 6: Introducing shorter insert size tends to increase absolute CAR T cell generation after non-viral gene insertion

The effect of insert size (full CAR vs short zeta-less CARs, Fig. 7) on absolute CAR+ T cell counts was compared after nuclease assisted gene insertion with two different non-viral dsDNA HDR template variants. HDR templates were either generated with regular homology arms (HA) (Fig. 3, previously described in PMID: 29995861) or with homology arms modified with truncated Cas9 targeting sequences (tCTS) (16 bp, previously described in PMID: 31819258). After Electroporation, cells were either cultured in T cell medium or in T cell medium supplemented with 15 µM (0.5%) Alt-R HDR-Enhancer (Integrated DNA Technologies, Catalog #1081073). Two HDR template concentrations (0.75 or 1.50 µg / 20 µl Electroporation) were compared. Regardless of the HDR template format used, smaller insert size due to the zeta-less CAR transgenes lead to improved CAR+ T cell recovery 7 days after transfection, thereby indicating an advantage for CAR T cell generation using this approach.

### Example 7: CAR expression level is regulated most strictly under control of the CD247 gene

In this experiment as presented by Figure 8, CAR expression levels were compared between CAR T cells generated by different strategies. Representative flow cytometry plots and summary of median fluorescence intensity of CAR+ CD3- cells are shown. CAR T cells generated through insertion of truncated CAR transgenes into the CD247 exon 2 (sg 2.1) display the lowest MFI compared to TRAC-integrated CARs or other controls. For overexpression controls, the CAR presented in Figure 6 was cloned into an expression cassette commonly used for generation of CAR T cells via lentiviruses or via transposase-transposon technology. It includes an EF1-alpha promoter and a BGH poly-A terminator sequence. The expression cassette was cloned into an HDR template for insertion into the human safe harbor locus hAAVS1. Additionally, the CAR was cloned into the SIN epHIV7 lentiviral vector plasmid for virus preparation and lentiviral transduction was performed as previously described (PMID: 22707919). [MFI = median fluorescence intensity]

### Example 8: Short CAR CD247 KI CAR T cell lyse CD19+ target cells in antigen-specific and dose-dependent manner (as well as at a comparable rate to TRAC/CD247-integrated full CARs)

CAR T cells generated either by short (truncated) or full CAR transgenes lysed target cells in an antigen- and dose-dependent manner and with similar efficacy as TRAC-integrated CAR T cells (Fig. 9). The only CAR T cell condition with reduced killing capacity at 2-1 and 1-1 T cell : target ratios was generated with the HDR template CD247 exon 3 inserting a full CAR expression cassette. To test the cytolytic activity of CAR T cells generated through transgene insertion into the CD247 locus, overnight co-culture assays with tumor cells were performed. CAR T cells generated either with nuclease assisted gene insertion into the CD247 exon 2, CD247 exon 3 or TRAC exon 1 were tested. CD19+ tumor cells (Nalm6) or CD19- tumor cells (Jurkat) were stained with CFSE to allow discrimination between targets (tumor cells) and effectors (CAR T cells). Co-cultures were performed in 96-U-Bottom-Well plates with 25.000 tumor cells per well. Depending on desired T cell : target ratio, respective numbers of CAR T cells were added to the specific wells. As controls, tumor cells were cultured without any T cells. On the following day, the cells were washed with PBS, centrifuged at 400 G for 10 minutes and stained with LIVE/Dead dye to exclude apoptotic cells. Subsequently, surviving cells were quantified by volume-based flow cytometric acquisition (Cytoflex LX, Beckman). The percentage of killing was calculated by normalizing to the negative control. TRAC-integrated CAR T cells served as a positive control. [KI = knock-in]

### Example 9: CD247 short CAR KI T cells perform comparable to full CAR knock-in in serial tumor challenge

CAR T cells reprogrammed by insertion of truncated (zeta-deficient) CARs into the CD247 gene were able to eliminate tumor cells repeatedly (Fig. 10). At the day of assay setup, 25.000 T cells were mixed with 50.000 CD19+ tumor cells (Nalm6) in cytokine-free medium (RPMI1640 + 10% FCS). Per condition, four wells were prepared. The first well was analysed at setup day to control absolute tumor cell counts. Every third day, one well per condition was analysed and 50.000 CD19+ tumor cells (Nalm6) are added to the remaining wells. In summary, absolute tumor cell counts are presented. In negative controls (conditions with no T cells or with wildtype T cells), tumor cells grew uncontrolled. Both TRAC-integrated full-CAR equipped and CD247-integrated short CAR-reprogrammed T cells repeatedly eliminated tumor cells. [KI = knock-in]

### Example 10: Reduced cytokine production after antigen-engagement by truncated CARs integrated in the CD247 gene vs full CAR in the TRAC locus

As presented before (e.g. Fig. 8), CD247 integrated zeta-deficient CAR-reprogrammed T cells have a lower CAR expression level. While cytolytic activity remained unaffected by lower CAR surface density (Fig. 9,10), secretion of inflammatory cytokine TNF-alpha was reduced after challenge with CD19+ tumor cells (Nalm6) in both CD4+ and CD8+ T cells (Fig. 11). One hour after start of co-culture, brefeldin A (Sigma) was added at 10 µg/ml to prevent cytokine secretion into the medium. After 6 hour total incubation, the reaction was stopped by washing in cold PBS. Then, surface staining with LIVE/dead Blue Cell Dye (1:100, Thermo Fisher) was performed to exclude dead cells. Fixation and permeabilization was performed with Foxp3 Transcription Staining Kit according to the manufacturer's recommendations (Ebioscience, ThermoFisher). Subsequent intracellular staining was performed in two steps; first anti-Fc Alexa Fluor 647 (1:50, Jackson) was stained at 4°C for 20 minutes. After wash with Permbuffer (same kit as before), a second intracellular staining was performed with antibodies for CD4, CD8, and TNF-alpha.

### Example 11: CD247 KI abolishes allo-reactivity of CAR T comparable to TRAC KI

As presented before (e.g. Fig. 6), insertion of (truncated) CARs into the TRAC as well as the CD247 gene eliminates the CD3-TCR from the T cell surface. To test whether this eliminates allogeneic cell killing, we performed co-culture assay with human T cells from two unmatched healthy donors with strong allo-reactivity against the CD19-negative cell line MM1S expressing a GFP reporter (Fig. 12). After four days, unmodified wildtype T cells eliminated a significant fraction of the tumor cells. In contrast the amount of tumor cells remained unchanged for TRAC-inserted CAR T cells or they increased in the presence of the CD247-integrated CAR T cells, indicating undetectable rate of allo-activity. [KI = knock-in]

### Example 12: Integration of truncated CARs into CD247 gene can be used to generate CAR+ regulatory T cells

CD4+ Treg were generated from PBMCs as described before (Fig. 3). On the day of PBMC isolation, CD8+ cells were eliminated using CD8+ microbead depletion (Miltenyi Biotec). The following day, CD25+ cells were enriched (CD25 microbeads, Miltenyi Biotec) from the CD8-negative cell fraction to isolate CD4+ CD25+ T cells which contain >70% regulatory T cells. Subsequently, the cells activated with anti-CD3/28-coated beads and expanded in X-Vivo cell medium (Lonza) supplemented with 10% FCS, high doses of IL-2 (500IU/ml) and Rapamycin (Treg medium) as described before (PMID: 33087345). On day 7 after PBMC isolation, regulatory T cells were re-stimulated with anti-CD3/28-coated beads. Next day, the beads were magnetically depleted and nucleofected as described before (Fig. 3). Rescue was performed with the Treg medium. After resting one more day, the nucleofected T cells were re-stimulated with irradiated CD19+ target cells (Nalm-6) and sub-cultured for additional 7 days prior to flow cytometric analysis.

### References

Elsallab, M., Levine, B. L., Wayne, A. S. & Abou-EI-Enein, M. CAR T-cell product performance in haematological malignancies before and after marketing authorisation. Lancet Oncol. 21, e104-e116 (2020).
Gundry, M. C. et al. Highly Efficient Genome Editing of Murine and Human Hematopoietic Progenitor Cells by CRISPR/Cas9. Cell Rep. 17, 1453-1461 (2016).
Osborn, M. J. et al. Evaluation of TCR Gene Editing Achieved by TALENs, CRISPR/Cas9, and megaTAL Nucleases. Mol. Ther. 24, 570-581 (2016).
Qasim, W. et al. Molecular remission of infant B-ALL after infusion of universal TALEN gene-edited CAR T cells. Sci. Transl. Med. 9, (2017).
Eyquem, J. et al. Targeting a CAR to the TRAC locus with CRISPR/Cas9 enhances tumour rejection. Nature 543, 113-117 (2017).
MacLeod, D. T. et al. Integration of a CD19 CAR into the TCR Alpha Chain Locus Streamlines Production of Allogeneic Gene-Edited CAR T Cells. Mol. Ther. 25, 949-961 (2017).

## Claims

1. A nucleic acid construct comprising:
- a first nucleic acid sequence region, encoding a chimeric antigen receptor (CAR) fragment without a sequence encoding a functional intracellular domain of a CD3 zeta protein,
- a second nucleic acid sequence region, comprising a targeting sequence configured for integrating the construct at an endogenous CD3 zeta gene of a human cell, and
- a third nucleic acid sequence region encoding and/or comprising a protein separation site upstream of the first sequence region.

2. The nucleic acid construct according to claim 1, wherein the first nucleic acid sequence region encoding a CAR fragment comprises:
- a nucleic acid sequence encoding an extracellular antigen-binding domain, said antigen-binding domain preferably comprising an antibody or antibody fragment,
- a nucleic acid sequence encoding a transmembrane domain, and
- a nucleic acid sequence encoding one or more intracellular co-stimulatory domains.

3. The nucleic acid construct according to any of the preceding claims, wherein the targeting sequence is configured for integrating the construct in-frame with an endogenous CD3 zeta encoding sequence, preferably into an exon of an endogenous CD3 zeta gene.

4. The nucleic acid construct according to any of the preceding claims, wherein the protein separation site of the third nucleic acid sequence encodes a first self-cleavage peptide, protease cleavage site or an internal ribosomal entry site (IRES).

5. The nucleic acid construct according to any of the preceding claims, wherein the target site of the CD3 zeta gene, into which the first sequence region is integrated, is an exon and/or an intron, preferably exon 2, and is positioned upstream of an endogenous CD3 zeta sequence encoding an intracellular domain or fragment thereof, said intracellular domain or fragment thereof comprising one or more immunoreceptor tyrosine-based activation motifs (ITAMs).

6. The nucleic acid construct according to any of the preceding claims, wherein the first nucleic acid sequence region encoding a CAR fragment additionally comprises a sequence encoding a CAR-regulating, immune-stimulating and/or therapeutic protein, in-frame and separated from the CAR fragment-encoding region by a protein separation site, such as a sequence encoding a second self-cleavage peptide, a promoter, and/or an IRES site.

7. The nucleic acid construct according to any of the preceding claims, wherein the targeting sequence of the second sequence region is configured for integration into a host genome by a gene editing technique, preferably CRISPR-Cas, zinc finger nucleases (ZFNs), integrases, site specific recombinases, meganucleases, homing endonucleases, or TALENs, more preferably a CRISPR-Cas9 derived from *Streptococcus pyogenes,*
and/or
wherein the targeting sequence comprises an identical sequence to a recognition site of the endogenous DNA sequence into which integration is intended, preferably selected from the group consisting of a homology arm, a guide RNA target site, a restriction enzyme recognition site, a ZFN recognition site, a ZFN cleaving site, a TALEN DNA-binding site, a recombinase recognition site, an integrase site and/or a homing nuclease recognition site.

8. A genetically modified human cell, comprising an exogenous nucleic acid sequence region encoding a chimeric antigen receptor (CAR) fragment, without a sequence encoding a functional intracellular domain of a CD3 zeta protein, integrated in-frame into an endogenous CD3 zeta gene.

9. The genetically modified cell according to claim 8, wherein a chimeric antigen receptor (CAR) is expressed as an in-frame fusion protein comprising the CAR fragment and an intracellular domain of the endogenous CD3 zeta gene, said intracellular domain comprising one or more immunoreceptor tyrosine-based activation motifs (ITAMs).

10. The genetically modified cell according to any of claims 8 or 9, wherein the chimeric antigen receptor (CAR)-CD3 zeta fusion gene is expressed under the control of an endogenous CD3 zeta promoter, wherein preferably expression of the chimeric antigen receptor (CAR) is terminated by an endogenous CD3 zeta gene expression termination signal and an endogenous poly-A signal.

11. The genetically modified cell according to any of claims 8 to 10, wherein the endogenous expression and/or function of a TCR complex, preferably the expression and/or function of a TCR alpha and/or beta chain, is disrupted.

12. The genetically modified cell according to any of claims 8 to 11, wherein the cell is an immune cell, preferably selected from the group consisting of a T lymphocyte, a T regulatory (Treg) cell, natural killer (NK) cell, a natural killer T (NKT) cell, cytokine-induced killer cell (CIK), an immortalized immune cell including NKT, NK-92 and YT cells, or a cell derived from peripheral human blood, human cord blood, bone marrow stem cells or induced pluripotent stem cells (iPSC), wherein the T lymphocyte is preferably a CD4 or CD8 T-cell, more preferably a cytotoxic T lymphocyte or a T helper cell.

13. The genetically modified cell according to any of claims 8 to 12 for use as a medicament, preferably for use in the treatment of a medical disorder associated with the presence of pathogenic cells expressing oncogenes, such as cancer cells, more preferably cancer stem cells of solid and hematologic malignancies, or preferably for use in the treatment of an autoimmune disease or a graft versus host disease.

14. The genetically modified cell for use according to claim 13, wherein the genetically modified cell is allogeneic with respect to a patient, preferably an allogeneic T-cell, NK-cell or Treg.

15. The genetically modified cell for use according to claim 13, wherein the genetically modified cell is autologous with respect to a patient, preferably an autologous T-cell, NK-cell or Treg.

16. A chimeric antigen receptor (CAR) polypeptide expressed from a genetically modified cell according to any of claims 8 to 12, comprising a chimeric antigen receptor (CAR) as an in-frame fusion protein comprising an exogenous CAR fragment without a sequence encoding a functional intracellular domain of a CD3 zeta protein, and an intracellular domain of the endogenous CD3 zeta gene, said intracellular domain comprising one or more immunoreceptor tyrosine-based activation motifs (ITAMs).

17. A pharmaceutical composition comprising a genetically modified cell according to any of claims 8 to 12 and a pharmaceutically acceptable carrier.
